(19) **Europäisches Patentamt / European Patent Office / Office européen des brevets**

(11) **EP 4 703 377 A1**

(12) **EUROPEAN PATENT APPLICATION**
published in accordance with Art. 153(4) EPC

(43) Date of publication:
04.03.2026 Bulletin 2026/10

(21) Application number: 24797149.2

(22) Date of filing: 25.04.2024

(51) International Patent Classification (IPC):
C07K 16/10 (2026.01)    A61K 39/395 (2006.01)
A61K 47/68 (2017.01)    A61P 31/16 (2006.01)
C12N 15/13 (2006.01)    G01N 33/569 (2006.01)

(52) Cooperative Patent Classification (CPC):
A61K 39/395; A61K 47/68; A61P 31/16;
C07K 16/00; C07K 16/10; G01N 33/569

(86) International application number:
PCT/JP2024/016344

(87) International publication number:
WO 2024/225404 (31.10.2024 Gazette 2024/44)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 26.04.2023 JP 2023072516

(71) Applicants:
• Japan Institute for Health Security
  Tokyo 162-8655 (JP)
• Sumitomo Pharma Co., Ltd.
  Osaka-shi, Osaka 541-0045 (JP)

(72) Inventors:
• TAKAHASHI, Yoshimasa
  Tokyo 162-8655 (JP)
• ADACHI, Yu
  Tokyo 162-8655 (JP)
• TONOUCHI, Keisuke
  Tokyo 162-8655 (JP)
• NAKAKO, Mayumi
  Osaka-shi, Osaka 541-0045 (JP)

(74) Representative: Mewburn Ellis LLP
Aurora Building
Counterslip
Bristol BS1 6BX (GB)

Remarks:
The complete document including Reference
Table(s) and the Sequence Listing(s) can be
downloaded from the EPO website

(54) **ANTI-INFLUENZA ANTIBODY**

(57) The present application provides a monoclonal antibody or an antibody fragment thereof, wherein the monoclonal antibody or antibody fragment thereof binds to a hemagglutinin stem region (HA stem region) of an influenza A virus.

EP 4 703 377 A1

## Description

Technical Field

The present disclosure includes a monoclonal antibody or antibody fragment that binds to an influenza virus-derived antigen.

Background Art

Influenza viruses are classified into type A, type B, and type C based on the difference in antigenicity of viral nucleoprotein (NP) and matrix protein (M1) (Non Patent Literature 3). Those that repeatedly prevail among humans are of the types A and B, and all the viruses that caused great pandemics are of the type A (Non Patent Literature 1). Influenza A and B viruses both have hemagglutinin (hereinafter, hemagglutinin is also abbreviated as "HA"), which functions in invading in host cells. There are 18 types of hemagglutinin A (H1 to H18), and influenza A viruses are classified into two phylogenetic groups (phylogenetic groups 1 and 2) based on the genetic homology of hemagglutinin (Non Patent Literature 2).

The hemagglutinin can be structurally and functionally divided into two regions called a globular head region and a stem region. The globular head region is externally exposed from the virus membrane, and antibodies induced by administration of an influenza HA vaccine that has been currently put into practical use bind to the globular head region, and exhibit a protective effect against infection by inhibiting the binding of viruses to cell surfaces. However, the hemagglutinin globular region is the moiety having the largest structural variation among virus strains, and hence most antibodies induced are "virus strain-specific antibodies", each being effective only to a specific strain, and thus fail to bind to virus strains with antigenic variation and exhibit no protective effect against infection; this has been considered a problem (Patent Literature 1).

As protective antibodies against infection that differ from those virus strain-specific antibodies, "cross-protective antibodies", which recognize conserved epitopes that are less likely to undergo antigenic variation to exhibit a wide range of protective effects against infection, have been reported in recent years (Non Patent Literatures 2, 3).

Citation List

Patent Literature

Patent Literature 1: Japanese Patent Laid-Open No. 2019-43937

Non Patent Literature

Non Patent Literature 1: VIRUS Vol. 69, No. 2, pp. 161-168, 2019
Non Patent Literature 2: Front. Immunol. (2018) 9:116
Non Patent Literature 3: Nat. Commun. (2019) 10:3883

Summary of Invention

Technical Problem

An object of the present disclosure is to provide a monoclonal antibody or an antibody fragment thereof, the monoclonal antibody or antibody fragment thereof being effective for treating or preventing an influenza A virus infection. A further object of the present disclosure is to provide a method for detecting or quantifying an influenza A vaccine antigen for quality control or the like in influenza A vaccine production, and a method for detecting or quantifying an influenza antibody for efficacy evaluation or the like in clinical vaccine development.

Solution to Problem

Through diligent examination, the present inventors have obtained a plurality of monoclonal antibodies that bind to an LAH (long alpha helix) region in a hemagglutinin stem region (hereinafter, also referred to as an HA stem region, stem region, or stem) of an influenza A virus, and found antibodies that exhibit protective effect against infection for an influenza virus in animals among those monoclonal antibodies. In addition, some of the antibodies have been found to have a wide range of binding activity against influenza A virus HA subtypes belonging to phylogenetic group 1, and to have a wide range of binding activity against influenza A virus HA subtypes belonging to phylogenetic group 2. Moreover, the present inventors have found a method for detecting or quantifying an influenza A vaccine antigen (in particular, a membrane fusion-type influenza vaccine antigen) by using those antibodies, and a method for detecting or quantifying an influenza antibody for efficacy evaluation or the like in clinical vaccine development by using those antibodies.

For example, the present disclosure provides the following.

[Item 1]

A monoclonal antibody or an antibody fragment thereof, wherein the monoclonal antibody or antibody fragment thereof binds to a hemagglutinin stem region (HA stem region) of an influenza A virus.

[Item 2]

The monoclonal antibody or antibody fragment thereof according to item 1, wherein the monoclonal antibody or antibody fragment thereof binds to at least one peptide consisting of an amino acid sequence selected from the group consisting of SEQ ID NOs: 1 to 18.

[Item 3]

The monoclonal antibody or antibody fragment thereof according to item 1 or 2, wherein the monoclonal antibody or antibody fragment thereof binds to an LAH (long alpha helix) region of the HA stem region.

[Item 4]

The monoclonal antibody or antibody fragment thereof according to any one of items 1 to 3, wherein the monoclonal antibody or antibody fragment thereof binds to at least one peptide consisting of an amino acid sequence selected from the group consisting of SEQ ID NOs: 19 to 36.

[Item 5]

The monoclonal antibody or antibody fragment thereof according to any one of items 1 to 4, wherein the monoclonal antibody or antibody fragment thereof comprises:

a heavy chain variable region comprising CDR1 comprising an amino acid sequence selected from the group consisting of SEQ ID NOs: 47, 55, 63, 71, 79, and 87 or an amino acid sequence in which one or two amino acid residues are independently substituted, deleted, added, or inserted in the amino acid sequence selected from the group consisting of SEQ ID NOs: 47, 55, 63, 71, 79, and 87, CDR2 comprising an amino acid sequence selected from the group consisting of SEQ ID NOs: 48, 56, 64, 72, 80, and 88 or an amino acid sequence in which one or two amino acid residues are independently substituted, deleted, added, or inserted in the amino acid sequence selected from the group consisting of SEQ ID NOs: 48, 56, 64, 72, 80, and 88, and CDR3 comprising an amino acid sequence selected from the group consisting of SEQ ID NOs: 49, 57, 65, 73, 81, and 89 or an amino acid sequence in which one or two amino acid residues are independently substituted, deleted, added, or inserted in the amino acid sequence selected from the group consisting of SEQ ID NOs: 49, 57, 65, 73, 81, and 89; and/or

a light chain variable region comprising CDR1 comprising an amino acid sequence selected from the group consisting of SEQ ID NOs: 50, 58, 66, 74, 82, and 90 or an amino acid sequence in which one or two amino acid residues are independently substituted, deleted, added, or inserted in the amino acid sequence selected from the group consisting of SEQ ID NOs: 50, 58, 66, 74, 82, and 90 , CDR2 comprising an amino acid sequence selected from the group consisting of SEQ ID NOs: 51, 59, 67, 75, 83, and 91 or an amino acid sequence in which one or two amino acid residues are independently substituted, deleted, added, or inserted in the amino acid sequence selected from the group consisting of SEQ ID NOs: 51, 59, 67, 75, 83, and 91, and CDR3 comprising an amino acid sequence selected from the group consisting of SEQ ID NOs: 52, 60, 68, 76, 84, and 92 or an amino acid sequence in which one or two amino acid residues are independently substituted, deleted, added, or inserted in the amino acid sequence selected from the group consisting of SEQ ID NOs: 52, 60, 68, 76, 84, and 92.

[Item 6]

The monoclonal antibody or antibody fragment thereof according to any one of items 1 to 5, wherein the monoclonal antibody or antibody fragment thereof comprises:

a heavy chain variable region comprising CDR1 comprising an amino acid sequence selected from the group consisting of SEQ ID NOs: 47, 55, 63, 71, 79, and 87, CDR2 comprising an amino acid sequence selected from the group consisting of SEQ ID NOs: 48, 56, 64, 72, 80, and 88, and CDR3 comprising an amino acid sequence selected from the group consisting of SEQ ID NOs: 49, 57, 65, 73, 81, and 89; and/or
a light chain variable region comprising CDR1 comprising an amino acid sequence selected from the group consisting of SEQ ID NOs: 50, 58, 66, 74, 82, and 90, CDR2 comprising an amino acid sequence selected from the group consisting of SEQ ID NOs: 51, 59, 67, 75, 83, and 91, and CDR3 comprising an amino acid sequence selected from the group consisting of SEQ ID NOs: 52, 60, 68, 76, 84, and 92.

[Item 7]

The monoclonal antibody or antibody fragment thereof according to any one of items 1 to 6, wherein the monoclonal antibody or antibody fragment thereof comprises:

a heavy chain variable region comprising CDR1 comprising an amino acid sequence selected from the group consisting of SEQ ID NOs: 47, 55, and 63, CDR2 comprising an amino acid sequence selected from the group consisting of SEQ ID NOs: 48, 56, and 64, and CDR3 comprising an amino acid sequence selected from the group consisting of SEQ ID NOs: 49, 57, and 65; and/or
a light chain variable region comprising CDR1 comprising an amino acid sequence selected from the group consisting of SEQ ID NOs: 50, 58, and 66, CDR2 comprising an amino acid sequence selected from the group consisting of SEQ ID NOs: 51, 59, and 67, and CDR3 comprising an amino acid sequence selected from the group consisting of SEQ ID NOs: 52, 60, and 68.

[Item 8]

The monoclonal antibody or antibody fragment thereof according to items 1 to 6, wherein the monoclonal antibody or antibody fragment thereof comprises:

a heavy chain variable region comprising CDR1 comprising an amino acid sequence selected from the group consisting of SEQ ID NOs: 71, 79, and 87, CDR2 comprising an amino acid sequence selected from the group consisting of SEQ ID NOs: 72, 80, and 88, and CDR3 comprising an amino acid sequence selected from the group consisting of SEQ ID NOs: 73, 81, and 89; and/or
a light chain variable region comprising CDR1 comprising an amino acid sequence selected from the group consisting of SEQ ID NOs: 74, 82, and 90, CDR2 comprising an amino acid sequence selected from the group consisting of SEQ ID NOs: 75, 83, and 91, and CDR3 comprising an amino acid sequence selected from the group consisting of SEQ ID NOs: 76, 84, and 92.

[Item 9]

The monoclonal antibody or antibody fragment thereof according to any one of items 1 to 5, wherein the monoclonal antibody or antibody fragment thereof comprises:

(a) a heavy chain variable region comprising

CDR1 comprising an amino acid sequence of SEQ ID NO: 47 or an amino acid sequence in which one or two amino acid residues are independently substituted, deleted, added, or inserted in the amino acid sequence,
CDR2 comprising an amino acid sequence of SEQ ID NO: 48 or an amino acid sequence in which one or two amino acid residues are independently substituted, deleted, added, or inserted in the amino acid sequence, and
CDR3 comprising an amino acid sequence of SEQ ID NO: 49 or an amino acid sequence in which one or two amino acid residues are independently substituted, deleted, added, or inserted in the amino acid sequence, and/or
a light chain variable region comprising
CDR1 comprising an amino acid sequence of SEQ ID NO: 50 or an amino acid sequence in which one or two amino acid residues are independently substituted, deleted, added, or inserted in the amino acid sequence,
CDR2 comprising an amino acid sequence of SEQ ID NO: 51 or an amino acid sequence in which one or two amino acid residues are independently substituted, deleted, added, or inserted in the amino acid sequence, and

CDR3 comprising an amino acid sequence of SEQ ID NO: 52 or an amino acid sequence in which one or two amino acid residues are independently substituted, deleted, added, or inserted in the amino acid sequence;

(b) a heavy chain variable region comprising

CDR1 comprising an amino acid sequence of SEQ ID NO: 55 or an amino acid sequence in which one or two amino acid residues are independently substituted, deleted, added, or inserted in the amino acid sequence, CDR2 comprising an amino acid sequence of SEQ ID NO: 56 or an amino acid sequence in which one or two amino acid residues are independently substituted, deleted, added, or inserted in the amino acid sequence, and CDR3 comprising an amino acid sequence of SEQ ID NO: 57 or an amino acid sequence in which one or two amino acid residues are independently substituted, deleted, added, or inserted in the amino acid sequence, and/or
a light chain variable region comprising
CDR1 comprising an amino acid sequence of SEQ ID NO: 58 or an amino acid sequence in which one or two amino acid residues are independently substituted, deleted, added, or inserted in the amino acid sequence, CDR2 comprising an amino acid sequence of SEQ ID NO: 59 or an amino acid sequence in which one or two amino acid residues are independently substituted, deleted, added, or inserted in the amino acid sequence, and CDR3 comprising an amino acid sequence of SEQ ID NO: 60 or an amino acid sequence in which one or two amino acid residues are independently substituted, deleted, added, or inserted in the amino acid sequence;

(c) a heavy chain variable region comprising

CDR1 comprising an amino acid sequence of SEQ ID NO: 63 or an amino acid sequence in which one or two amino acid residues are independently substituted, deleted, added, or inserted in the amino acid sequence, CDR2 comprising an amino acid sequence of SEQ ID NO: 64 or an amino acid sequence in which one or two amino acid residues are independently substituted, deleted, added, or inserted in the amino acid sequence, and CDR3 comprising an amino acid sequence of SEQ ID NO: 65 or an amino acid sequence in which one or two amino acid residues are independently substituted, deleted, added, or inserted in the amino acid sequence, and/or
a light chain variable region comprising
CDR1 comprising an amino acid sequence of SEQ ID NO: 66 or an amino acid sequence in which one or two amino acid residues are independently substituted, deleted, added, or inserted in the amino acid sequence, CDR2 comprising an amino acid sequence of SEQ ID NO: 67 or an amino acid sequence in which one or two amino acid residues are independently substituted, deleted, added, or inserted in the amino acid sequence, and CDR3 comprising an amino acid sequence of SEQ ID NO: 68 or an amino acid sequence in which one or two amino acid residues are independently substituted, deleted, added, or inserted in the amino acid sequence;

(d) a heavy chain variable region comprising

CDR1 comprising an amino acid sequence of SEQ ID NO: 71 or an amino acid sequence in which one or two amino acid residues are independently substituted, deleted, added, or inserted in the amino acid sequence, CDR2 comprising an amino acid sequence of SEQ ID NO: 72 or an amino acid sequence in which one or two amino acid residues are independently substituted, deleted, added, or inserted in the amino acid sequence, and CDR3 comprising an amino acid sequence of SEQ ID NO: 73 or an amino acid sequence in which one or two amino acid residues are independently substituted, deleted, added, or inserted in the amino acid sequence, and/or
a light chain variable region comprising
CDR1 comprising an amino acid sequence of SEQ ID NO: 74 or an amino acid sequence in which one or two amino acid residues are independently substituted, deleted, added, or inserted in the amino acid sequence, CDR2 comprising an amino acid sequence of SEQ ID NO: 75 or an amino acid sequence in which one or two amino acid residues are independently substituted, deleted, added, or inserted in the amino acid sequence, and CDR3 comprising an amino acid sequence of SEQ ID NO: 76 or an amino acid sequence in which one or two amino acid residues are independently substituted, deleted, added, or inserted in the amino acid sequence;

(e) a heavy chain variable region comprising

CDR1 comprising an amino acid sequence of SEQ ID NO: 79 or an amino acid sequence in which one or two amino acid residues are independently substituted, deleted, added, or inserted in the amino acid sequence,

CDR2 comprising an amino acid sequence of SEQ ID NO: 80 or an amino acid sequence in which one or two amino acid residues are independently substituted, deleted, added, or inserted in the amino acid sequence, and CDR3 comprising an amino acid sequence of SEQ ID NO: 81 or an amino acid sequence in which one or two amino acid residues are independently substituted, deleted, added, or inserted in the amino acid sequence , and/or

a light chain variable region comprising

CDR1 comprising an amino acid sequence of SEQ ID NO: 82 or an amino acid sequence in which one or two amino acid residues are independently substituted, deleted, added, or inserted in the amino acid sequence, CDR2 comprising an amino acid sequence of SEQ ID NO: 83 or an amino acid sequence in which one or two amino acid residues are independently substituted, deleted, added, or inserted in the amino acid sequence, and CDR3 comprising an amino acid sequence of SEQ ID NO: 84 or an amino acid sequence in which one or two amino acid residues are independently substituted, deleted, added, or inserted in the amino acid sequence; or

(f) a heavy chain variable region comprising

CDR1 comprising an amino acid sequence of SEQ ID NO: 87 or an amino acid sequence in which one or two amino acid residues are independently substituted, deleted, added, or inserted in the amino acid sequence, CDR2 comprising an amino acid sequence of SEQ ID NO: 88 or an amino acid sequence in which one or two amino acid residues are independently substituted, deleted, added, or inserted in the amino acid sequence, and CDR3 comprising an amino acid sequence of SEQ ID NO: 89 or an amino acid sequence in which one or two amino acid residues are independently substituted, deleted, added, or inserted in the amino acid sequence, and/or

a light chain variable region comprising

CDR1 comprising an amino acid sequence of SEQ ID NO: 90 or an amino acid sequence in which one or two amino acid residues are independently substituted, deleted, added, or inserted in the amino acid sequence, CDR2 comprising an amino acid sequence of SEQ ID NO: 91 or an amino acid sequence in which one or two amino acid residues are independently substituted, deleted, added, or inserted in the amino acid sequence, and CDR3 comprising an amino acid sequence of SEQ ID NO: 92 or an amino acid sequence in which one or two amino acid residues are independently substituted, deleted, added, or inserted in the amino acid sequence.

[Item 10]

The monoclonal antibody or antibody fragment thereof according to any one of items 1 to 6, wherein the monoclonal antibody or antibody fragment thereof comprises:

(a') a heavy chain variable region comprising

CDR1 comprising an amino acid sequence of SEQ ID NO: 47, CDR2 comprising an amino acid sequence of SEQ ID NO: 48, and CDR3 comprising an amino acid sequence of SEQ ID NO: 49, and/or a light chain variable region comprising CDR1 comprising an amino acid sequence of SEQ ID NO: 50, CDR2 comprising an amino acid sequence of SEQ ID NO: 51, and CDR3 comprising an amino acid sequence of SEQ ID NO: 52;

(b') a heavy chain variable region comprising

CDR1 comprising an amino acid sequence of SEQ ID NO: 55, CDR2 comprising an amino acid sequence of SEQ ID NO: 56, and CDR3 comprising an amino acid sequence of SEQ ID NO: 57, and/or a light chain variable region comprising CDR1 comprising an amino acid sequence of SEQ ID NO: 58, CDR2 comprising an amino acid sequence of SEQ ID NO: 59, and CDR3 comprising an amino acid sequence of SEQ ID NO: 60;

(c') a heavy chain variable region comprising

CDR1 comprising an amino acid sequence of SEQ ID NO: 63,

CDR2 comprising an amino acid sequence of SEQ ID NO: 64, and
CDR3 comprising an amino acid sequence of SEQ ID NO: 65, and/or
a light chain variable region comprising
CDR1 comprising an amino acid sequence of SEQ ID NO: 66,
CDR2 comprising an amino acid sequence of SEQ ID NO: 67, and
CDR3 comprising an amino acid sequence of SEQ ID NO: 68;

(d') a heavy chain variable region comprising

CDR1 comprising an amino acid sequence of SEQ ID NO: 71,
CDR2 comprising an amino acid sequence of SEQ ID NO: 72, and
CDR3 comprising an amino acid sequence of SEQ ID NO: 73, and/or
a light chain variable region comprising
CDR1 comprising an amino acid sequence of SEQ ID NO: 74,
CDR2 comprising an amino acid sequence of SEQ ID NO: 75, and
CDR3 comprising an amino acid sequence of SEQ ID NO: 76;

(e') a heavy chain variable region comprising

CDR1 comprising an amino acid sequence of SEQ ID NO: 79,
CDR2 comprising an amino acid sequence of SEQ ID NO: 80, and
CDR3 comprising an amino acid sequence of SEQ ID NO: 81, and/or
a light chain variable region comprising
CDR1 comprising an amino acid sequence of SEQ ID NO: 82,
CDR2 comprising an amino acid sequence of SEQ ID NO: 83, and
CDR3 comprising an amino acid sequence of SEQ ID NO: 84; or

(f) a heavy chain variable region comprising

CDR1 comprising an amino acid sequence of SEQ ID NO: 87,
CDR2 comprising an amino acid sequence of SEQ ID NO: 88, and
CDR3 comprising an amino acid sequence of SEQ ID NO: 89, and/or
a light chain variable region comprising
CDR1 comprising an amino acid sequence of SEQ ID NO: 90,
CDR2 comprising an amino acid sequence of SEQ ID NO: 91, and
CDR3 comprising an amino acid sequence of SEQ ID NO: 92.

[Item 11]

The monoclonal antibody or antibody fragment thereof according to any one of items 1 to 6, wherein the monoclonal antibody or antibody fragment thereof comprises:

a heavy chain variable region comprising
CDR1 comprising an amino acid sequence of SEQ ID NO: 47,
CDR2 comprising an amino acid sequence of SEQ ID NO: 48, and
CDR3 comprising an amino acid sequence of SEQ ID NO: 49; and/or
a light chain variable region comprising
CDR1 comprising an amino acid sequence of SEQ ID NO: 50,
CDR2 comprising an amino acid sequence of SEQ ID NO: 51, and
CDR3 comprising an amino acid sequence of SEQ ID NO: 52.

[Item 12]

The monoclonal antibody or antibody fragment thereof according to any one of items 1 to 6, wherein the monoclonal antibody or antibody fragment thereof comprises:

a heavy chain variable region comprising
CDR1 comprising an amino acid sequence of SEQ ID NO: 55,

CDR2 comprising an amino acid sequence of SEQ ID NO: 56, and
CDR3 comprising an amino acid sequence of SEQ ID NO: 57; and/or
a light chain variable region comprising
CDR1 comprising an amino acid sequence of SEQ ID NO: 58,
CDR2 comprising an amino acid sequence of SEQ ID NO: 59, and
CDR3 comprising an amino acid sequence of SEQ ID NO: 60.

[Item 13]

The monoclonal antibody or antibody fragment thereof according to any one of items 1 to 6, wherein the monoclonal antibody or antibody fragment thereof comprises:

a heavy chain variable region comprising
CDR1 comprising an amino acid sequence of SEQ ID NO: 63,
CDR2 comprising an amino acid sequence of SEQ ID NO: 64, and
CDR3 comprising an amino acid sequence of SEQ ID NO: 65; and/or
a light chain variable region comprising
CDR1 comprising an amino acid sequence of SEQ ID NO: 66,
CDR2 comprising an amino acid sequence of SEQ ID NO: 67, and
CDR3 comprising an amino acid sequence of SEQ ID NO: 68.

[Item 14]

The monoclonal antibody or antibody fragment thereof according to any one of items 1 to 6, wherein the monoclonal antibody or antibody fragment thereof comprises:

a heavy chain variable region comprising
CDR1 comprising an amino acid sequence of SEQ ID NO: 71,
CDR2 comprising an amino acid sequence of SEQ ID NO: 72, and
CDR3 comprising an amino acid sequence of SEQ ID NO: 73; and/or
a light chain variable region comprising
CDR1 comprising an amino acid sequence of SEQ ID NO: 74,
CDR2 comprising an amino acid sequence of SEQ ID NO: 75, and
CDR3 comprising an amino acid sequence of SEQ ID NO: 76.

[Item 15]

The monoclonal antibody or antibody fragment thereof according to items 1 to 6, wherein the monoclonal antibody or antibody fragment thereof comprises:

a heavy chain variable region comprising
CDR1 comprising an amino acid sequence of SEQ ID NO: 79,
CDR2 comprising an amino acid sequence of SEQ ID NO: 80, and
CDR3 comprising an amino acid sequence of SEQ ID NO: 81; and/or
a light chain variable region comprising
CDR1 comprising an amino acid sequence of SEQ ID NO: 82,
CDR2 comprising an amino acid sequence of SEQ ID NO: 83, and
CDR3 comprising an amino acid sequence of SEQ ID NO: 84.

[Item 16]

The monoclonal antibody or antibody fragment thereof according to items 1 to 6, wherein the monoclonal antibody or antibody fragment thereof comprises:

a heavy chain variable region comprising
CDR1 comprising an amino acid sequence of SEQ ID NO: 87,
CDR2 comprising an amino acid sequence of SEQ ID NO: 88, and
CDR3 comprising an amino acid sequence of SEQ ID NO: 89; and/or

a light chain variable region comprising
CDR1 comprising an amino acid sequence of SEQ ID NO: 90,
CDR2 comprising an amino acid sequence of SEQ ID NO: 91, and
CDR3 comprising an amino acid sequence of SEQ ID NO: 92.

[Item 17]

The monoclonal antibody or antibody fragment thereof according to any one of items 1 to 16, wherein the monoclonal antibody or antibody fragment thereof comprises:

(a") a heavy chain variable region comprising an amino acid sequence having a sequence identity of at least 90% with an amino acid sequence of SEQ ID NO: 53 and/or a light chain variable region comprising an amino acid sequence having a sequence identity of at least 90% with an amino acid sequence of SEQ ID NO: 54;
(b") a heavy chain variable region comprising an amino acid sequence having a sequence identity of at least 90% with an amino acid sequence of SEQ ID NO: 61 and/or a light chain variable region comprising an amino acid sequence having a sequence identity of at least 90% with an amino acid sequence of SEQ ID NO: 62;
(c") a heavy chain variable region comprising an amino acid sequence having a sequence identity of at least 90% with an amino acid sequence of SEQ ID NO: 69 and/or a light chain variable region comprising an amino acid sequence having a sequence identity of at least 90% with an amino acid sequence of SEQ ID NO: 70;
(d") a heavy chain variable region comprising an amino acid sequence having a sequence identity of at least 90% with an amino acid sequence of SEQ ID NO: 77 and/or a light chain variable region comprising an amino acid sequence having a sequence identity of at least 90% with an amino acid sequence of SEQ ID NO: 78;
(e") a heavy chain variable region comprising an amino acid sequence having a sequence identity of at least 90% with an amino acid sequence of SEQ ID NO: 85 and/or a light chain variable region comprising an amino acid sequence having a sequence identity of at least 90% with an amino acid sequence of SEQ ID NO: 86; or
(f') a heavy chain variable region comprising an amino acid sequence having a sequence identity of at least 90% with an amino acid sequence of SEQ ID NO: 93 and/or a light chain variable region comprising an amino acid sequence having a sequence identity of at least 90% with an amino acid sequence of SEQ ID NO: 94.

[Item 18]

The monoclonal antibody or antibody fragment thereof according to any one of items 1 to 17, wherein the monoclonal antibody or antibody fragment thereof comprises:

(a''') a heavy chain variable region comprising an amino acid sequence of SEQ ID NO: 53 and/or a light chain variable region comprising an amino acid sequence of SEQ ID NO: 54;
(b''') a heavy chain variable region comprising an amino acid sequence of SEQ ID NO: 61 and/or a light chain variable region comprising an amino acid sequence of SEQ ID NO: 62;
(c''') a heavy chain variable region comprising an amino acid sequence of SEQ ID NO: 69 and/or a light chain variable region comprising an amino acid sequence of SEQ ID NO: 70;
(d''') a heavy chain variable region comprising an amino acid sequence of SEQ ID NO: 77 and/or a light chain variable region comprising an amino acid sequence of SEQ ID NO: 78;
(e''') a heavy chain variable region comprising an amino acid sequence of SEQ ID NO: 85 and/or a light chain variable region comprising an amino acid sequence of SEQ ID NO: 86; or
(f''') a heavy chain variable region comprising an amino acid sequence of SEQ ID NO: 93 and/or a light chain variable region comprising an amino acid sequence of SEQ ID NO: 94.

[Item 19]

The monoclonal antibody or antibody fragment thereof according to any one of items 1 to 18, wherein the monoclonal antibody or antibody fragment thereof comprises:

(a''') a heavy chain variable region comprising an amino acid sequence of SEQ ID NO: 53 and/or a light chain variable region comprising an amino acid sequence of SEQ ID NO: 54;
(b''') a heavy chain variable region comprising an amino acid sequence of SEQ ID NO: 61 and/or a light chain variable region comprising an amino acid sequence of SEQ ID NO: 62; or
(c''') a heavy chain variable region comprising an amino acid sequence of SEQ ID NO: 69 and/or a light chain variable region comprising an amino acid sequence of SEQ ID NO: 70.

[Item 20]

The monoclonal antibody or antibody fragment thereof according to any one of items 1 to 18, wherein the monoclonal antibody or antibody fragment thereof comprises:

(d''') a heavy chain variable region comprising an amino acid sequence of SEQ ID NO: 77 and/or a light chain variable region comprising an amino acid sequence of SEQ ID NO: 78;
(e''') a heavy chain variable region comprising an amino acid sequence of SEQ ID NO: 85 and/or a light chain variable region comprising an amino acid sequence of SEQ ID NO: 86; or
(f'') a heavy chain variable region comprising an amino acid sequence of SEQ ID NO: 93 and/or a light chain variable region comprising an amino acid sequence of SEQ ID NO: 94.

[Item 21]

The monoclonal antibody or antibody fragment thereof according to any one of items 1 to 10, wherein the monoclonal antibody or antibody fragment thereof comprises a heavy chain variable region comprising an amino acid sequence of SEQ ID NO: 53 and/or a light chain variable region comprising an amino acid sequence of SEQ ID NO: 54.

[Item 22]

The monoclonal antibody or antibody fragment thereof according to any one of items 1 to 10, wherein the monoclonal antibody or antibody fragment thereof comprises a heavy chain variable region comprising an amino acid sequence of SEQ ID NO: 61 and/or a light chain variable region comprising an amino acid sequence of SEQ ID NO: 62.

[Item 23]

The monoclonal antibody or antibody fragment thereof according to any one of items 1 to 10, wherein the monoclonal antibody or antibody fragment thereof comprises a heavy chain variable region comprising an amino acid sequence of SEQ ID NO: 69 and/or a light chain variable region comprising an amino acid sequence of SEQ ID NO: 70.

[Item 24]

The monoclonal antibody or antibody fragment thereof according to any one of items 1 to 10, wherein the monoclonal antibody or antibody fragment thereof comprises a heavy chain variable region comprising an amino acid sequence of SEQ ID NO: 77 and/or a light chain variable region comprising an amino acid sequence of SEQ ID NO: 78.

[Item 25]

The monoclonal antibody or antibody fragment thereof according to any one of items 1 to 10, wherein the monoclonal antibody or antibody fragment thereof comprises a heavy chain variable region comprising an amino acid sequence of SEQ ID NO: 85 and/or a light chain variable region comprising an amino acid sequence of SEQ ID NO: 86.

[Item 26]

The monoclonal antibody or antibody fragment thereof according to any one of items 1 to 10, wherein the monoclonal antibody or antibody fragment thereof comprises a heavy chain variable region comprising an amino acid sequence of SEQ ID NO: 93 and/or a light chain variable region comprising an amino acid sequence of SEQ ID NO: 94.

[Item 27]

A monoclonal antibody or an antibody fragment thereof, wherein the monoclonal antibody or antibody fragment thereof competes with the monoclonal antibody or antibody fragment thereof according to any one of items 5 to 26 for binding to the HA stem region.

[Item 28]

The monoclonal antibody or antibody fragment thereof according to any one of items 1 to 27, wherein the monoclonal antibody or antibody fragment thereof binds to HA stem regions of two or more influenza virus HA subtypes.

[Item 29]

The monoclonal antibody or antibody fragment thereof according to any one of items 1 to 28, wherein the monoclonal antibody or antibody fragment thereof binds to an HA stem region of an influenza A virus HA subtype belonging to phylogenetic group 1, and binds to an HA stem region of an influenza A virus HA subtype belonging to phylogenetic group 2.

[Item 29']

The monoclonal antibody or antibody fragment thereof according to any one of items 15, 16, 25, and 26, wherein the monoclonal antibody or antibody fragment thereof binds to an HA stem region of an influenza A virus HA subtype belonging to phylogenetic group 1, and binds to an HA stem region of an influenza A virus HA subtype belonging to phylogenetic group 2.

[Item 30]

The monoclonal antibody or antibody fragment thereof according to any one of items 1 to 29, wherein the monoclonal antibody or antibody fragment thereof has protective capacity against infection for an influenza A virus.

[Item 31]

The monoclonal antibody or antibody fragment thereof according to any one of items 1 to 30, wherein the monoclonal antibody or antibody fragment thereof is a mouse antibody, chimeric antibody, humanized antibody, or fully human antibody, or a fragment thereof.

[Item 32]

The monoclonal antibody or antibody fragment thereof according to any one of items 1 to 7, 9 to 13, 17 to 19, 21 to 23, and 27 to 31, wherein the monoclonal antibody or antibody fragment thereof is a mouse antibody or a fragment thereof.

[Item 32']

The monoclonal antibody or antibody fragment thereof according to any one of items 1 to 7, 9 to 13, 17 to 19, 21 to 23, and 27 to 31, wherein the monoclonal antibody or antibody fragment thereof is a chimeric antibody or a fragment thereof.

[Item 33]

The monoclonal antibody or antibody fragment thereof according to any one of items 1 to 18, 20, and 24 to 31, wherein the monoclonal antibody or antibody fragment thereof is a fully human antibody or a fragment thereof.

[Item 34]

The monoclonal antibody or antibody fragment thereof according to any one of items 1 to 33, comprising a modified constant region.

[Item 35]

The monoclonal antibody or antibody fragment thereof according to any one of items 1 to 34, wherein a drug has been bound to the monoclonal antibody or antibody fragment thereof.

[Item 36]

A pharmaceutical composition for treating or preventing an influenza A virus infection, the pharmaceutical composition comprising the monoclonal antibody or antibody fragment thereof according to any one of items 1 to 35.

[Item 37]

The pharmaceutical composition according to item 36, wherein the monoclonal antibody or antibody fragment thereof is the monoclonal antibody or antibody fragment thereof according to any one of items 1 to 18, 20, 24 to 31, and 33 to 35.

[Item 38]

The pharmaceutical composition according to item 36, wherein the monoclonal antibody or antibody fragment thereof is the monoclonal antibody or antibody fragment thereof according to any one of items 8, 14 to 16, 20, and 24 to 26.

[Item 39]

The monoclonal antibody or antibody fragment thereof according to any one of items 1 to 35, for use in treating or preventing an influenza A virus infection.

[Item 40]

A method for treating or preventing an influenza A virus infection, the method including administering the monoclonal antibody or antibody fragment thereof according to any one of items 1 to 35 to a subject.

[Item 41]

Use of the monoclonal antibody or antibody fragment thereof according to any one of items 1 to 35 in production of a medicament for treating or preventing an influenza A virus infection.

[Item 42]

Use of the monoclonal antibody or antibody fragment thereof according to any one of items 1 to 35 for treating or preventing an influenza A virus infection.

[Item 43]

The pharmaceutical composition according to any one of items 36 to 38, the monoclonal antibody or antibody fragment thereof according to item 39, the method according to item 40, the use according to item 41, or the use according to item 42, wherein the influenza A virus infection is an infection with an influenza A virus HA subtype belonging to phylogenetic group 1 and an infection with an influenza A virus HA subtype belonging to phylogenetic group 2.

[Item 44]

The pharmaceutical composition according to any one of items 36 to 38, the monoclonal antibody or antibody fragment thereof according to item 39, the method according to item 40, the use according to item 41, or the use according to item 42, wherein the influenza A virus infection is an infection with an influenza A virus HA subtype belonging to phylogenetic group 1 and/or an infection with an influenza A virus HA subtype belonging to phylogenetic group 2.

[Item 45]

The pharmaceutical composition according to item 36, the monoclonal antibody or antibody fragment thereof according to item 39, the method according to item 40, the use according to item 41, or the use according to item 42, wherein the monoclonal antibody or antibody fragment thereof is the monoclonal antibody or antibody fragment thereof according to any one of items 1 to 11, 14 to 21, and 24 to 35, and the influenza virus infection is an infection with an influenza A virus HA subtype belonging to phylogenetic group 2.

[Item 46]

The pharmaceutical composition, monoclonal antibody or antibody fragment thereof, method, or use according to item 45, wherein the influenza virus infection is an infection with an influenza A virus H3 or H7 subtype.

[Item 47]

The pharmaceutical composition, monoclonal antibody or antibody fragment thereof, method, or use according to item 45, wherein the influenza virus infection is an infection with an influenza A virus H3 subtype.

[Item 48]

The pharmaceutical composition, monoclonal antibody or antibody fragment thereof, method, or use according to item 45, wherein the influenza virus infection is an infection with an influenza A virus H7 subtype.

[Item 49]

The pharmaceutical composition according to item 36, the monoclonal antibody or antibody fragment thereof according to item 39, the method according to item 40, the use according to item 41, or the use according to item 42, wherein the monoclonal antibody or antibody fragment thereof is the monoclonal antibody or antibody fragment thereof according to any one of items 1 to 10, 12, 13, 15 to 20, 22, 23, and 25 to 35, and the influenza virus infection is an infection with an influenza A virus of an HA subtype belonging to phylogenetic group 1.

[Item 50]

The pharmaceutical composition, monoclonal antibody or antibody fragment thereof, method, or use according to item 49, wherein the influenza virus infection is an infection with an influenza A virus H1 or H5 subtype.

[Item 51]

The pharmaceutical composition, monoclonal antibody or antibody fragment thereof, method, or use according to item 49, wherein the influenza virus infection is an infection with an influenza A virus H1 subtype.

[Item 52]

The pharmaceutical composition, monoclonal antibody or antibody fragment thereof, method, or use according to item 49, wherein the influenza virus infection is an infection with an influenza A virus H5 subtype.

[Item 53]

A method for detecting or quantifying a membrane fusion-type influenza HA antigen, the method comprising bringing the monoclonal antibody or antibody fragment thereof according to any one of items 1 to 35 into contact with a sample.

[Item 54]

The method according to item 53, wherein the monoclonal antibody or antibody fragment thereof is the monoclonal antibody or antibody fragment thereof according to any one of items 1 to 4, comprising:

(a) a heavy chain variable region comprising

CDR1 comprising an amino acid sequence of SEQ ID NO: 47 or an amino acid sequence in which one or two amino acid residues are independently substituted, deleted, added, or inserted in the amino acid sequence, CDR2 comprising an amino acid sequence of SEQ ID NO: 48 or an amino acid sequence in which one or two amino acid residues are independently substituted, deleted, added, or inserted in the amino acid sequence, and CDR3 comprising an amino acid sequence of SEQ ID NO: 49 or an amino acid sequence in which one or two amino acid residues are independently substituted, deleted, added, or inserted in the amino acid sequence, and/or
a light chain variable region comprising
CDR1 comprising an amino acid sequence of SEQ ID NO: 50 or an amino acid sequence in which one or two amino acid residues are independently substituted, deleted, added, or inserted in the amino acid sequence, CDR2 comprising an amino acid sequence of SEQ ID NO: 51 or an amino acid sequence in which one or two amino acid residues are independently substituted, deleted, added, or inserted in the amino acid sequence, and

CDR3 comprising an amino acid sequence of SEQ ID NO: 52 or an amino acid sequence in which one or two amino acid residues are independently substituted, deleted, added, or inserted in the amino acid sequence;

(b) a heavy chain variable region comprising

CDR1 comprising an amino acid sequence of SEQ ID NO: 55 or an amino acid sequence in which one or two amino acid residues are independently substituted, deleted, added, or inserted in the amino acid sequence, CDR2 comprising an amino acid sequence of SEQ ID NO: 56 or an amino acid sequence in which one or two amino acid residues are independently substituted, deleted, added, or inserted in the amino acid sequence, and CDR3 comprising an amino acid sequence of SEQ ID NO: 57 or an amino acid sequence in which one or two amino acid residues are independently substituted, deleted, added, or inserted in the amino acid sequence, and/or
a light chain variable region comprising
CDR1 comprising an amino acid sequence of SEQ ID NO: 58 or an amino acid sequence in which one or two amino acid residues are independently substituted, deleted, added, or inserted in the amino acid sequence, CDR2 comprising an amino acid sequence of SEQ ID NO: 59 or an amino acid sequence in which one or two amino acid residues are independently substituted, deleted, added, or inserted in the amino acid sequence, and CDR3 comprising an amino acid sequence of SEQ ID NO: 60 or an amino acid sequence in which one or two amino acid residues are independently substituted, deleted, added, or inserted in the amino acid sequence;

(c) a heavy chain variable region comprising

CDR1 comprising an amino acid sequence of SEQ ID NO: 63 or an amino acid sequence in which one or two amino acid residues are independently substituted, deleted, added, or inserted in the amino acid sequence, CDR2 comprising an amino acid sequence of SEQ ID NO: 64 or an amino acid sequence in which one or two amino acid residues are independently substituted, deleted, added, or inserted in the amino acid sequence, and CDR3 comprising an amino acid sequence of SEQ ID NO: 65 or an amino acid sequence in which one or two amino acid residues are independently substituted, deleted, added, or inserted in the amino acid sequence, and/or
a light chain variable region comprising
CDR1 comprising an amino acid sequence of SEQ ID NO: 66 or an amino acid sequence in which one or two amino acid residues are independently substituted, deleted, added, or inserted in the amino acid sequence, CDR2 comprising an amino acid sequence of SEQ ID NO: 67 or an amino acid sequence in which one or two amino acid residues are independently substituted, deleted, added, or inserted in the amino acid sequence, and CDR3 comprising an amino acid sequence of SEQ ID NO: 68 or an amino acid sequence in which one or two amino acid residues are independently substituted, deleted, added, or inserted in the amino acid sequence;

(d) a heavy chain variable region comprising

CDR1 comprising an amino acid sequence of SEQ ID NO: 71 or an amino acid sequence in which one or two amino acid residues are independently substituted, deleted, added, or inserted in the amino acid sequence, CDR2 comprising an amino acid sequence of SEQ ID NO: 72 or an amino acid sequence in which one or two amino acid residues are independently substituted, deleted, added, or inserted in the amino acid sequence, and CDR3 comprising an amino acid sequence of SEQ ID NO: 73 or an amino acid sequence in which one or two amino acid residues are independently substituted, deleted, added, or inserted in the amino acid sequence, and/or
a light chain variable region comprising
CDR1 comprising an amino acid sequence of SEQ ID NO: 74 or an amino acid sequence in which one or two amino acid residues are independently substituted, deleted, added, or inserted in the amino acid sequence, CDR2 comprising an amino acid sequence of SEQ ID NO: 75 or an amino acid sequence in which one or two amino acid residues are independently substituted, deleted, added, or inserted in the amino acid sequence, and CDR3 comprising an amino acid sequence of SEQ ID NO: 76 or an amino acid sequence in which one or two amino acid residues are independently substituted, deleted, added, or inserted in the amino acid sequence;

(e) a heavy chain variable region comprising

CDR1 comprising an amino acid sequence of SEQ ID NO: 79 or an amino acid sequence in which one or two amino acid residues are independently substituted, deleted, added, or inserted in the amino acid sequence,

CDR2 comprising an amino acid sequence of SEQ ID NO: 80 or an amino acid sequence in which one or two amino acid residues are independently substituted, deleted, added, or inserted in the amino acid sequence, and CDR3 comprising an amino acid sequence of SEQ ID NO: 81 or an amino acid sequence in which one or two amino acid residues are independently substituted, deleted, added, or inserted in the amino acid sequence, and/or
a light chain variable region comprising
CDR1 comprising an amino acid sequence of SEQ ID NO: 82 or an amino acid sequence in which one or two amino acid residues are independently substituted, deleted, added, or inserted in the amino acid sequence,
CDR2 comprising an amino acid sequence of SEQ ID NO: 83 or an amino acid sequence in which one or two amino acid residues are independently substituted, deleted, added, or inserted in the amino acid sequence, and
CDR3 comprising an amino acid sequence of SEQ ID NO: 84 or an amino acid sequence in which one or two amino acid residues are independently substituted, deleted, added, or inserted in the amino acid sequence;

(f) a heavy chain variable region comprising

CDR1 comprising an amino acid sequence of SEQ ID NO: 87 or an amino acid sequence in which one or two amino acid residues are independently substituted, deleted, added, or inserted in the amino acid sequence,
CDR2 comprising an amino acid sequence of SEQ ID NO: 88 or an amino acid sequence in which one or two amino acid residues are independently substituted, deleted, added, or inserted in the amino acid sequence, and
CDR3 comprising an amino acid sequence of SEQ ID NO: 89 or an amino acid sequence in which one or two amino acid residues are independently substituted, deleted, added, or inserted in the amino acid sequence, and/or
a light chain variable region comprising
CDR1 comprising an amino acid sequence of SEQ ID NO: 90 or an amino acid sequence in which one or two amino acid residues are independently substituted, deleted, added, or inserted in the amino acid sequence,
CDR2 comprising an amino acid sequence of SEQ ID NO: 91 or an amino acid sequence in which one or two amino acid residues are independently substituted, deleted, added, or inserted in the amino acid sequence, and
CDR3 comprising an amino acid sequence of SEQ ID NO: 92 or an amino acid sequence in which one or two amino acid residues are independently substituted, deleted, added, or inserted in the amino acid sequence; or

(g) a heavy chain variable region comprising

CDR1 comprising an amino acid sequence of SEQ ID NO: 39 or an amino acid sequence in which one or two amino acid residues are independently substituted, deleted, added, or inserted in the amino acid sequence,
CDR2 comprising an amino acid sequence of SEQ ID NO: 40 or an amino acid sequence in which one or two amino acid residues are independently substituted, deleted, added, or inserted in the amino acid sequence, and
CDR3 comprising an amino acid sequence of SEQ ID NO: 41 or an amino acid sequence in which one or two amino acid residues are independently substituted, deleted, added, or inserted in the amino acid sequence, and/or
a light chain variable region comprising
CDR1 comprising an amino acid sequence of SEQ ID NO: 42 or an amino acid sequence in which one or two amino acid residues are independently substituted, deleted, added, or inserted in the amino acid sequence,
CDR2 comprising an amino acid sequence of SEQ ID NO: 43 or an amino acid sequence in which one or two amino acid residues are independently substituted, deleted, added, or inserted in the amino acid sequence, and
CDR3 comprising an amino acid sequence of SEQ ID NO: 44 or an amino acid sequence in which one or two amino acid residues are independently substituted, deleted, added, or inserted in the amino acid sequence,

or a combination of any of them.

[Item 55]

The method according to item 53 or 54, wherein the monoclonal antibody or antibody fragment is a combination of a first antibody and a second antibody, wherein

the first antibody is: the monoclonal antibody or antibody fragment according to any one of items 1 to 35; or a monoclonal antibody or an antibody fragment, with a heavy chain variable region comprising CDR1 comprising an amino acid sequence of SEQ ID NO: 39, CDR2 comprising an amino acid sequence of SEQ ID NO: 40, and CDR3 comprising an amino acid sequence of SEQ ID NO: 41, and a light chain variable region comprising CDR1 comprising an amino acid sequence of SEQ ID NO: 42, CDR2 comprising an amino acid sequence of SEQ ID NO: 43, and CDR3 comprising an amino acid sequence of SEQ ID NO: 44, and

the second antibody is a monoclonal antibody or an antibody fragment, wherein the monoclonal antibody or antibody fragment differs from the first antibody, and binds to a peptide that consists of an amino acid sequence selected from the group consisting of SEQ ID NOs: 1 to 18 and is capable of being bound by the first antibody.

Effects of Invention

The present disclosure provides a monoclonal antibody or an antibody fragment thereof, wherein the monoclonal antibody or antibody fragment thereof binds to an HA stem region of an influenza virus. The present disclosure also provides a pharmaceutical composition for treating or preventing an influenza virus infection and the like, and a method for detecting or quantifying a universal influenza vaccine antigen derived from hemagglutinin, for each of which the monoclonal antibody or antibody fragment thereof according to the present disclosure is used.

Brief Description of Drawings

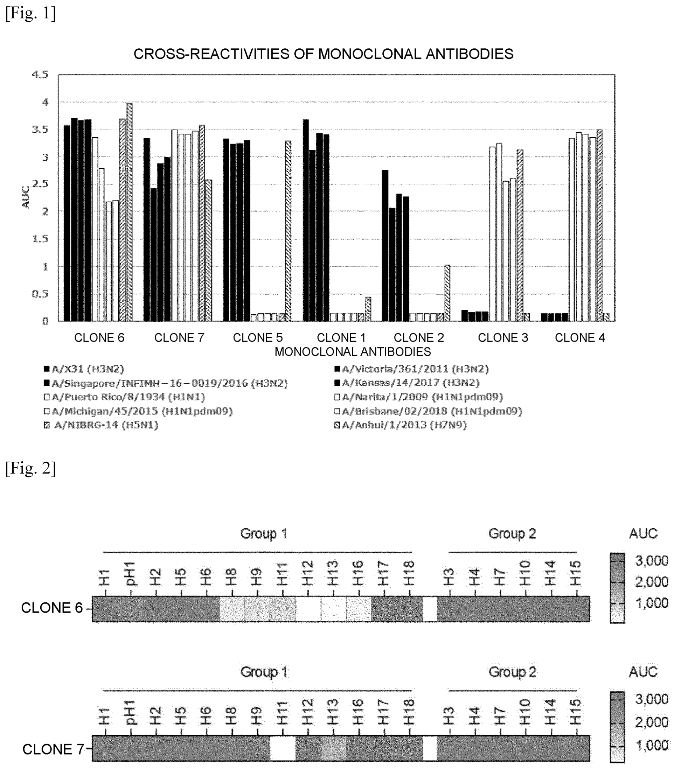

[Figure 1] Figure 1 shows areas under the curve (AUCs) obtained from curves for different antibodies, clones 1 to 7, in graphs prepared therefor with the ordinate representing ELISA measurements (absorbance) and the abscissa representing the reciprocals of antibody concentrations. From the left: A/X-31 (H3N2), A/Victoria/361/2011 (H3N2), A/Singapore/INFIMH-16-0019/2016 (H3N2), A/Kansas/14/2017 (H3N2), A/Puerto Rico/8/1934 (H1N1), A/Narita/1/2009 (H1N1pdm09), A/Michigan/45/2015 (H1N1pdm09), A/Brisbane/02/2018 (H1N1pdm09), ANIBRG-14 (H5N1), A/Anhui/1/2013 (H7N9).

[Figure 2] Figure 2 shows a series of heat maps for AUCs calculated from ELISA measurements (absorbance) for clones 6 and 7.

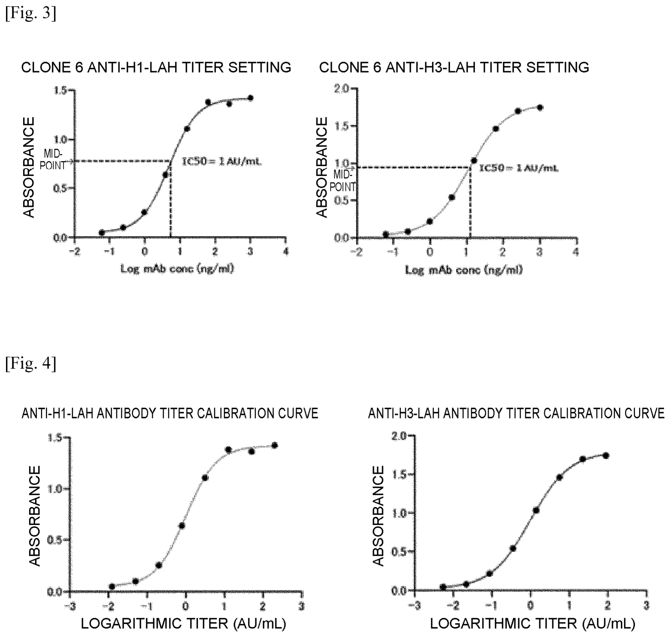

[Figure 3] Figure 3 is a series of graphs obtained by plotting for clone 6 with the ordinate representing absorbance and the abscissa representing logarithmic values of antibody concentration, in each graph of which the antibody concentration at the midpoint (IC50) of the resulting sigmoid curve was set to 1 arbitrary titer (AU/mL).

[Figure 4] Figure 4 is a series of graphs obtained by plotting with the ordinate representing absorbance and the abscissa representing logarithmic values of titer for calculating titers for clone 5.

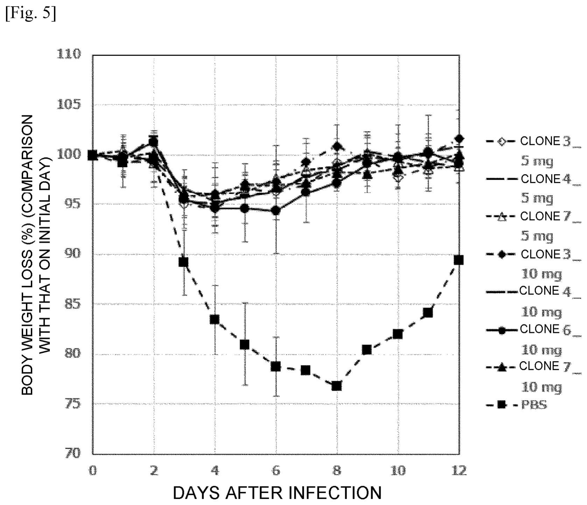

[Figure 5] Figure 5 is a graph showing protective capacity against infection for strain H1N1pdm09 for a PBS administration group and monoclonal antibody (clone 3, clone 4, clone 6, clone 7) administration groups with the ordinate representing body weight variation rates and the abscissa representing days after infection.

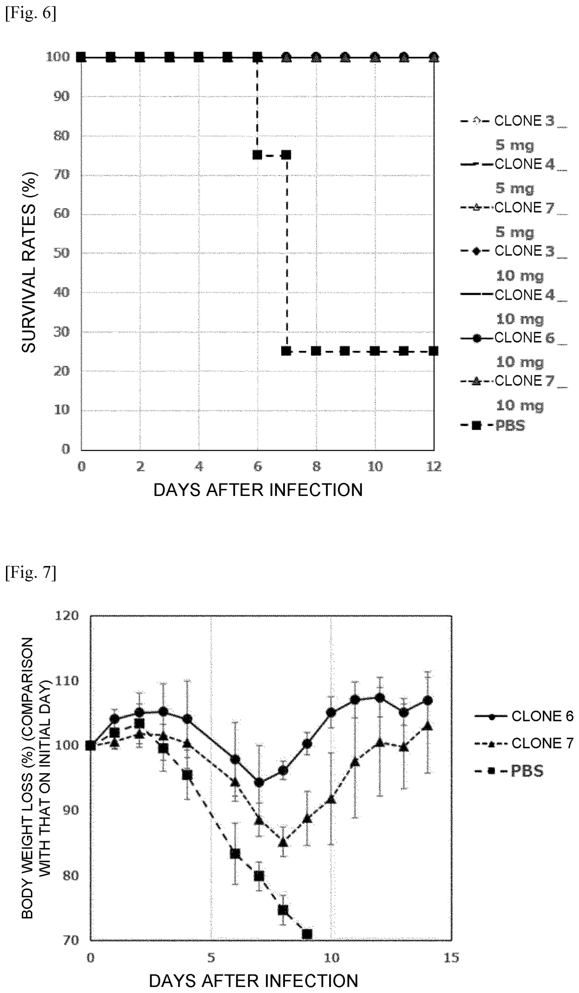

[Figure 6] Figure 6 is a graph showing protective capacity against infection for strain H1N1pdm09 for a PBS administration group and monoclonal antibody (clone 3, clone 4, clone 6, clone 7) administration groups with the ordinate representing survival rates and the abscissa representing days after infection.

[Figure 7] Figure 7 is a graph showing protective capacity against infection for strain H3N2 for a PBS administration group and monoclonal antibody (clone 6, clone 7) administration groups with the ordinate representing body weight variation rates and the abscissa representing days after infection.

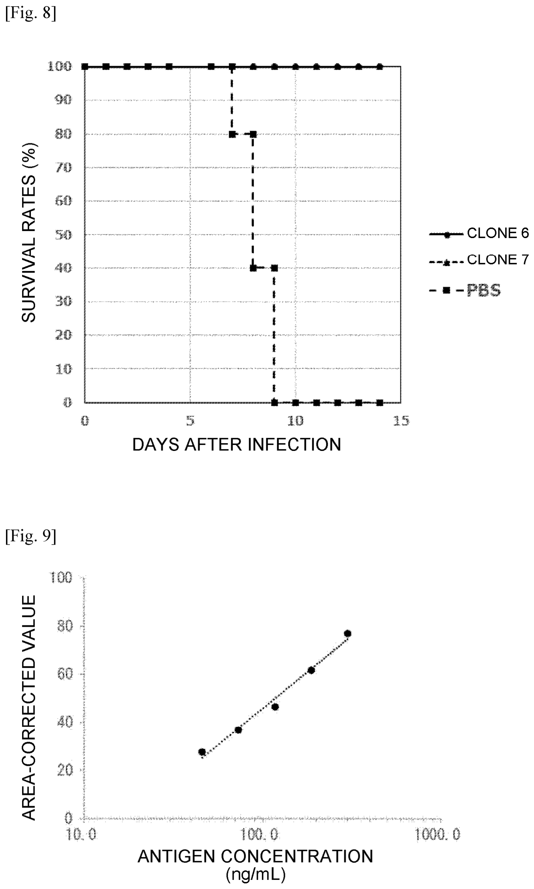

[Figure 8] Figure 8 is a graph showing protective capacity against infection for strain H3N2 for a PBS administration group and monoclonal antibody (clone 6, clone 7) administration groups with the ordinate representing survival rates and the abscissa representing days after infection.

[Figure 9] Figure 9 shows detection of an H1 membrane fusion-type influenza HA antigen and measurement results for diluted solutions containing the H1 membrane fusion-type influenza HA antigen (obtained by serially diluting 300 ng/mL solution at a common ratio of 1.6; 45.8 ng/mL to 300 ng/mL) by slot blotting with clone 3.

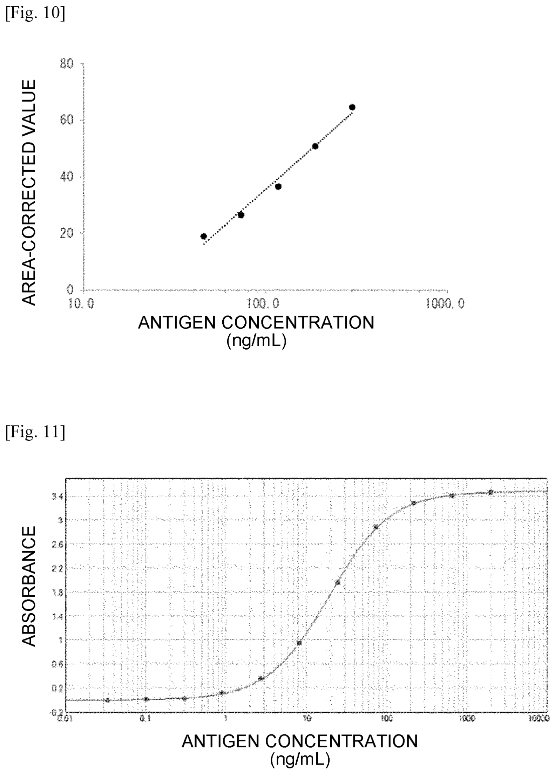

[Figure 10] Figure 10 shows detection of an H3 membrane fusion-type influenza HA antigen and measurement results for diluted solutions containing the H3 membrane fusion-type influenza HA antigen (obtained by serially diluting 300 ng/mL solution at a common ratio of 1.6; 45.8 ng/mL to 300 ng/mL) by slot blotting with clone 1.

[Figure 11] Figure 11 shows detection of an H1 membrane fusion-type influenza HA antigen and measurement results for diluted solutions containing the H1 membrane fusion-type influenza HA antigen (obtained by serially diluting 2000 ng/mL solution at a common ratio of 3; 34 pg/mL to 2000 ng/mL) by ELISA with clone 3.

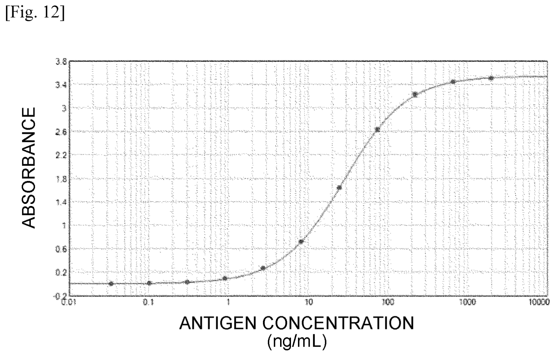

[Figure 12] Figure 12 shows detection of an H3 membrane fusion-type influenza HA antigen and measurement results for diluted solutions containing the H3 membrane fusion-type influenza HA antigen (obtained by serially diluting 2000 ng/mL solution at a common ratio of 3; 34 pg/mL to 2000 ng/mL) by ELISA with clone 1.

Description of Embodiments

Herein, expressions with abbreviations for amino acids, (poly)peptides, (poly)nucleotides, and the like are in accordance with the definitions by IUPAC-IUB [IUPAC-IUB Communication on Biological Nomenclature, Eur. J. Biochem., 138:9

(1984)], "Guidelines for Describing the Specification etc. including Nucleotide Sequences or Amino Acid Sequences" (edited by Japan Patent Office), and conventional signs in the field.

In the present disclosure, the statement that an antibody/antibody fragment binds to a specific HA stem region (or LAH region) means that an epitope for the antibody/antibody fragment is present in the region. Whether an antibody/antibody fragment binds to a specific HA stem region (or LAH region) can be confirmed by a conventional method, for example, by examining whether the antibody/antibody fragment binds to a peptide (e.g., a synthetic peptide) corresponding to the amino acid sequence of the specific HA stem region (or LAH region) or corresponding to a part of the specific amino acid sequence, as in a method described in Examples in the present application. To a peptide to be used for binding assay, an amino acid sequence such as a signal peptide and a tag for purification may be added unless the amino acid sequence affects results of the binding assay.

In the present disclosure, the statement that an antibody/antibody fragment binds to a peptide consisting of a specific amino acid sequence means that an epitope for the antibody/antibody fragment is present in a peptide consisting of the amino acid sequence. Whether an antibody/antibody fragment binds to a peptide consisting of a specific amino acid sequence can be confirmed by a conventional method, for example, by examining whether the antibody/antibody fragment binds to a peptide (e.g., a synthetic peptide) corresponding to the specific amino acid sequence or corresponding to a part of the specific amino acid sequence, as in a method described in Examples in the present application. To a peptide to be used for binding assay, an amino acid sequence such as a signal peptide and a tag for purification may be added unless the amino acid sequence affects results of the binding assay.

Herein, an epitope is a region of an antigen to which an antibody binds. Each epitope may contain a chemically active surface group, for example, an amino acid, a sugar side chain, a phosphoryl group, or a sulfonyl group, and may have a characteristic three-dimensional structure and/or a characteristic electric charge. Herein, if a monoclonal antibody or an antibody fragment preferentially recognizes a certain antigen or epitope in a mixture of antigenic substances including proteins and peptides, the monoclonal antibody or antibody fragment can be said to specifically bind to the antigen or epitope.

Epitope analysis can be performed with various methods (EpitopeMapping Protocols/Second Edition, Mike Schutkowski, Ulrich Reineke, Ann NY AcadSci. 2010 Jan; 1183:267-87.). More detailed analysis of epitopes can be performed, for example, by Western blotting with polypeptide fragments.

The present disclosure provides, in an aspect, a monoclonal antibody that binds to a hemagglutinin stem region (HA stem region) of an influenza A virus (hereinafter, also referred to as "the monoclonal antibody according to the present disclosure") or such an antibody fragment (hereinafter, also referred to as "the antibody fragment according to the present disclosure"). Although the amino acid sequence of the HA stem region may differ, for example, among different HA subtypes and strains, the statement that "an antibody/antibody fragment binds to an HA stem region of an influenza virus" in the present disclosure means that an antibody/antibody fragment is capable of binding to at least one HA stem region.

Whether an antibody/antibody fragment binds to an HA stem region of an influenza virus can be confirmed by a conventional method, for example, by examining whether the antibody/antibody fragment binds to a peptide (e.g., a synthetic peptide) corresponding to the amino acid sequence of an HA stem region or corresponding to a part thereof, as in a method described in Examples in the present application.

In an embodiment, the epitope for the monoclonal antibody/antibody fragment according to the present disclosure is composed of 5 to 20 amino acid residues present in the amino acid sequence of an HA stem region.

In the present disclosure, the term "influenza virus" can mean an influenza A virus.

Examples of the amino acid sequence of an HA stem region include the amino acid sequences of SEQ ID NOs: 1 to 18. The amino acid sequences of SEQ ID NOs: 1 to 18 are the respective amino acid sequences of the HA stem regions of specific strains, H1 to 18 subtypes. Although being a highly conserved region, the amino acid sequence of an HA stem region of an influenza A virus can slightly differ, for example, among different subtypes and strains, and a monoclonal antibody or antibody fragment thereof that binds to the amino acid sequence of an HA stem region other than the amino acid sequences of SEQ ID NOs: 1 to 18 can also be included in the definition of the monoclonal antibody/antibody fragment according to the present disclosure.

In an embodiment, the monoclonal antibody/antibody fragment according to the present disclosure binds to a peptide consisting of an amino acid sequence selected from the group consisting of SEQ ID NOs: 1 to 18 or an amino acid sequence having a sequence identity of at least 50% (e.g., at least 60%, at least 70%, at least 80%, at least 90%, at least 95%, at least 97%, at least 99%) with an amino acid sequence selected from the group consisting of SEQ ID NOs: 1 to 18.

It is known that an α-helix structure of approximately 55 residues (Long alpha helix (LAH) region) is present in HA stem regions. Accordingly, the meaning of the statement "bind to an HA stem region of an influenza A virus" includes "bind to an LAH region of an HA stem region".

In an embodiment, the monoclonal antibody/antibody fragment according to the present disclosure binds to an LAH (long alpha helix) region of an HA stem region. Whether an antibody/antibody fragment binds to an LAH region can be confirmed by a conventional method, for example, by examining whether the antibody/antibody fragment binds to a peptide corresponding to the amino acid sequence of the LAH region or corresponding to a part thereof, as in a method described in Examples in the present application.

In an embodiment, the epitope for the monoclonal antibody/antibody fragment according to the present disclosure is composed of 5 to 20 amino acid residues present in the amino acid sequence of an LAH region.

Examples of the amino acid sequence of an LAH region include the amino acid sequences of SEQ ID NOs: 19 to 36. The amino acid sequences of SEQ ID NOs: 19 to 36 are the respective amino acid sequences of LAH regions in SEQ ID NOs: 1 to 18. The amino acid sequence of an LAH region, although the LAH region is a highly conserved region in HA stem regions, can differ, for example, among different subtypes and strains, and a monoclonal antibody or antibody fragment thereof that binds to the amino acid sequence of an LAH region other than the amino acid sequences of SEQ ID NOs: 19 to 36 (e.g., the amino acid sequences of SEQ ID NOs: 37, 38, and 95 to 98) can also be included in the definition of the monoclonal antibody/antibody fragment according to the present disclosure.

In an embodiment, the monoclonal antibody/antibody fragment according to the present disclosure binds to a peptide consisting of an amino acid sequence selected from the group consisting of SEQ ID NOs: 19 to 36 or an amino acid sequence having a sequence identity of at least 90% (e.g., at least 95%, at least 97%) with an amino acid sequence selected from the group consisting of SEQ ID NOs: 19 to 36. In a further embodiment, the monoclonal antibody/antibody fragment according to the present disclosure binds to an epitope consisting of several to about a dozen of amino acids having common or similar characteristics in an amino acid sequence selected from the group consisting of SEQ ID NOs: 19 to 36 or an amino acid sequence having a sequence identity of at least 90% (e.g., at least 95%, at least 97%) with an amino acid sequence selected from the group consisting of SEQ ID NOs: 19 to 36.

The following shows SEQ ID NOs: 1 to 36 and the corresponding virus strain names.

- Example of HA stem region of H1 (A/Narita/1/2009 (HIN1pmd09))

YHHQNEQGSGYAADLKSTQNAIDEITNKVNSVIEKMNTQFTAVGKEFNHLE
KRIENLNKKVDDGFLDIWTYNAELLVLLENERTLDYHDSNVKNLYEKVRSQ
LKNNAKEIGNGCFEFYHKCDNTCMESVKNGTYDYPKYSEEAKLNREEID
(SEQ ID NO: 1)

- Example of HA stem region of H2 (A/Korea/426/1968 (H2N2))

YHHSNDQGSGYAADKESTQKAFNGITNKVNSVIEKMNTQFEAVGKEFSNLE
KRLENLNKKMEDGFLDVWTYNAELLVLMENERTLDFHDSNVKNLYDKVR
MQLRDNVKELGNGCFEFYHKCDNECMDSVKNGTYDYPKYEEESKLNRNEI
K (SEQ ID NO: 2)

- Example of HA stem region of H3 (A/X-31 (H3N2))

FRHQNSEGTGQAADLKSTQAAIDQINGKLNRVIEKTNEKFHQIEKEFSEVEGR
IQDLEKYVEDTKIDLWSYNAELLVALENQHTIDLTDSEMNKLFEKTRRQLRE
NAEDMGNGCFKIYHKCDNACIESIRNGTYDHDVYRDEALNNRFQIK (SEQ ID
NO: 3)

- Example of HA stem region of H4 (A/Duck/Alberta/28/1976 (H4N6))

FRHQNAEGTGTAADLKSTQAAIDQINGKLNRLIEKTNEKYHQIEKEFEQVEG

RIQDLEKYVEDTKIDLWSYNAEFLVALENQHTIDVTDSEMNKLFERVRRQLR

ENAEDKGNGCFEIFHQCDNNCIESIRNGTYDHDIYRDEAINNRFQIQ (SEQ ID

NO: 4)

- Example of HA stem region of H5 (A/NIBRG-14 (H5N1))

YHHSNEQGSGYAADKESTQKAIDGVTNKVNSIIDKMNTQFEAVGREFNNLE

RRIENLNKKMEDGFLDVWTYNAELLVLMENERTLDFHDSNVKNLYDKVRL

QLRDNAKELGNGCFEFYHKCDNECMESVRNGTYDYPQYSEEARLKREEIS

(SEQ ID NO: 5)

- Example of HA stem region of H6 (A/duck/Fujia5498/2006 (H6N6))

YHHENSQGSGYAADRESTQKAIDGITTKVNSIIDKMNTQFEAVGHEFSNLER

RIDNLNKRMEDGFLDVWTYNAELLVLLENERTLDLHDANVKNLHERVKSQ

LRDNANDLGNGCFEFWHKCDNECMESVKNGTYNYPKYQAESRLNKQKIE

(SEQ ID NO: 6)

- Example of HA stem region of H7 (A/Anhui/1/2013 (H7N9))

FRHQNAQGEGTAADYKSTQSAIDQITGKLNRLIEKTNQQFELIDNEFNEVEK

QIGNVINWTRDSITEVWSYNAELLVAMENQHTIDLADSEMDKLYERVKRQL

RENAEEDGTGCFEIFHKCDDDCMASIRNNTYDHSKYREEAMQNRIQID (SEQ

ID NO: 7)

- Example of HA stem region of H8 (A/Shelduck/Ukraine/Arabatska Strilka-1-10-10/2010 (H8N4))

FHHSNSEGTGMAADQKSTQEAIDKITNKVNNIVDKMNREFEVVNHEFSEVE

KRINMINGKIDDQIEDLWAYNAELLVLLENQKTLDEHDSNVKNLFDEVRRR

LSANAIDTGNGCFDILHKCDNECMETIKNGTYNHKEYEEEAKLERSKIN (SEQ

ID NO: 8)

- Example of HA stem region of H9 (A/wild bird/Anhui/S93/2014 (H9N2))

FQHSNDQGVGMAADRESTQEAVDKITSKVNNIIDKMNKQYEIIDHEFSEIEA

RLNMINNKIDDQIQDIWAYNAELLVLLENQKTLDEHDANVNNLYNKVKRAL

GSNAREDGNGCFELYHKCDDQCMETIRNGTYDRQKYQEESKLERQKIE

(SEQ ID NO: 9)

- Example of HA stem region of H10 (A/mandarin duck/Singapore/805_F-72_7/1993 (H10N5))

FRHQNAQGTGQAADYKSTQAAIDQITGKLNRLIEKTNTEFESIESEFSEIEHQI GNIINWTKDSITDIWTYQAELLVAMENQHTIDMADSEMLNLYERVRKQLRQ NAEEDGKGCFEIYHACDDSCMESIRNNTYDHSQYREEALLNRLNIN (SEQ ID NO: 10)

- Example of HA stem region of H11 (A/blue-winged teal/Ohio/12OS2099/2012 (H11))

FQHRNEEGTGIAADKESTQKAIDQITSKVNNIVDRMNTNFESAQHEFSEIEERI NQLSKHVDDSVIDIWSYNAQLLVLLENEKTLDLHDSNVRNLHERVRRMLKD

NAKDEGNGCFTFYHKCDNECIEKVRNGTYDHKEFEEESKLNRQEIE (SEQ ID NO: 11)

- Example of HA stem region of H12 (A/mallard/Interior Alaska/9BM1907R1/2009 (H12))

FQHQNAEGTGIAADRDSTQKAIDNMQNKLNNVIDKMNKQFEVVNHEFSEVE SRINMINSKIDDQITDIWAYNAELLVLLENQKTLDEHDANVRNLHDRVRRVL RENAIDTGDGCFEILHKCDNNCMDTIRNGTYNHKEYEEESKIERQKIN (SEQ ID NO: 12)

- Example of HA stem region of H13 (A/black-headed gull/Netherlands/7/2009 (H13N2))

FQHQNEQGTGIAADKESTQKAIDQITTKINNIIDKMNGNYDSIRGEFNQVEKR INMLADRIDDAVTDIWSYNAKLLVLLENDKTLDMHDANVRNLHEQVRREL KDNAIDEGNGCFELLHKCNDSCMETIRNGTYDHTEYAEESKLKRQEIN (SEQ ID NO: 13)

- Example of HA stem region of H14 (A/blue-winged teal/Guatemala/CIP049H105-15/2011 (H14N3))

FRHQNAEGTGTAADLKSTQAAIDQINGKLNRLVEKTNEKYHQIEKEFEQVEG RIQDLEKYVEDTKIDLWSYNAELLVALENQHTIDVTDSEMNKLFERVRRQLR ENAEDQGNGCFEIFHQCDNNCIESIRNGTYDHNIYRDEAINNRIKIN (SEQ ID NO: 14)

- Example of HA stem region of H15 (A/mallard/Novomychalivka/2-23-12/2010 (H15N7))

FRHQNAQGQGTAADYKSTQAAIDQITGKLNRLIEKTNQQFELIDNEFTEVEQ

QIGNVINWTRDSLTEIWSYNAELLVAMENQHTIDLADSEMNKLYERVRRQL

RENAEEDGTGCFEIFHRCDDQCMESIRNNTYNHTEYRQESLQNRIMID (SEQ ID NO: 15)

- Example of HA stem region of H16 (A/black-headed gull/Netherlands/26/2009 (H16N3))

FQHQNEQGTGIAADKASTQKAIDEITTKINNIIEKMNGNYDSIRGEFNQVEKR

INMLADRVDDAVTDVWSYNAKLLVLLENGRTLDLHDANVRNLHDQVKRA

LKNNAIDEGDGCFNLLHKCNDSCMETIRNGTYNHEDYQEESQLKRQEIE (SEQ ID NO: 16)

- Example of HA stem region of H17 (A/little yellow-shouldered bat/Guatemala/060/2010 (H17N10))

YHHENQEGSGYAADKEATQKAVDGITNKVNSIIDKMNSQFESNIKEFNRLEL

RIQHLSDRVDDALLDIWSYNTELLVLLENERTLDFHDANVKNLFEKVKAQL

KDNAIDEGNGCFLLLHKCNNSCMDDIKNGTYKYMDYREESHIEKQKID (SEQ ID NO: 17)

- Example of HA stem region of H18 (A/flat-faced Bat/peru/033/2010 (H18N11))

YHHQNSEGSGYAADKEATQKAVDAITTKVNNIIDKMNTQFESTAKEFNKIE

MRIKHLSDRVDDGFLDVWSYNAELLVLLENERTLDFHDANVNNLYQKVKV

QLKDNAIDMGNGCFKILHKCNNTCMDDIKNGTYNYYEYRKESHLEKQKID (SEQ ID NO: 18)

- Example of LAH region of H1

RIENLNKKVDDGFLDIWTYNAELLVLLENERTLDYHDSNVKNLYEKVRSQL

KNNA (SEQ ID NO: 19)

- Example of LAH region of H2

RLENLNKKMEDGFLDVWTYNAELLVLMENERTLDFHDSNVKNLYDKVRM

QLRDNV (SEQ ID NO: 20)

- Example of LAH region of H3

RIQDLEKYVEDTKIDLWSYNAELLVALENQHTIDLTDSEMNKLFEKTRRQLR

ENA (SEQ ID NO: 21)

- Example of LAH region of H4

RIQDLEKYVEDTKIDLWSYNAEFLVALENQHTIDVTDSEMNKLFERVRRQLR

ENA (SEQ ID NO: 22)

- Example of LAH region of H5

RIENLNKKMEDGFLDVWTYNAELLVLMENERTLDFHDSNVKNLYDKVRLQ

LRDNA (SEQ ID NO: 23)

- Example of LAH region of H6

RIDNLNKRMEDGFLDVWTYNAELLVLLENERTLDLHDANVKNLHERVKSQ

LRDNA (SEQ ID NO: 24)

- Example of LAH region of H7

QIGNVINWTRDSITEVWSYNAELLVAMENQHTIDLADSEMDKLYERVKRQL

RENA (SEQ ID NO: 25)

- Example of LAH region of H8

RINMINGKIDDQIEDLWAYNAELLVLLENQKTLDEHDSNVKNLFDEVRRRLS

ANA (SEQ ID NO: 26)

- Example of LAH region of H9

RLNMINNKIDDQIQDIWAYNAELLVLLENQKTLDEHDANVNNLYNKVKRAL

GSNA (SEQ ID NO: 27)

- Example of LAH region of H10

QIGNIINWTKDSITDIWTYQAELLVAMENQHTIDMADSEMLNLYERVRKQLR

QNA (SEQ ID NO: 28)

- Example of LAH region of H1 1

RINQLSKHVDDSVIDIWSYNAQLLVLLENEKTLDLHDSNVRNLHERVRRML

KDNA (SEQ ID NO: 29)

- Example of LAH region of H12

RINMINSKIDDQITDIWAYNAELLVLLENQKTLDEHDANVRNLHDRVRRVLR

ENA (SEQ ID NO: 30)

- Example of LAH region of H13

RINMLADRIDDAVTDIWSYNAKLLVLLENDKTLDMHDANVRNLHEQVRRE

LKDNA (SEQ ID NO: 31)

- Example of LAH region of H14

RIQDLEKYVEDTKIDLWSYNAELLVALENQHTIDVTDSEMNKLFERVRRQLR

ENA (SEQ ID NO: 32)

- Example of LAH region of H15

QIGNVINWTRDSLTEIWSYNAELLVAMENQHTIDLADSEMNKLYERVRRQL

RENA (SEQ ID NO: 33)

- Example of LAH region of H16

RINMLADRVDDAVTDVWSYNAKLLVLLENGRTLDLHDANVRNLHDQVKR

ALKNNA (SEQ ID NO: 34)

- Example of LAH region of H17

RIQHLSDRVDDALLDIWSYNTELLVLLENERTLDFHDANVKNLFEKVKAQL

KDNA (SEQ ID NO: 35)

- Example of LAH region of H18

RIKHLSDRVDDGFLDVWSYNAELLVLLENERTLDFHDANVNNLYQKVKVQ

LKDNA (SEQ ID NO: 36)

In an embodiment, the monoclonal antibody/antibody fragment according to the present disclosure has protective capacity against infection for an influenza A virus.

Herein, the definition of protective capacity against infection for an influenza virus includes capability of preventing a subject from being infected with an influenza virus, capability of increasing the survival rate of subjects infected with an influenza virus, and/or capability of mitigating a disease symptom in a subject infected with an influenza virus. Herein, the statement "having protective capacity against infection for an influenza virus" means having protective capacity against infection for at least one influenza virus strain. Whether the monoclonal antibody/antibody fragment according to the present disclosure has protective capacity against infection can be confirmed by a conventional method, for example, in such a manner that, as in a method described in Examples in the present application, mice having received preliminary administration of the monoclonal antibody/antibody fragment according to the present disclosure are challenged by inoculation with an influenza virus, the time course of the body weights and survival rate after that are examined, and if less body weight loss and a higher survival rate than those of a negative control are found, the monoclonal antibody/antibody fragment according to the present disclosure is determined to have protective capacity against infection.

The present disclosure provides, in an aspect, a pharmaceutical composition comprising the monoclonal antibody/antibody fragment according to the present disclosure (hereinafter, also referred to as the pharmaceutical composition according to the present disclosure). Since the monoclonal antibody or antibody fragment thereof according to the present disclosure can be useful for treating or preventing an influenza A infection, the monoclonal antibody or antibody fragment thereof according to the present disclosure can serve as an active ingredient of a pharmaceutical composition for treating or preventing an influenza A infection. In an embodiment, the pharmaceutical composition according to the present disclosure can be used for treating or preventing an influenza A virus infection.

The definition of preventing an influenza A infection includes inhibition of the adsorption of an influenza A virus on cell surfaces, inhibition of the invasion thereof into cells, and prevention of onset by removing the damage of virus-infected cells. The meaning of "treating an influenza A infection" includes providing cure of or ameliorating a symptom caused by infection with influenza A.

The present disclosure provides, in an aspect, the monoclonal antibody or antibody fragment thereof according to the present disclosure for use in treating or preventing an influenza A virus infection.

The present disclosure provides, in an aspect, a method for treating or preventing an influenza A virus infection, the method including administering the monoclonal antibody or antibody fragment thereof according to the present disclosure to a subject.

The present disclosure provides, in an aspect, use of the monoclonal antibody or antibody fragment thereof according to the present disclosure in production of a medicament for treating or preventing an influenza A virus infection.

The present disclosure provides, in an aspect, use of the monoclonal antibody or antibody fragment thereof according to the present disclosure for treating or preventing an influenza A virus infection.

In an embodiment, the monoclonal antibody/antibody fragment according to the present disclosure binds to HA stem regions (or LAH regions) derived from two or more different influenza A virus strains. Herein, the term "cross-reactivity" is occasionally used, and herein means binding to HA stem regions (or LAH regions) derived from two or more different influenza A virus strains.

In an embodiment, the monoclonal antibody/antibody fragment according to the present disclosure has protective capacity against infection for two or more different influenza A virus strains.

In an embodiment, the pharmaceutical composition according to the present disclosure is used for treating or preventing an infection with two or more different influenza A virus strains.

The two or more different influenza virus strains may be two or more influenza virus strains belonging to the same HA subtype or two or more influenza virus strains belonging to different HA subtypes.

In an embodiment, the monoclonal antibody/antibody fragment according to the present disclosure binds to HA stem regions (or LAH regions) derived from two or more different influenza virus strains belonging to the same HA subtype.

In an embodiment, the monoclonal antibody/antibody fragment according to the present disclosure has protective capacity against infection for two or more different influenza virus strains belonging to the same HA subtype.

In an embodiment, the pharmaceutical composition according to the present disclosure can be used for treating or preventing an infection with two or more different influenza virus strains belonging to the same HA subtype.

For example, the monoclonal antibody/antibody fragment according to the present disclosure that binds to an HA stem region (SEQ ID NO: 3) or LAH region (SEQ ID NO: 21) of A/X-31 (H3N2) can have binding capacity and/or protective capacity against infection not only for A/X-31 (H3N2) but also for another virus strain of H3 subtype, for example, A/Victoria/361/2011 (H3N2), A/Singapore/INFIMH-16-0019/2016 (H3N2), A/Kansas/14/2017 (H3N2), A/Aichi/2/1968 (H3N2), A/Fujian/411/02(H3N2), A/Guizhou/54/89 (H3N2), A/OMS/5389/88 (H3N2), A/Beijing/32/92 (H3N2), A/England/427/88 (H3N2), A/Johannesburg/33/94 (H3N2), A/Leningrad/360/86 (H3N2), A/Mississippi/1/85 (H3N2), A/Philippines/2/82 (H3N2), A/Shangdong/9/93 (H3N2), A/Shanghai/16/89 (H3N2), A/Shanghai/24/90 (H3N2), A/Sichuan/2/87 (H3N2), A/Kitakyushyu/159/93 (H3N2), A/Akita/1/94 (H3N2), A/Panama/2007/99 (H3N2), A/Wyoming/03/03 (H3N2), A/New York/55/2004 (H3N2), or A/Hiroshima/52/2005 (H3N2).

For example, the monoclonal antibody/antibody fragment according to the present disclosure that binds to an HA stem region (SEQ ID NO: 1) or LAH region (SEQ ID NO: 19) of A/Narita/1/2009 (H1N1pdm09) has binding capacity and/or protective capacity against infection not only for A/Narita/1/2009 (H1N1pdm09) but also for another virus strain of H1 subtype, for example, A/Puerto Rico/8/34 (H1N1), A/Michigan/45/2015 (H1N1pdm09), A/Brisbane/02/2018 (H1N1pdm09) A/Beijing/262/95 (H1N1), A/Brazil/11/78 (H1N1), A/Chile/1/83 (H1N1), A/New Jersey/8/76 (H1N1), A/Taiwan/1/86 (H1N1), A/Yamagata/32/89 (H1N), A/New Caledonia/20/99 (H1N1), A/Solomon Islands/3/2006 (H1N1), A/Brisbane/59/2007 (H1N1), or A/Mexico/4108/2009 (H1N1).

In an embodiment, the monoclonal antibody/antibody fragment according to the present disclosure binds to HA stem regions of two or more different influenza A virus HA subtypes. Herein, the statement "the monoclonal antibody/antibody fragment according to the present disclosure binds to HA stem regions of two or more different influenza A virus HA subtypes" means that the monoclonal antibody/antibody fragment according to the present disclosure binds to an HA stem region (or LAH region) derived from at least one influenza A virus strain of a certain HA subtype and binds to an HA stem region (or LAH region) derived from at least one influenza A virus strain of another HA subtype.

In an embodiment, the monoclonal antibody/antibody fragment according to the present disclosure has protective capacity against infection for two or more different influenza A virus HA subtypes. The statement "the monoclonal antibody/antibody fragment according to the present disclosure has protective capacity against infection for two or more different influenza A virus HA subtypes" means that the monoclonal antibody/antibody fragment according to the present disclosure has protective capacity against infection for at least one influenza virus strain of a certain HA subtype and has protective capacity against infection for at least one influenza virus strain of another HA subtype.

In an embodiment, the pharmaceutical composition according to the present disclosure is used for treating or preventing an infection with two or more different influenza A virus HA subtypes.

In an embodiment, the monoclonal antibody/antibody fragment according to the present disclosure binds to HA stem regions (or LAH regions) of two or more different influenza A virus HA subtypes belonging to phylogenetic group 1. Herein, the statement "the monoclonal antibody/antibody fragment according to the present disclosure binds to HA stem regions (or LAH regions) of two or more different influenza A virus HA subtypes belonging to phylogenetic group 1" means that the monoclonal antibody/antibody fragment according to the present disclosure binds to an HA stem region (or LAH region) derived from at least one influenza virus strain of a certain influenza A virus HA subtype belonging to phylogenetic group 1, and binds to an HA stem region (or LAH region) derived from at least one influenza virus strain of another influenza A virus HA subtype belonging to phylogenetic group 1.

In an embodiment, the monoclonal antibody/antibody fragment according to the present disclosure has protective capacity against infection for two or more different influenza A virus HA subtypes belonging to phylogenetic group 1. Herein, the statement "the monoclonal antibody/antibody fragment according to the present disclosure has protective capacity against infection for two or more different influenza A virus HA subtypes belonging to phylogenetic group 1" means that the monoclonal antibody/antibody fragment according to the present disclosure has protective capacity against infection for at least one influenza virus strain of a certain influenza A virus HA subtype belonging to phylogenetic group 1, and has protective capacity against infection for at least one influenza virus strain of another influenza A virus HA subtype belonging to phylogenetic group 1.

For example, the monoclonal antibody or antibody fragment thereof with binding capacity to an LAH region of H1 according to the present disclosure can have binding capacity and protective capacity against infection also for an influenza virus or viruses of H2, H5, H6, H8, H9, H11, H12, H13, H16, H17, and/or H18 subtype(s) of the same type A belonging to phylogenetic group 1. The monoclonal antibody or antibody fragment thereof with binding capacity to an LAH region of H1 according to the present disclosure preferably has binding capacity and/or protective capacity against infection also for an influenza virus of H5 subtype of the same type A belonging to phylogenetic group 1. Specific examples of the monoclonal antibody or antibody fragment thereof according to the present disclosure that binds to an LAH region of H1 and binds to an LAH region of H5 include clone 3, clone 4, clone 6, clone 7, and clone 8 described in Examples in the present application.

In an embodiment, the pharmaceutical composition according to the present disclosure is used for treating or preventing an infection with two or more different influenza A virus HA subtypes belonging to phylogenetic group 1.

In an embodiment, the monoclonal antibody/antibody fragment according to the present disclosure binds to HA stem regions (or LAH regions) of two or more different influenza A virus HA subtypes belonging to phylogenetic group 2. Herein, the statement "the monoclonal antibody/antibody fragment according to the present disclosure binds to HA stem regions (or LAH regions) of two or more different influenza A virus HA subtypes belonging to phylogenetic group 2" means that the monoclonal antibody/antibody fragment according to the present disclosure binds to an HA stem region (or LAH region) derived from at least one influenza virus strain of a certain influenza A virus HA subtype belonging to phylogenetic group 2, and binds to an HA stem region (or LAH region) derived from at least one influenza virus strain of another influenza A virus HA subtype belonging to phylogenetic group 2.

In an embodiment, the monoclonal antibody/antibody fragment according to the present disclosure has protective capacity against infection for two or more different influenza A virus HA subtypes belonging to phylogenetic group 2. Herein, the statement "the monoclonal antibody/antibody fragment according to the present disclosure has protective capacity against infection for two or more different influenza A virus HA subtypes belonging to phylogenetic group 2" means that the monoclonal antibody/antibody fragment according to the present disclosure has protective capacity against infection for at least one influenza virus strain of a certain influenza A virus HA subtype belonging to phylogenetic group 2, and has protective capacity against infection for at least one influenza virus strain of another influenza A virus HA subtype belonging to phylogenetic group 2.

For example, the monoclonal antibody or antibody fragment thereof with binding capacity to an LAH region of H3 according to the present disclosure can have binding capacity and protective capacity against infection also for an influenza virus or viruses of H4, H7, H10, H14, and/or H15 subtype(s) of the same type A belonging to phylogenetic group 2. The monoclonal antibody or antibody fragment thereof with binding capacity to an LAH region of H3 according to the present disclosure preferably has binding capacity and/or protective capacity against infection also for an influenza virus of H7 subtype of the same type A belonging to phylogenetic group 2. Specific examples of the monoclonal antibody or antibody fragment thereof according to the present disclosure that binds to an LAH region of H3 and binds to an LAH region of H7 include clone 1, clone 2, clone 5, clone 6, and clone 7 described in Examples in the present application.

In an embodiment, the pharmaceutical composition according to the present disclosure is used for treating or preventing an infection with two or more different influenza A virus HA subtypes belonging to phylogenetic group 2.

In an embodiment, the monoclonal antibody/antibody fragment according to the present disclosure binds to an HA stem region (or LAH region) of an influenza A virus HA subtype belonging to phylogenetic group 1, and binds to an HA stem region (or LAH region) of an influenza A virus HA subtype belonging to phylogenetic group 2.

Herein, the statement "the monoclonal antibody/antibody fragment according to the present disclosure binds to an HA stem region (or LAH region) of an influenza A virus HA subtype belonging to phylogenetic group 1" means that the monoclonal antibody/antibody fragment according to the present disclosure binds to an HA stem region (or LAH region) derived from at least one influenza virus strain of an influenza A virus HA subtype belonging to phylogenetic group 1.

Herein, the statement "the monoclonal antibody/antibody fragment according to the present disclosure binds to an HA stem region (or LAH region) of an influenza A virus HA subtype belonging to phylogenetic group 2" means that the monoclonal antibody/antibody fragment according to the present disclosure binds to an HA stem region (or LAH region) derived from at least one influenza virus strain of an influenza A virus HA subtype belonging to phylogenetic group 2.

In an embodiment, the monoclonal antibody/antibody fragment according to the present disclosure has protective capacity against infection for an influenza A virus HA subtype belonging to phylogenetic group 1, and has protective capacity against infection for an influenza A virus HA subtype belonging to phylogenetic group 2.

Herein, the statement "the monoclonal antibody/antibody fragment according to the present disclosure has protective capacity against infection for an influenza A virus HA subtype belonging to phylogenetic group 1" means that the monoclonal antibody/antibody fragment according to the present disclosure has protective capacity against infection for at least one influenza virus strain of an influenza A virus HA subtype belonging to phylogenetic group 1.

Herein, the statement "the monoclonal antibody/antibody fragment according to the present disclosure has protective capacity against infection for an influenza A virus HA subtype belonging to phylogenetic group 2" means that the monoclonal antibody/antibody fragment according to the present disclosure has protective capacity against infection for at least one influenza virus strain of an influenza A virus HA subtype belonging to phylogenetic group 2.

For example, the monoclonal antibody or antibody fragment with binding capacity to an LAH region of H3 according to the present disclosure can have binding capacity and protective capacity against infection not only for an influenza virus(es) of H3, H4, H7, H10, H14, and/or H15 subtype(s) of phylogenetic group 2, but also for an influenza virus(es) of H1, H2, H5, H6, H8, H9, H11, H12, H13, H16, H17, and/or H18 subtype(s) of phylogenetic group 1. Specific examples include clone 6 and clone 7 described in Examples.

The monoclonal antibody or antibody fragment that binds to an HA stem region (or LAH region) of an influenza A virus HA subtype belonging to phylogenetic group 1 and binds to an HA stem region (or LAH region) of an influenza A virus HA subtype belonging to phylogenetic group 2 can have cross-reactivity for all subtypes having infectivity to humans (specifically, H1, H3, H5, H7, H9), and hence can also be effective as a therapeutic in pandemics.

In an embodiment, the pharmaceutical composition according to the present disclosure is used for treating or preventing an infection with an influenza A virus HA subtype belonging to phylogenetic group 1 and an influenza A virus HA subtype belonging to phylogenetic group 2.

It is known that at least HA subtypes H1, H2, H5, H6, H8, H9, H11, H12, H13, H16, H17, and H18 belong to phylogenetic group 1, and at least HA subtypes H3, H4, H7, H10, H14, and H15 belong to phylogenetic group 2. To these groups, further subtypes may be added through further discoveries.

Herein, the term "antibody" means an immunoglobulin molecule that includes two light chains and two heavy chains and binds to an antigen, and, when the term is used, the definition includes mouse antibodies, chimeric antibodies (e.g., mouse-human chimeric antibodies), humanized antibodies, fully human antibodies (also referred to as human antibodies, simply), multispecific antibodies (e.g., bispecific antibodies), and so on. Herein, the term "monoclonal antibodies" means a group of antibodies each substantially consisting of one molecular species, unless otherwise stated. A monoclonal antibody can be produced by a common method in the field. In an embodiment, the monoclonal antibody according to the present disclosure is an isolated monoclonal antibody.

The immunoglobulin classes of antibodies include IgG, IgM, IgA, IgD, and IgE. Immunoglobulin molecules of different classes share a common basic structure, which is composed of heavy chains each having a molecular weight of 50000 to 70000 and light chains each having a molecular weight of 20000 to 30000.

A heavy chain is a polypeptide typically comprising approximately 440 amino acids, and heavy chains have structures characteristic to their classes. The heavy chains corresponding to IgG, IgM, IgA, IgD, and IgE are called a γ chain, a μ chain, an α chain, a δ chain, and an ε chain, respectively. Each immunoglobulin class is further divided into subclasses; for IgG, for example, the subclasses IgG1, IgG2, IgG3, and IgG4 are present, and their heavy chains are called γ1, γ2, γ3, and γ4, respectively. The monoclonal antibody according to the present disclosure is not limited to any particular immunoglobulin class. In an embodiment, the monoclonal antibody according to the present disclosure is IgG.

A light chain is a polypeptide typically comprising approximately 220 amino acids, and L type and K type are known therefor, and called a λ chain and a κ, chain, respectively. The light chains of the monoclonal antibody according to the present disclosure may be each a λ chain or a κ chain.

The basic structure of immunoglobulin molecules includes two heavy chains and two light chains bound via disulfide bonds (S-S bonds) and noncovalent bonds, and has a molecular weight of 150000 to 190000. In the monoclonal antibody according to the present disclosure, the two heavy chains in one molecule of the immunoglobulin may be the same or different. The two light chains in one molecule of the immunoglobulin may be the same or different.

A heavy chain has four (γ chain, α chain, δ chain) or five (μ chain, ε chain) intrachain S-S bonds, and a light chain has two intrachain S-S bonds. In any of heavy chains and light chains, one constant region loop is formed every 100 to 110 amino acid residues. The three-dimensional structure formed by such a constant region loop is similar among structural units of linked constant region loops, and each of the structural units is called a domain. In any of heavy chains and light chains, the amino acid sequence of a domain positioned at the N terminus is not constant even among antibodies of the same class (subclass) of the same animal species, and the domain is called a variable region (V region). A heavy chain variable region is occasionally abbreviated as $V_H$, and a light chain variable region as $V_L$. The amino acid sequence in the C-terminal side of the variable region is almost constant in each class or subclass, and the part is called a constant region (C region). The domains of a heavy chain constant region are occasionally abbreviated as $C_H1$, $C_H2$, and $C_H3$, and the domain of a light chain constant region as $C_L$.

An antigen-binding site of an antibody is composed of a heavy chain variable region ($V_H$) and a light chain variable region ($V_L$), and the specificity of binding is determined by the amino acid sequence of this site. The variability of variable regions of heavy chains and light chains has been known to be provided by three small hypervariable regions present in each chain, and these regions are called complementarity-determining regions (CDRs). Several numbering systems to determine CDRs are commonly used, and examples thereof include Kabat, Chothia, AbM, and IMGT (Martin et al. (1989) Proc. Natl. Acad. Sci. USA 86: 9268-9272; Martin et al. (1991) Methods Enzymol. 203: 121-153; Pedersen et al. (1992) Immunomethods 1: 126; Reeset al. (1996) In Sternberg M. J.E. (ed.), Protein Structure Prediction, Oxford University Press, Oxford, pp. 141-172; Lefranc et al. (2003) Dev Comp Immunol. 27: 55-77). In addition, online tools including IMGT/V-QUEST (http://www.imgt.org/) are used for identifying CDRs. In the present disclosure, CDRs of variable regions of heavy chains and light chains are determined by IMGT, unless otherwise stated.

In each variable region, the parts except CDRs are called framework regions (FRs), and the amino acid sequences are relatively constant. Accordingly, the binding specificity of an antibody can be transplanted to another antibody by transplanting CDRs. Each framework region has the β sheet structure, and each CDR can form a loop to link such β sheet structures. CDRs in heavy chains and light chains are retained in their three-dimensional structures by framework regions, forming an antigen-binding site.

The biological activities of each immunoglobulin class, such as binding to complements and various cells, depend on the structure of the constant region. The constant region of the monoclonal antibody according to the present disclosure may be modified, for example, for modulating antibody-dependent cellular cytotoxicity (ADCC) activity, complement-dependent cytotoxicity (CDC) activity, and biokinetics. The modification includes modification of an amino acid sequence or a sugar chain.

In the present disclosure, a human antibody is an antibody in which the whole sequence is a human sequence, a humanized antibody is an antibody in which the sequence except CDRs is a human sequence, a chimeric antibody is an antibody in which the variable region is a non-human sequence and the constant region is a human sequence, and a mouse antibody is an antibody in which the whole sequence is a mouse sequence. Humanized antibodies and chimeric antibodies can be produced by genetic engineering in accordance with a conventional method. Human antibodies can be produced by a genetic engineering method with a human-human (or human-mouse) hybridoma, or by a phage display method, or with a human antibody-producing animal (e.g., a mouse), or with an antibody gene prepared from a human B cell. Mouse antibodies can be produced by a cell fusion method with lymphocytes from an immunized animal, or by a genetic engineering method. Antibody fragments can be produced by genetic engineering, or acquired from a library such as a Fab expression library.

The antibody fragment according to the present disclosure is a molecule comprising part of the antibody molecule of the monoclonal antibody according to the present disclosure and having antigen-binding capacity. Examples thereof include a single-chain antibody (Single-Chain FV, scFV), F (ab')$_2$, Fab', Fab, Fv, and a single-domain antibody. The antibody fragment can be produced by a common method in the field.

For scFv, for example, reference can be made to Ahmad, Z. A. et al. scFv antibody: principles and clinical application. Clin. Dev. Immunol. 2012, 980250 (2012).

A single-domain antibody is an antibody fragment comprising all or some of the heavy or light chain variable domains of an antibody. For single-domain antibodies, for example, reference can be made to Ward et al., 1989 Nature 341:544-546.

In an embodiment, the antibody fragment according to the present disclosure is an isolated antibody fragment.

An isolated nucleic acid encoding the monoclonal antibody or antibody fragment thereof according to the present disclosure is also included in an aspect of the present invention.

In the present disclosure, the term "isolated" means having been altered from the natural state, or having been partially or completely separated from the natural state. For example, an isolated nucleic acid or protein can exist in a substantially purified form, or exist in a non-natural environment such as a host cell. The term isolated antibody can mean, for example, that substantially no other antibodies having different antigen specificity are not included. Furthermore, the term isolated antibody can mean that substantially no other cellular substances and/or chemicals are not included.

In the present disclosure, the term "at least one" means a number of one or more, and examples include at least two, at least three, at least four, at least five, at least six, at least seven, at least eight, at least nine, and at least 10, unless any conflict arises with the contents. Furthermore, examples of "at least one" include 1 to 18, 1 to 10, 1 to 5, 1 to 3 (e.g., 1, 2, 3, 4, 5, 6, 7, 8, 9, 10, 11, 12, 13, 14, 15, 16, 17, 18), and "at least one" may mean any number greater than or equal to them, unless any conflict arises with the contents.

The present inventors obtained LAH region-binding monoclonal antibodies (clones 1 to 8), analyzed the amino acid sequences of the antibodies, and even determined the variable regions and CDRs of the antibodies.

The amino acid sequences of the variable regions and CDRs of clones 1 to 8 are shown in Tables 1 to 7 in Examples.

In an embodiment, the monoclonal antibody or antibody fragment thereof according to the present disclosure comprises:

(a)-1 a heavy chain variable region comprising
CDR1 comprising (or consisting of) an amino acid sequence of SEQ ID NO: 47 or an amino acid sequence in which one or two amino acid residues are independently substituted, deleted, added, or inserted in the amino acid sequence, CDR2 comprising (or consisting of) an amino acid sequence of SEQ ID NO: 48 or an amino acid sequence in which one or two amino acid residues are independently substituted, deleted, added, or inserted in the amino acid sequence, and CDR3 comprising (or consisting of) an amino acid sequence of SEQ ID NO: 49 or an amino acid sequence in which one or two amino acid residues are independently substituted, deleted, added, or inserted in the amino acid sequence; and/or
a light chain variable region comprising
CDR1 comprising (or consisting of) an amino acid sequence of SEQ ID NO: 50 or an amino acid sequence in which one or two amino acid residues are independently substituted, deleted, added, or inserted in the amino acid sequence, CDR2 comprising (or consisting of) an amino acid sequence of SEQ ID NO: 51 or an amino acid sequence in which one or two amino acid residues are independently substituted, deleted, added, or inserted in the amino acid sequence, and CDR3 comprising (or consisting of) an amino acid sequence of SEQ ID NO: 52 or an amino acid sequence in which one or two amino acid residues are independently substituted, deleted, added, or inserted in the amino acid sequence.

In an embodiment, the monoclonal antibody or antibody fragment thereof according to the present disclosure comprises:

(a)-2 a heavy chain variable region comprising
CDR1 comprising (or consisting of) an amino acid sequence of SEQ ID NO: 47 or an amino acid sequence in which one or two amino acid residues are independently substituted, deleted, added, or inserted in the amino acid sequence, CDR2 comprising (or consisting of) an amino acid sequence of SEQ ID NO: 48 or an amino acid sequence in which one or two amino acid residues are independently substituted, deleted, added, or inserted in the amino acid sequence, and CDR3 comprising (or consisting of) an amino acid sequence of SEQ ID NO: 49 or an amino acid sequence in which one or two amino acid residues are independently substituted, deleted, added, or inserted in the amino acid sequence; and
a light chain variable region comprising
CDR1 comprising (or consisting of) an amino acid sequence of SEQ ID NO: 50 or an amino acid sequence in which one or two amino acid residues are independently substituted, deleted, added, or inserted in the amino acid sequence, CDR2 comprising (or consisting of) an amino acid sequence of SEQ ID NO: 51 or an amino acid sequence in which one or two amino acid residues are independently substituted, deleted, added, or inserted in the amino acid sequence, and CDR3 comprising (or consisting of) an amino acid sequence of SEQ ID NO: 52 or an amino acid sequence in which one or two amino acid residues are independently substituted, deleted, added, or inserted in the amino acid sequence.

In an embodiment, the monoclonal antibody or antibody fragment thereof according to the present disclosure comprises:

(a')-1 a heavy chain variable region comprising
CDR1 comprising (or consisting of) an amino acid sequence of SEQ ID NO: 47,
CDR2 comprising (or consisting of) an amino acid sequence of SEQ ID NO: 48, and
CDR3 comprising (or consisting of) an amino acid sequence of SEQ ID NO: 49; and/or
a light chain variable region comprising
CDR1 comprising (or consisting of) an amino acid sequence of SEQ ID NO: 50,
CDR2 comprising (or consisting of) an amino acid sequence of SEQ ID NO: 51, and
CDR3 comprising (or consisting of) an amino acid sequence of SEQ ID NO: 52.

In an embodiment, the monoclonal antibody or antibody fragment thereof according to the present disclosure comprises:

(a')-2 a heavy chain variable region comprising
CDR1 comprising (or consisting of) an amino acid sequence of SEQ ID NO: 47,

CDR2 comprising (or consisting of) an amino acid sequence of SEQ ID NO: 48, and

CDR3 comprising (or consisting of) an amino acid sequence of SEQ ID NO: 49; and

a light chain variable region comprising

CDR1 comprising (or consisting of) an amino acid sequence of SEQ ID NO: 50,

CDR2 comprising (or consisting of) an amino acid sequence of SEQ ID NO: 51, and

CDR3 comprising (or consisting of) an amino acid sequence of SEQ ID NO: 52.

In an embodiment, the monoclonal antibody or antibody fragment thereof according to the present disclosure comprises:

(a")-1 a heavy chain variable region comprising (or consisting of) an amino acid sequence having a sequence identity of at least 90%, 95%, 97%, or 99% with an amino acid sequence of SEQ ID NO: 53 and/or a light chain variable region comprising (or consisting of) an amino acid sequence having a sequence identity of at least 90%, 95%, 97%, or 99% with an amino acid sequence of SEQ ID NO: 54.

In an embodiment, the monoclonal antibody or antibody fragment thereof according to the present disclosure comprises:

(a")-2 a heavy chain variable region comprising (or consisting of) an amino acid sequence having a sequence identity of at least 90%, 95%, 97%, or 99% with an amino acid sequence of SEQ ID NO: 53 and a light chain variable region comprising (or consisting of) an amino acid sequence having a sequence identity of at least 90%, 95%, 97%, or 99% with an amino acid sequence of SEQ ID NO: 54.

In an embodiment, the monoclonal antibody or antibody fragment thereof according to the present disclosure comprises:

(a‴)-1 a heavy chain variable region comprising (or consisting of) an amino acid sequence of SEQ ID NO: 53 and/or a light chain variable region comprising (or consisting of) an amino acid sequence of SEQ ID NO: 54.

In an embodiment, the monoclonal antibody or antibody fragment thereof according to the present disclosure comprises:

(a‴)-2 a heavy chain variable region comprising (or consisting of) an amino acid sequence of SEQ ID NO: 53 and a light chain variable region comprising (or consisting of) an amino acid sequence of SEQ ID NO: 54.

In an embodiment, the monoclonal antibody or antibody fragment thereof according to the present disclosure comprises:

(a⁗)-1 a heavy chain variable region comprising (or consisting of) an amino acid sequence of SEQ ID NO: 53 or an amino acid sequence in which one to several (e.g., 1 to 10, 1 to 5, 1 to 3) amino acid residues are independently substituted, deleted, added, or inserted in the amino acid sequence and/or a light chain variable region comprising (or consisting of) an amino acid sequence of SEQ ID NO: 54 or an amino acid sequence in which one to several (e.g., 1 to 10, 1 to 5, 1 to 3) amino acid residues are independently substituted, deleted, added, or inserted in the amino acid sequence.

In an embodiment, the monoclonal antibody or antibody fragment thereof according to the present disclosure comprises:

(a⁗)-2 a heavy chain variable region comprising (or consisting of) an amino acid sequence of SEQ ID NO: 53 or an amino acid sequence in which one to several (e.g., 1 to 10, 1 to 5, 1 to 3) amino acid residues are independently substituted, deleted, added, or inserted in the amino acid sequence and a light chain variable region comprising (or consisting of) an amino acid sequence of SEQ ID NO: 54 or an amino acid sequence in which one to several (e.g., 1 to 10, 1 to 5, 1 to 3) amino acid residues are independently substituted, deleted, added, or inserted in the amino acid sequence.

In an embodiment, each of the monoclonal antibodies or antibody fragments thereof in (a)-1 and -2, (a')-1 and -2, (a")-1 and -2, (a‴)-1 and -2, and (a⁗)-1 and -2 (hereinafter, also referred to as a clone 2-derived antibody/antibody fragment) binds to a peptide consisting of an amino acid sequence of SEQ ID NO: 3 or SEQ ID NO: 21.

In an embodiment, each of the clone 2-derived antibodies/antibody fragments binds to a peptide consisting of an amino acid sequence of SEQ ID NO: 7 or SEQ ID NO: 25.

In an embodiment, each of the clone 2-derived antibodies/antibody fragments binds to a peptide consisting of an amino acid sequence of SEQ ID NO: 3 or SEQ ID NO: 21 and/or binds to a peptide consisting of an amino acid sequence of SEQ ID NO: 7 or SEQ ID NO: 25.

In an embodiment, each of the clone 2-derived antibodies/antibody fragments has protective capacity against infection for an influenza A virus H3 and/or H7 subtype(s).

In an embodiment, each of the clone 2-derived antibodies/antibody fragments is used for treating or preventing an infection with an influenza A virus HA subtype belonging to phylogenetic group 2 (e.g., two or more different influenza A virus HA subtypes belonging to phylogenetic group 2).

In an embodiment, each of the clone 2-derived antibodies/antibody fragments is used for treating or preventing an infection with an H3 and/or H7 subtype(s).

In an embodiment, each of the clone 2-derived antibodies/antibody fragments is used for detecting or quantifying a membrane fusion-type influenza HA antigen derived from an influenza A virus HA subtype belonging to phylogenetic group 2.

In an embodiment, each of the clone 2-derived antibodies/antibody fragments is used for detecting or quantifying a membrane fusion-type influenza HA antigen(s) derived from an H3 and/or H7 subtype(s).

In an embodiment, the monoclonal antibody or antibody fragment thereof according to the present disclosure comprises:

(b)-1 a heavy chain variable region comprising
CDR1 comprising (or consisting of) an amino acid sequence of SEQ ID NO: 55 or an amino acid sequence in which one or two amino acid residues are independently substituted, deleted, added, or inserted in the amino acid sequence, CDR2 comprising (or consisting of) an amino acid sequence of SEQ ID NO: 56 or an amino acid sequence in which one or two amino acid residues are independently substituted, deleted, added, or inserted in the amino acid sequence, and CDR3 comprising (or consisting of) an amino acid sequence of SEQ ID NO: 57 or an amino acid sequence in which one or two amino acid residues are independently substituted, deleted, added, or inserted in the amino acid sequence; and/or

a light chain variable region comprising
CDR1 comprising (or consisting of) an amino acid sequence of SEQ ID NO: 58 or an amino acid sequence in which one or two amino acid residues are independently substituted, deleted, added, or inserted in the amino acid sequence, CDR2 comprising (or consisting of) an amino acid sequence of SEQ ID NO: 59 or an amino acid sequence in which one or two amino acid residues are independently substituted, deleted, added, or inserted in the amino acid sequence, and CDR3 comprising (or consisting of) an amino acid sequence of SEQ ID NO: 60 or an amino acid sequence in which one or two amino acid residues are independently substituted, deleted, added, or inserted in the amino acid sequence.

In an embodiment, the monoclonal antibody or antibody fragment thereof according to the present disclosure comprises:

(b)-2 a heavy chain variable region comprising
CDR1 comprising (or consisting of) an amino acid sequence of SEQ ID NO: 55 or an amino acid sequence in which one or two amino acid residues are independently substituted, deleted, added, or inserted in the amino acid sequence, CDR2 comprising (or consisting of) an amino acid sequence of SEQ ID NO: 56 or an amino acid sequence in which one or two amino acid residues are independently substituted, deleted, added, or inserted in the amino acid sequence, and CDR3 comprising (or consisting of) an amino acid sequence of SEQ ID NO: 57 or an amino acid sequence in which one or two amino acid residues are independently substituted, deleted, added, or inserted in the amino acid sequence; and a light chain variable region comprising
CDR1 comprising (or consisting of) an amino acid sequence of SEQ ID NO: 58 or an amino acid sequence in which one or two amino acid residues are independently substituted, deleted, added, or inserted in the amino acid sequence, CDR2 comprising (or consisting of) an amino acid sequence of SEQ ID NO: 59 or an amino acid sequence in which one or two amino acid residues are independently substituted, deleted, added, or inserted in the amino acid sequence, and CDR3 comprising (or consisting of) an amino acid sequence of SEQ ID NO: 60 or an amino acid sequence in which one or two amino acid residues are independently substituted, deleted, added, or inserted in the amino acid sequence.

In an embodiment, the monoclonal antibody or antibody fragment thereof according to the present disclosure comprises:

(b')-1 a heavy chain variable region comprising
CDR1 comprising (or consisting of) an amino acid sequence of SEQ ID NO: 55,
CDR2 comprising (or consisting of) an amino acid sequence of SEQ ID NO: 56, and
CDR3 comprising (or consisting of) an amino acid sequence of SEQ ID NO: 57; and/or
a light chain variable region comprising
CDR1 comprising (or consisting of) an amino acid sequence of SEQ ID NO: 58,
CDR2 comprising (or consisting of) an amino acid sequence of SEQ ID NO: 59, and
CDR3 comprising (or consisting of) an amino acid sequence of SEQ ID NO: 60.

In an embodiment, the monoclonal antibody or antibody fragment thereof according to the present disclosure comprises:

(b')-2 a heavy chain variable region comprising
CDR1 comprising (or consisting of) an amino acid sequence of SEQ ID NO: 55,
CDR2 comprising (or consisting of) an amino acid sequence of SEQ ID NO: 56, and

CDR3 comprising (or consisting of) an amino acid sequence of SEQ ID NO: 57; and
a light chain variable region comprising
CDR1 comprising (or consisting of) an amino acid sequence of SEQ ID NO: 58,
CDR2 comprising (or consisting of) an amino acid sequence of SEQ ID NO: 59, and
CDR3 comprising (or consisting of) an amino acid sequence of SEQ ID NO: 60.

In an embodiment, the monoclonal antibody or antibody fragment thereof according to the present disclosure comprises:
(b")-1 a heavy chain variable region comprising (or consisting of) an amino acid sequence having a sequence identity of at least 90%, 95%, 97%, or 99% with an amino acid sequence of SEQ ID NO: 61 and/or a light chain variable region comprising (or consisting of) an amino acid sequence having a sequence identity of at least 90%, 95%, 97%, or 99% with an amino acid sequence of SEQ ID NO: 62.

In an embodiment, the monoclonal antibody or antibody fragment thereof according to the present disclosure comprises:
(b")-2 a heavy chain variable region comprising (or consisting of) an amino acid sequence having a sequence identity of at least 90%, 95%, 97%, or 99% with an amino acid sequence of SEQ ID NO: 61 and a light chain variable region comprising (or consisting of) an amino acid sequence having a sequence identity of at least 90%, 95%, 97%, or 99% with an amino acid sequence of SEQ ID NO: 62.

In an embodiment, the monoclonal antibody or antibody fragment thereof according to the present disclosure comprises:
(b′″)-1 a heavy chain variable region comprising (or consisting of) an amino acid sequence of SEQ ID NO: 61 and/or a light chain variable region comprising (or consisting of) an amino acid sequence of SEQ ID NO: 62.

In an embodiment, the monoclonal antibody or antibody fragment thereof according to the present disclosure comprises:
(b′″)-2 a heavy chain variable region comprising (or consisting of) an amino acid sequence of SEQ ID NO: 61 and a light chain variable region comprising (or consisting of) an amino acid sequence of SEQ ID NO: 62.

In an embodiment, the monoclonal antibody or antibody fragment thereof according to the present disclosure comprises:
(b′″′)-1 a heavy chain variable region comprising (or consisting of) an amino acid sequence of SEQ ID NO: 61 or an amino acid sequence in which one to several (e.g., 1 to 10, 1 to 5, 1 to 3) amino acid residues are independently substituted, deleted, added, or inserted in the amino acid sequence and/or a light chain variable region comprising (or consisting of) an amino acid sequence of SEQ ID NO: 62 or an amino acid sequence in which one to several (e.g., 1 to 10, 1 to 5, 1 to 3) amino acid residues are independently substituted, deleted, added, or inserted in the amino acid sequence.

In an embodiment, the monoclonal antibody or antibody fragment thereof according to the present disclosure comprises:
(b′″′)-2 a heavy chain variable region comprising (or consisting of) an amino acid sequence of SEQ ID NO: 61 or an amino acid sequence in which one to several (e.g., 1 to 10, 1 to 5, 1 to 3) amino acid residues are independently substituted, deleted, added, or inserted in the amino acid sequence and a light chain variable region comprising (or consisting of) an amino acid sequence of SEQ ID NO: 62 or an amino acid sequence in which one to several (e.g., 1 to 10, 1 to 5, 1 to 3) amino acid residues are independently substituted, deleted, added, or inserted in the amino acid sequence.

In an embodiment, each of the monoclonal antibodies or antibody fragments thereof in (b)-1 and -2, (b′)-1 and -2, (b")-1 and -2, (b′″)-1 and -2, and (b′″′)-1 and -2 (hereinafter, also referred to as a clone 3-derived antibody/antibody fragment) binds to a peptide consisting of an amino acid sequence of SEQ ID NO: 1 or SEQ ID NO: 19.

In an embodiment, each of the clone 3-derived antibodies/antibody fragments binds to a peptide consisting of an amino acid sequence of SEQ ID NO: 5 or SEQ ID NO: 23.

In an embodiment, each of the clone 3-derived antibodies/antibody fragments binds to a peptide consisting of an amino acid sequence of SEQ ID NO: 1 or SEQ ID NO: 19 and binds to a peptide consisting of an amino acid sequence of SEQ ID NO: 5 or SEQ ID NO: 23.

In an embodiment, each of the clone 3-derived antibodies/antibody fragments has protective capacity against infection for an influenza A virus H1 and/or H5 subtype(s).

In an embodiment, each of the clone 3-derived antibodies/antibody fragments is used for treating or preventing an infection with an influenza A virus HA subtype belonging to phylogenetic group 1 (e.g., two or more different influenza A virus HA subtypes belonging to phylogenetic group 1).

In an embodiment, each of the clone 3-derived antibodies/antibody fragments is used for treating or preventing an infection with an H1 and/or H5 subtype(s).

In an embodiment, each of the clone 3-derived antibodies/antibody fragments is used for detecting or quantifying a membrane fusion-type influenza HA antigen derived from an influenza A virus HA subtype belonging to phylogenetic group 1.

In an embodiment, each of the clone 3-derived antibodies/antibody fragments is used for detecting or quantifying a membrane fusion-type influenza HA antigen(s) derived from an H1 and/or H5 subtype(s).

In an embodiment, the monoclonal antibody or antibody fragment thereof according to the present disclosure comprises:

(c)-1 a heavy chain variable region comprising
CDR1 comprising (or consisting of) an amino acid sequence of SEQ ID NO: 63 or an amino acid sequence in which one or two amino acid residues are independently substituted, deleted, added, or inserted in the amino acid sequence,
CDR2 comprising (or consisting of) an amino acid sequence of SEQ ID NO: 64 or an amino acid sequence in which one or two amino acid residues are independently substituted, deleted, added, or inserted in the amino acid sequence, and
CDR3 comprising (or consisting of) an amino acid sequence of SEQ ID NO: 65 or an amino acid sequence in which one or two amino acid residues are independently substituted, deleted, added, or inserted in the amino acid sequence; and/or
a light chain variable region comprising
CDR1 comprising (or consisting of) an amino acid sequence of SEQ ID NO: 66 or an amino acid sequence in which one or two amino acid residues are independently substituted, deleted, added, or inserted in the amino acid sequence,
CDR2 comprising (or consisting of) an amino acid sequence of SEQ ID NO: 67 or an amino acid sequence in which one or two amino acid residues are independently substituted, deleted, added, or inserted in the amino acid sequence, and
CDR3 comprising (or consisting of) an amino acid sequence of SEQ ID NO: 68 or an amino acid sequence in which one or two amino acid residues are independently substituted, deleted, added, or inserted in the amino acid sequence.

In an embodiment, the monoclonal antibody or antibody fragment thereof according to the present disclosure comprises:

(c)-2 a heavy chain variable region comprising
CDR1 comprising (or consisting of) an amino acid sequence of SEQ ID NO: 63 or an amino acid sequence in which one or two amino acid residues are independently substituted, deleted, added, or inserted in the amino acid sequence,
CDR2 comprising (or consisting of) an amino acid sequence of SEQ ID NO: 64 or an amino acid sequence in which one or two amino acid residues are independently substituted, deleted, added, or inserted in the amino acid sequence, and
CDR3 comprising (or consisting of) an amino acid sequence of SEQ ID NO: 65 or an amino acid sequence in which one or two amino acid residues are independently substituted, deleted, added, or inserted in the amino acid sequence; and
a light chain variable region comprising
CDR1 comprising (or consisting of) an amino acid sequence of SEQ ID NO: 66 or an amino acid sequence in which one or two amino acid residues are independently substituted, deleted, added, or inserted in the amino acid sequence,
CDR2 comprising (or consisting of) an amino acid sequence of SEQ ID NO: 67 or an amino acid sequence in which one or two amino acid residues are independently substituted, deleted, added, or inserted in the amino acid sequence, and
CDR3 comprising (or consisting of) an amino acid sequence of SEQ ID NO: 68 or an amino acid sequence in which one or two amino acid residues are independently substituted, deleted, added, or inserted in the amino acid sequence.

In an embodiment, the monoclonal antibody or antibody fragment thereof according to the present disclosure comprises:

(c')-1 a heavy chain variable region comprising
CDR1 comprising (or consisting of) an amino acid sequence of SEQ ID NO: 63,
CDR2 comprising (or consisting of) an amino acid sequence of SEQ ID NO: 64, and
CDR3 comprising (or consisting of) an amino acid sequence of SEQ ID NO: 65; and/or
a light chain variable region comprising
CDR1 comprising (or consisting of) an amino acid sequence of SEQ ID NO: 66,
CDR2 comprising (or consisting of) an amino acid sequence of SEQ ID NO: 67, and
CDR3 comprising (or consisting of) an amino acid sequence of SEQ ID NO: 68.

In an embodiment, the monoclonal antibody or antibody fragment thereof according to the present disclosure comprises:

(c')-2 a heavy chain variable region comprising
CDR1 comprising (or consisting of) an amino acid sequence of SEQ ID NO: 63,
CDR2 comprising (or consisting of) an amino acid sequence of SEQ ID NO: 64, and
CDR3 comprising (or consisting of) an amino acid sequence of SEQ ID NO: 65; and

a light chain variable region comprising
CDR1 comprising (or consisting of) an amino acid sequence of SEQ ID NO: 66,
CDR2 comprising (or consisting of) an amino acid sequence of SEQ ID NO: 67, and
CDR3 comprising (or consisting of) an amino acid sequence of SEQ ID NO: 68.

In an embodiment, the monoclonal antibody or antibody fragment thereof according to the present disclosure comprises:
(c")-1 a heavy chain variable region comprising (or consisting of) an amino acid sequence having a sequence identity of at least 90%, 95%, 97%, or 99% with an amino acid sequence of SEQ ID NO: 69 and/or a light chain variable region comprising (or consisting of) an amino acid sequence having a sequence identity of at least 90%, 95%, 97%, or 99% with an amino acid sequence of SEQ ID NO: 70.

In an embodiment, the monoclonal antibody or antibody fragment thereof according to the present disclosure comprises:
(c")-2 a heavy chain variable region comprising (or consisting of) an amino acid sequence having a sequence identity of at least 90%, 95%, 97%, or 99% with an amino acid sequence of SEQ ID NO: 69 and a light chain variable region comprising (or consisting of) an amino acid sequence having a sequence identity of at least 90%, 95%, 97%, or 99% with an amino acid sequence of SEQ ID NO: 70.

In an embodiment, the monoclonal antibody or antibody fragment thereof according to the present disclosure comprises:
(c‴)-1 a heavy chain variable region comprising (or consisting of) an amino acid sequence of SEQ ID NO: 69 and/or a light chain variable region comprising (or consisting of) an amino acid sequence of SEQ ID NO: 70.

In an embodiment, the monoclonal antibody or antibody fragment thereof according to the present disclosure comprises:
(c‴)-2 a heavy chain variable region comprising (or consisting of) an amino acid sequence of SEQ ID NO: 69 and a light chain variable region comprising (or consisting of) an amino acid sequence of SEQ ID NO: 70.

In an embodiment, the monoclonal antibody or antibody fragment thereof according to the present disclosure comprises:
(c⁗)-1 a heavy chain variable region comprising (or consisting of) an amino acid sequence of SEQ ID NO: 69 or an amino acid sequence in which one to several (e.g., 1 to 10, 1 to 5, 1 to 3) amino acid residues are independently substituted, deleted, added, or inserted in the amino acid sequence and/or a light chain variable region comprising (or consisting of) an amino acid sequence of SEQ ID NO: 70 or an amino acid sequence in which one to several (e.g., 1 to 10, 1 to 5, 1 to 3) amino acid residues are independently substituted, deleted, added, or inserted in the amino acid sequence.

In an embodiment, the monoclonal antibody or antibody fragment thereof according to the present disclosure comprises:
(c⁗)-2 a heavy chain variable region comprising (or consisting of) an amino acid sequence of SEQ ID NO: 69 or an amino acid sequence in which one to several (e.g., 1 to 10, 1 to 5, 1 to 3) amino acid residues are independently substituted, deleted, added, or inserted in the amino acid sequence and a light chain variable region comprising (or consisting of) an amino acid sequence of SEQ ID NO: 70 or an amino acid sequence in which one to several (e.g., 1 to 10, 1 to 5, 1 to 3) amino acid residues are independently substituted, deleted, added, or inserted in the amino acid sequence.

In an embodiment, each of the monoclonal antibodies or antibody fragments thereof in (c)-1 and -2, (c')-1 and -2, (c")-1 and -2, (c‴)-1 and -2, and (c⁗)-1 and -2 (hereinafter, also referred to as a clone 4-derived antibody/antibody fragment or clone 8-derived antibody/antibody fragment) binds to a peptide consisting of an amino acid sequence of SEQ ID NO: 1 or SEQ ID NO: 19.

In an embodiment, each of the clone 4-derived antibodies/antibody fragments binds to a peptide consisting of an amino acid sequence of SEQ ID NO: 5 or SEQ ID NO: 23.

In an embodiment, each of the clone 4-derived antibodies/antibody fragments binds to a peptide consisting of an amino acid sequence of SEQ ID NO: 1 or SEQ ID NO: 19 and binds to a peptide consisting of an amino acid sequence of SEQ ID NO: 5 or SEQ ID NO: 23.

In an embodiment, each of the clone 4-derived antibodies/antibody fragments has protective capacity against infection for an influenza A virus H1 and/or H5 subtype(s).

In an embodiment, each of the clone 4-derived antibodies/antibody fragments is used for treating or preventing an infection with an influenza A virus HA subtype belonging to phylogenetic group 1 (e.g., two or more different influenza A virus HA subtypes belonging to phylogenetic group 1).

In an embodiment, each of the clone 4-derived antibodies/antibody fragments is used for treating or preventing an infection with an H1 and/or H5 subtype(s).

In an embodiment, each of the clone 4-derived antibodies/antibody fragments is used for detecting or quantifying a membrane fusion-type influenza HA antigen derived from an influenza A virus HA subtype belonging to phylogenetic group 1.

In an embodiment, each of the clone 4-derived antibodies/antibody fragments is used for detecting or quantifying a membrane fusion-type influenza HA antigen(s) derived from an H1 and/or H5 subtype(s).

In an embodiment, the monoclonal antibody or antibody fragment thereof according to the present disclosure comprises:

(d)-1 a heavy chain variable region comprising
CDR1 comprising (or consisting of) an amino acid sequence of SEQ ID NO: 71 or an amino acid sequence in which one or two amino acid residues are independently substituted, deleted, added, or inserted in the amino acid sequence, CDR2 comprising (or consisting of) an amino acid sequence of SEQ ID NO: 72 or an amino acid sequence in which one or two amino acid residues are independently substituted, deleted, added, or inserted in the amino acid sequence, and CDR3 comprising (or consisting of) an amino acid sequence of SEQ ID NO: 73 or an amino acid sequence in which one or two amino acid residues are independently substituted, deleted, added, or inserted in the amino acid sequence; and/or
a light chain variable region comprising
CDR1 comprising (or consisting of) an amino acid sequence of SEQ ID NO: 74 or an amino acid sequence in which one or two amino acid residues are independently substituted, deleted, added, or inserted in the amino acid sequence, CDR2 comprising (or consisting of) an amino acid sequence of SEQ ID NO: 75 or an amino acid sequence in which one or two amino acid residues are independently substituted, deleted, added, or inserted in the amino acid sequence, and CDR3 comprising (or consisting of) an amino acid sequence of SEQ ID NO: 76 or an amino acid sequence in which one or two amino acid residues are independently substituted, deleted, added, or inserted in the amino acid sequence.

In an embodiment, the monoclonal antibody or antibody fragment thereof according to the present disclosure comprises:

(d)-2 a heavy chain variable region comprising
CDR1 comprising (or consisting of) an amino acid sequence of SEQ ID NO: 71 or an amino acid sequence in which one or two amino acid residues are independently substituted, deleted, added, or inserted in the amino acid sequence, CDR2 comprising (or consisting of) an amino acid sequence of SEQ ID NO: 72 or an amino acid sequence in which one or two amino acid residues are independently substituted, deleted, added, or inserted in the amino acid sequence, and CDR3 comprising (or consisting of) an amino acid sequence of SEQ ID NO: 73 or an amino acid sequence in which one or two amino acid residues are independently substituted, deleted, added, or inserted in the amino acid sequence; and
a light chain variable region comprising
CDR1 comprising (or consisting of) an amino acid sequence of SEQ ID NO: 74 or an amino acid sequence in which one or two amino acid residues are independently substituted, deleted, added, or inserted in the amino acid sequence, CDR2 comprising (or consisting of) an amino acid sequence of SEQ ID NO: 75 or an amino acid sequence in which one or two amino acid residues are independently substituted, deleted, added, or inserted in the amino acid sequence, and CDR3 comprising (or consisting of) an amino acid sequence of SEQ ID NO: 76 or an amino acid sequence in which one or two amino acid residues are independently substituted, deleted, added, or inserted in the amino acid sequence.

In an embodiment, the monoclonal antibody or antibody fragment thereof according to the present disclosure comprises:

(d')-1 a heavy chain variable region comprising
CDR1 comprising (or consisting of) an amino acid sequence of SEQ ID NO: 71,
CDR2 comprising (or consisting of) an amino acid sequence of SEQ ID NO: 72, and
CDR3 comprising (or consisting of) an amino acid sequence of SEQ ID NO: 73; and/or
a light chain variable region comprising
CDR1 comprising (or consisting of) an amino acid sequence of SEQ ID NO: 74,
CDR2 comprising (or consisting of) an amino acid sequence of SEQ ID NO: 75, and
CDR3 comprising (or consisting of) an amino acid sequence of SEQ ID NO: 76.

In an embodiment, the monoclonal antibody or antibody fragment thereof according to the present disclosure comprises:

(d')-2 a heavy chain variable region comprising
CDR1 comprising (or consisting of) an amino acid sequence of SEQ ID NO: 71,
CDR2 comprising (or consisting of) an amino acid sequence of SEQ ID NO: 72, and
CDR3 comprising (or consisting of) an amino acid sequence of SEQ ID NO: 73; and

a light chain variable region comprising

CDR1 comprising (or consisting of) an amino acid sequence of SEQ ID NO: 74,

CDR2 comprising (or consisting of) an amino acid sequence of SEQ ID NO: 75, and

CDR3 comprising (or consisting of) an amino acid sequence of SEQ ID NO: 76.

In an embodiment, the monoclonal antibody or antibody fragment thereof according to the present disclosure comprises:

(d")-1 a heavy chain variable region comprising (or consisting of) an amino acid sequence having a sequence identity of at least 90%, 95%, 97%, or 99% with an amino acid sequence of SEQ ID NO: 77 and/or a light chain variable region comprising (or consisting of) an amino acid sequence having a sequence identity of at least 90%, 95%, 97%, or 99% with an amino acid sequence of SEQ ID NO: 78.

In an embodiment, the monoclonal antibody or antibody fragment thereof according to the present disclosure comprises:

(d")-2 a heavy chain variable region comprising (or consisting of) an amino acid sequence having a sequence identity of at least 90%, 95%, 97%, or 99% with an amino acid sequence of SEQ ID NO: 77 and a light chain variable region comprising (or consisting of) an amino acid sequence having a sequence identity of at least 90%, 95%, 97%, or 99% with an amino acid sequence of SEQ ID NO: 78.

In an embodiment, the monoclonal antibody or antibody fragment thereof according to the present disclosure comprises:

(d‴)-1 a heavy chain variable region comprising (or consisting of) an amino acid sequence of SEQ ID NO: 77 and/or a light chain variable region comprising (or consisting of) an amino acid sequence of SEQ ID NO: 78.

In an embodiment, the monoclonal antibody or antibody fragment thereof according to the present disclosure comprises:

(d‴)-2 a heavy chain variable region comprising (or consisting of) an amino acid sequence of SEQ ID NO: 77 and a light chain variable region comprising (or consisting of) an amino acid sequence of SEQ ID NO: 78.

In an embodiment, the monoclonal antibody or antibody fragment thereof according to the present disclosure comprises:

(d‴′)-1 a heavy chain variable region comprising (or consisting of) an amino acid sequence of SEQ ID NO: 77 or an amino acid sequence in which one to several (e.g., 1 to 10, 1 to 5, 1 to 3) amino acid residues are independently substituted, deleted, added, or inserted in the amino acid sequence and/or a light chain variable region comprising (or consisting of) an amino acid sequence of SEQ ID NO: 78 or an amino acid sequence in which one to several (e.g., 1 to 10, 1 to 5, 1 to 3) amino acid residues are independently substituted, deleted, added, or inserted in the amino acid sequence.

In an embodiment, the monoclonal antibody or antibody fragment thereof according to the present disclosure comprises:

(d‴′)-2 a heavy chain variable region comprising (or consisting of) an amino acid sequence of SEQ ID NO: 77 or an amino acid sequence in which one to several (e.g., 1 to 10, 1 to 5, 1 to 3) amino acid residues are independently substituted, deleted, added, or inserted in the amino acid sequence and a light chain variable region comprising (or consisting of) an amino acid sequence of SEQ ID NO: 78 or an amino acid sequence in which one to several (e.g., 1 to 10, 1 to 5, 1 to 3) amino acid residues are independently substituted, deleted, added, or inserted in the amino acid sequence.

In an embodiment, each of the monoclonal antibodies or antibody fragments thereof in (d)-1 and -2, (d')-1 and -2, (d")-1 and -2, (d‴)-1 and -2, and (d‴′)-1 and -2 (hereinafter, also referred to as a clone 5-derived antibody/antibody fragment) binds to a peptide consisting of an amino acid sequence of SEQ ID NO: 3 or SEQ ID NO: 21.

In an embodiment, each of the clone 5-derived antibodies/antibody fragments binds to a peptide consisting of an amino acid sequence of SEQ ID NO: 7 or SEQ ID NO: 25.

In an embodiment, each of the clone 5-derived antibodies/antibody fragments binds to a peptide consisting of an amino acid sequence of SEQ ID NO: 3 or SEQ ID NO: 21 and binds to a peptide consisting of an amino acid sequence of SEQ ID NO: 7 or SEQ ID NO: 25.

In an embodiment, each of the clone 5-derived antibodies/antibody fragments has protective capacity against infection for an influenza A virus H3 and/or H7 subtype(s).

In an embodiment, each of the clone 5-derived antibodies/antibody fragments is used for treating or preventing an infection with an influenza A virus HA subtype belonging to phylogenetic group 2 (e.g., two or more different influenza A virus HA subtypes belonging to phylogenetic group 2).

In an embodiment, each of the clone 5-derived antibodies/antibody fragments is used for treating or preventing an infection with an H3 and/or H7 subtype(s).

In an embodiment, each of the clone 5-derived antibodies/antibody fragments is used for detecting or quantifying a membrane fusion-type influenza HA antigen derived from an influenza A virus HA subtype belonging to phylogenetic group 2.

In an embodiment, each of the clone 5-derived antibodies/antibody fragments is used for detecting or quantifying a membrane fusion-type influenza HA antigen(s) derived from an H3 and/or H7 subtype(s).

In an embodiment, the monoclonal antibody or antibody fragment thereof according to the present disclosure comprises:

(e)-1 a heavy chain variable region comprising
CDR1 comprising (or consisting of) an amino acid sequence of SEQ ID NO: 79 or an amino acid sequence in which one or two amino acid residues are independently substituted, deleted, added, or inserted in the amino acid sequence,
CDR2 comprising (or consisting of) an amino acid sequence of SEQ ID NO: 80 or an amino acid sequence in which one or two amino acid residues are independently substituted, deleted, added, or inserted in the amino acid sequence, and
CDR3 comprising (or consisting of) an amino acid sequence of SEQ ID NO: 81 or an amino acid sequence in which one or two amino acid residues are independently substituted, deleted, added, or inserted in the amino acid sequence; and/or
a light chain variable region comprising
CDR1 comprising (or consisting of) an amino acid sequence of SEQ ID NO: 82 or an amino acid sequence in which one or two amino acid residues are independently substituted, deleted, added, or inserted in the amino acid sequence,
CDR2 comprising (or consisting of) an amino acid sequence of SEQ ID NO: 83 or an amino acid sequence in which one or two amino acid residues are independently substituted, deleted, added, or inserted in the amino acid sequence, and
CDR3 comprising (or consisting of) an amino acid sequence of SEQ ID NO: 84 or an amino acid sequence in which one or two amino acid residues are independently substituted, deleted, added, or inserted in the amino acid sequence.

In an embodiment, the monoclonal antibody or antibody fragment thereof according to the present disclosure comprises:

(e)-2 a heavy chain variable region comprising
CDR1 comprising (or consisting of) an amino acid sequence of SEQ ID NO: 79 or an amino acid sequence in which one or two amino acid residues are independently substituted, deleted, added, or inserted in the amino acid sequence,
CDR2 comprising (or consisting of) an amino acid sequence of SEQ ID NO: 80 or an amino acid sequence in which one or two amino acid residues are independently substituted, deleted, added, or inserted in the amino acid sequence, and
CDR3 comprising (or consisting of) an amino acid sequence of SEQ ID NO: 81 or an amino acid sequence in which one or two amino acid residues are independently substituted, deleted, added, or inserted in the amino acid sequence; and
a light chain variable region comprising
CDR1 comprising (or consisting of) an amino acid sequence of SEQ ID NO: 82 or an amino acid sequence in which one or two amino acid residues are independently substituted, deleted, added, or inserted in the amino acid sequence,
CDR2 comprising (or consisting of) an amino acid sequence of SEQ ID NO: 83 or an amino acid sequence in which one or two amino acid residues are independently substituted, deleted, added, or inserted in the amino acid sequence, and
CDR3 comprising (or consisting of) an amino acid sequence of SEQ ID NO: 84 or an amino acid sequence in which one or two amino acid residues are independently substituted, deleted, added, or inserted in the amino acid sequence.

In an embodiment, the monoclonal antibody or antibody fragment thereof according to the present disclosure comprises:

(e')-1 a heavy chain variable region comprising
CDR1 comprising (or consisting of) an amino acid sequence of SEQ ID NO: 79,
CDR2 comprising (or consisting of) an amino acid sequence of SEQ ID NO: 80, and
CDR3 comprising (or consisting of) an amino acid sequence of SEQ ID NO: 81; and/or
a light chain variable region comprising
CDR1 comprising (or consisting of) an amino acid sequence of SEQ ID NO: 82,
CDR2 comprising (or consisting of) an amino acid sequence of SEQ ID NO: 83, and
CDR3 comprising (or consisting of) an amino acid sequence of SEQ ID NO: 84.

In an embodiment, the monoclonal antibody or antibody fragment thereof according to the present disclosure comprises:

(e')-2 a heavy chain variable region comprising
CDR1 comprising (or consisting of) an amino acid sequence of SEQ ID NO: 79,
CDR2 comprising (or consisting of) an amino acid sequence of SEQ ID NO: 80, and
CDR3 comprising (or consisting of) an amino acid sequence of SEQ ID NO: 81; and
a light chain variable region comprising

CDR1 comprising (or consisting of) an amino acid sequence of SEQ ID NO: 82,
CDR2 comprising (or consisting of) an amino acid sequence of SEQ ID NO: 83, and
CDR3 comprising (or consisting of) an amino acid sequence of SEQ ID NO: 84.

In an embodiment, the monoclonal antibody or antibody fragment thereof according to the present disclosure comprises:
(e")-1 a heavy chain variable region comprising (or consisting of) an amino acid sequence having a sequence identity of at least 90%, 95%, 97%, or 99% with an amino acid sequence of SEQ ID NO: 85 and/or a light chain variable region comprising (or consisting of) an amino acid sequence having a sequence identity of at least 90%, 95%, 97%, or 99% with an amino acid sequence of SEQ ID NO: 86.

In an embodiment, the monoclonal antibody or antibody fragment thereof according to the present disclosure comprises:
(e")-2 a heavy chain variable region comprising (or consisting of) an amino acid sequence having a sequence identity of at least 90%, 95%, 97%, or 99% with an amino acid sequence of SEQ ID NO: 85 and a light chain variable region comprising (or consisting of) an amino acid sequence having a sequence identity of at least 90%, 95%, 97%, or 99% with an amino acid sequence of SEQ ID NO: 86.

In an embodiment, the monoclonal antibody or antibody fragment thereof according to the present disclosure comprises:
(e‴)-1 a heavy chain variable region comprising (or consisting of) an amino acid sequence of SEQ ID NO: 85 and/or a light chain variable region comprising (or consisting of) an amino acid sequence of SEQ ID NO: 86.

In an embodiment, the monoclonal antibody or antibody fragment thereof according to the present disclosure comprises:
(e‴)-2 a heavy chain variable region comprising (or consisting of) an amino acid sequence of SEQ ID NO: 85 and a light chain variable region comprising (or consisting of) an amino acid sequence of SEQ ID NO: 86.

In an embodiment, the monoclonal antibody or antibody fragment thereof according to the present disclosure comprises:
(e‴')-1 a heavy chain variable region comprising (or consisting of) an amino acid sequence of SEQ ID NO: 85 or an amino acid sequence in which one to several (e.g., 1 to 10, 1 to 5, 1 to 3) amino acid residues are independently substituted, deleted, added, or inserted in the amino acid sequence and/or a light chain variable region comprising (or consisting of) an amino acid sequence of SEQ ID NO: 86 or an amino acid sequence in which one to several (e.g., 1 to 10, 1 to 5, 1 to 3) amino acid residues are independently substituted, deleted, added, or inserted in the amino acid sequence.

In an embodiment, the monoclonal antibody or antibody fragment thereof according to the present disclosure comprises:
(e"")-2 a heavy chain variable region comprising (or consisting of) an amino acid sequence of SEQ ID NO: 85 or an amino acid sequence in which one to several (e.g., 1 to 10, 1 to 5, 1 to 3) amino acid residues are independently substituted, deleted, added, or inserted in the amino acid sequence and a light chain variable region comprising (or consisting of) an amino acid sequence of SEQ ID NO: 86 or an amino acid sequence in which one to several (e.g., 1 to 10, 1 to 5, 1 to 3) amino acid residues are independently substituted, deleted, added, or inserted in the amino acid sequence.

In an embodiment, each of the monoclonal antibodies or antibody fragments thereof in (e)-1 and -2, (e')-1 and -2, (e")-1 and -2, (e‴)-1 and -2, and (e‴')-1 and -2 (hereinafter, also referred to as a clone 6-derived antibody/antibody fragment) binds to a peptide consisting of an amino acid sequence of SEQ ID NO: 3 or SEQ ID NO: 21.

In an embodiment, each of the clone 6-derived antibodies/antibody fragments binds to a peptide consisting of an amino acid sequence of SEQ ID NO: 7 or SEQ ID NO: 25.

In an embodiment, each of the clone 6-derived antibodies/antibody fragments binds to a peptide consisting of an amino acid sequence of SEQ ID NO: 3 or SEQ ID NO: 21 and binds to a peptide consisting of an amino acid sequence of SEQ ID NO: 7 or SEQ ID NO: 25.

In an embodiment, each of the clone 6-derived antibodies/antibody fragments binds to a peptide consisting of an amino acid sequence of SEQ ID NO: 1 or SEQ ID NO: 19.

In an embodiment, each of the clone 6-derived antibodies/antibody fragments binds to a peptide consisting of an amino acid sequence of SEQ ID NO: 5 or SEQ ID NO: 23.

In an embodiment, each of the clone 6-derived antibodies/antibody fragments binds to a peptide consisting of an amino acid sequence of SEQ ID NO: 1 or SEQ ID NO: 19 and binds to a peptide consisting of an amino acid sequence of SEQ ID NO: 5 or SEQ ID NO: 23.

In an embodiment, each of the clone 6-derived antibodies/antibody fragments binds to a peptide consisting of an amino acid sequence of SEQ ID NO: 3 or SEQ ID NO: 21, binds to a peptide consisting of an amino acid sequence of SEQ ID NO: 7 or SEQ ID NO: 25, binds to a peptide consisting of an amino acid sequence of SEQ ID NO: 1 or SEQ ID NO: 19, and binds to a peptide consisting of an amino acid sequence of SEQ ID NO: 5 or SEQ ID NO: 23.

In an embodiment, each of the clone 6-derived antibodies/antibody fragments has protective capacity against infection for at least one subtype selected from the group consisting of an influenza A virus H3, H7, H1, and H5 subtypes (e.g., an H3 and/or H1 subtype(s)).

In an embodiment, each of the clone 6-derived antibodies/antibody fragments is used for treating or preventing an infection with an influenza A virus HA subtype(s) belonging to phylogenetic group(s) 1 and/or 2 (e.g., two or more different influenza A virus HA subtypes belonging to phylogenetic group 1 and/or two or more different influenza A virus HA subtypes belonging to phylogenetic group 2).

In an embodiment, each of the clone 6-derived antibodies/antibody fragments is used for treating or preventing an infection with an H1, H3, H5, and/or H7 subtype(s).

In an embodiment, each of the clone 6-derived antibodies/antibody fragments is used for detecting or quantifying a membrane fusion-type influenza HA antigen(s) derived from an influenza A virus HA subtype(s) belonging to phylogenetic group(s) 1 and/or 2.

In an embodiment, each of the clone 6-derived antibodies/antibody fragments is used for detecting or quantifying a membrane fusion-type influenza HA antigen(s) derived from an H1, H3, H5, and/or H7 subtype(s).

In an embodiment, each of the clone 6-derived antibodies/antibody fragments has protective capacity against infection for at least one subtype selected from the group consisting of an influenza A virus H1, H2, H5, H6, H8, H9, H11, H16, H17, H18, H3, H4, H7, H10, H14, and H15 subtypes (e.g., H1, H2, H5, H6, H17, H18, H3, H4, H7, H10, H14, and H15 subtypes).

In an embodiment, each of the clone 6-derived antibodies/antibody fragments is used for treating or preventing an infection with at least one subtype selected from the group consisting of an influenza A virus H1, H2, H5, H6, H8, H9, H11, H16, H17, H18, H3, H4, H7, H10, H14, and H15 subtypes (e.g., H1, H2, H5, H6, H17, H18, H3, H4, H7, H10, H14, and H15 subtypes).

In an embodiment, each of the clone 6-derived antibodies/antibody fragments is used for detecting or quantifying a membrane fusion-type influenza HA antigen derived from at least one subtype selected from the group consisting of H1, H2, H5, H6, H8, H9, H11, H16, H17, H18, H3, H4, H7, H10, H14, and H15 subtypes (e.g., H1, H2, H5, H6, H17, H18, H3, H4, H7, H10, H14, and H15 subtypes).

In an embodiment, the monoclonal antibody or antibody fragment thereof according to the present disclosure comprises:

(f)-1 a heavy chain variable region comprising
CDR1 comprising (or consisting of) an amino acid sequence of SEQ ID NO: 87 or an amino acid sequence in which one or two amino acid residues are independently substituted, deleted, added, or inserted in the amino acid sequence, CDR2 comprising (or consisting of) an amino acid sequence of SEQ ID NO: 88 or an amino acid sequence in which one or two amino acid residues are independently substituted, deleted, added, or inserted in the amino acid sequence, and CDR3 comprising (or consisting of) an amino acid sequence of SEQ ID NO: 89 or an amino acid sequence in which one or two amino acid residues are independently substituted, deleted, added, or inserted in the amino acid sequence; and/or
a light chain variable region comprising
CDR1 comprising (or consisting of) an amino acid sequence of SEQ ID NO: 90 or an amino acid sequence in which one or two amino acid residues are independently substituted, deleted, added, or inserted in the amino acid sequence, CDR2 comprising (or consisting of) an amino acid sequence of SEQ ID NO: 91 or an amino acid sequence in which one or two amino acid residues are independently substituted, deleted, added, or inserted in the amino acid sequence, and CDR3 comprising (or consisting of) an amino acid sequence of SEQ ID NO: 92 or an amino acid sequence in which one or two amino acid residues are independently substituted, deleted, added, or inserted in the amino acid sequence.

In an embodiment, the monoclonal antibody or antibody fragment thereof according to the present disclosure comprises:

(f)-2 a heavy chain variable region comprising
CDR1 comprising (or consisting of) an amino acid sequence of SEQ ID NO: 87 or an amino acid sequence in which one or two amino acid residues are independently substituted, deleted, added, or inserted in the amino acid sequence, CDR2 comprising (or consisting of) an amino acid sequence of SEQ ID NO: 88 or an amino acid sequence in which one or two amino acid residues are independently substituted, deleted, added, or inserted in the amino acid sequence, and CDR3 comprising (or consisting of) an amino acid sequence of SEQ ID NO: 89 or an amino acid sequence in which one or two amino acid residues are independently substituted, deleted, added, or inserted in the amino acid sequence; and
a light chain variable region comprising
CDR1 comprising (or consisting of) an amino acid sequence of SEQ ID NO: 90 or an amino acid sequence in which one or two amino acid residues are independently substituted, deleted, added, or inserted in the amino acid sequence, CDR2 comprising (or consisting of) an amino acid sequence of SEQ ID NO: 91 or an amino acid sequence in which one or two amino acid residues are independently substituted, deleted, added, or inserted in the amino acid sequence, and CDR3 comprising (or consisting of) an amino acid sequence of SEQ ID NO: 92 or an amino acid sequence in which one or two amino acid residues are independently substituted, deleted, added, or inserted in the amino acid sequence.

In an embodiment, the monoclonal antibody or antibody fragment thereof according to the present disclosure comprises:

(f)-1 a heavy chain variable region comprising
CDR1 comprising (or consisting of) an amino acid sequence of SEQ ID NO: 87,
CDR2 comprising (or consisting of) an amino acid sequence of SEQ ID NO: 88, and
CDR3 comprising (or consisting of) an amino acid sequence of SEQ ID NO: 89; and/or
a light chain variable region comprising
CDR1 comprising (or consisting of) an amino acid sequence of SEQ ID NO: 90,
CDR2 comprising (or consisting of) an amino acid sequence of SEQ ID NO: 91, and
CDR3 comprising (or consisting of) an amino acid sequence of SEQ ID NO: 92.

In an embodiment, the monoclonal antibody or antibody fragment thereof according to the present disclosure comprises:

(f)-2 a heavy chain variable region comprising
CDR1 comprising (or consisting of) an amino acid sequence of SEQ ID NO: 87,
CDR2 comprising (or consisting of) an amino acid sequence of SEQ ID NO: 88, and
CDR3 comprising (or consisting of) an amino acid sequence of SEQ ID NO: 89; and
a light chain variable region comprising
CDR1 comprising (or consisting of) an amino acid sequence of SEQ ID NO: 90,
CDR2 comprising (or consisting of) an amino acid sequence of SEQ ID NO: 91, and
CDR3 comprising (or consisting of) an amino acid sequence of SEQ ID NO: 92.

In an embodiment, the monoclonal antibody or antibody fragment thereof according to the present disclosure comprises:
(f")-1 a heavy chain variable region comprising (or consisting of) an amino acid sequence having a sequence identity of at least 90%, 95%, 97%, or 99% with an amino acid sequence of SEQ ID NO: 93 and/or a light chain variable region comprising (or consisting of) an amino acid sequence having a sequence identity of at least 90%, 95%, 97%, or 99% with an amino acid sequence of SEQ ID NO: 94.

In an embodiment, the monoclonal antibody or antibody fragment thereof according to the present disclosure comprises:
(f")-2 a heavy chain variable region comprising (or consisting of) an amino acid sequence having a sequence identity of at least 90%, 95%, 97%, or 99% with an amino acid sequence of SEQ ID NO: 93 and a light chain variable region comprising (or consisting of) an amino acid sequence having a sequence identity of at least 90%, 95%, 97%, or 99% with an amino acid sequence of SEQ ID NO: 94.

In an embodiment, the monoclonal antibody or antibody fragment thereof according to the present disclosure comprises:
(f")-1 a heavy chain variable region comprising (or consisting of) an amino acid sequence of SEQ ID NO: 93 and/or a light chain variable region comprising (or consisting of) an amino acid sequence of SEQ ID NO: 94.

In an embodiment, the monoclonal antibody or antibody fragment thereof according to the present disclosure comprises:
(f")-2 a heavy chain variable region comprising (or consisting of) an amino acid sequence of SEQ ID NO: 93 and a light chain variable region comprising (or consisting of) an amino acid sequence of SEQ ID NO: 94.

In an embodiment, the monoclonal antibody or antibody fragment thereof according to the present disclosure comprises:
(f‴)-1 a heavy chain variable region comprising (or consisting of) an amino acid sequence of SEQ ID NO: 93 or an amino acid sequence in which one to several (e.g., 1 to 10, 1 to 5, 1 to 3) amino acid residues are independently substituted, deleted, added, or inserted in the amino acid sequence and/or a light chain variable region comprising (or consisting of) an amino acid sequence of SEQ ID NO: 94 or an amino acid sequence in which one to several (e.g., 1 to 10, 1 to 5, 1 to 3) amino acid residues are independently substituted, deleted, added, or inserted in the amino acid sequence.

In an embodiment, the monoclonal antibody or antibody fragment thereof according to the present disclosure comprises:
(f‴)-2 a heavy chain variable region comprising (or consisting of) an amino acid sequence of SEQ ID NO: 93 or an amino acid sequence in which one to several (e.g., 1 to 10, 1 to 5, 1 to 3) amino acid residues are independently substituted, deleted, added, or inserted in the amino acid sequence and a light chain variable region comprising (or consisting of) an amino acid sequence of SEQ ID NO: 94 or an amino acid sequence in which one to several (e.g., 1 to 10, 1 to 5, 1 to 3) amino acid residues are independently substituted, deleted, added, or inserted in the amino acid sequence.

In an embodiment, each of the monoclonal antibodies or antibody fragments thereof in (f)-1 and -2, (f)-1 and -2, (f")-1 and -2, (f'')-1 and -2, and (f''''')-1 and -2 (hereinafter, also referred to as a clone 7-derived antibody/antibody fragment) binds to a peptide consisting of an amino acid sequence of SEQ ID NO: 3 or SEQ ID NO: 21.

In an embodiment, each of the clone 7-derived antibodies/antibody fragments binds to a peptide consisting of an amino acid sequence of SEQ ID NO: 7 or SEQ ID NO: 25.

In an embodiment, each of the clone 7-derived antibodies/antibody fragments binds to a peptide consisting of an amino acid sequence of SEQ ID NO: 3 or SEQ ID NO: 21 and binds to a peptide consisting of an amino acid sequence of SEQ ID NO: 7 or SEQ ID NO: 25.

In an embodiment, each of the clone 7-derived antibodies/antibody fragments binds to a peptide consisting of an amino acid sequence of SEQ ID NO: 1 or SEQ ID NO: 19.

In an embodiment, each of the clone 7-derived antibodies/antibody fragments binds to a peptide consisting of an amino acid sequence of SEQ ID NO: 5 or SEQ ID NO: 23.

In an embodiment, each of the clone 7-derived antibodies/antibody fragments binds to a peptide consisting of an amino acid sequence of SEQ ID NO: 1 or SEQ ID NO: 19 and binds to a peptide consisting of an amino acid sequence of SEQ ID NO: 5 or SEQ ID NO: 23.

In an embodiment, each of the clone 7-derived antibodies/antibody fragments binds to a peptide consisting of an amino acid sequence of SEQ ID NO: 3 or SEQ ID NO: 21, binds to a peptide consisting of an amino acid sequence of SEQ ID NO: 7 or SEQ ID NO: 25, binds to a peptide consisting of an amino acid sequence of SEQ ID NO: 1 or SEQ ID NO: 19, and binds to a peptide consisting of an amino acid sequence of SEQ ID NO: 5 or SEQ ID NO: 23.

In an embodiment, each of the clone 7-derived antibodies/antibody fragments has protective capacity against infection for at least one subtype selected from the group consisting of an influenza A virus H3, H7, H1, and H5 subtypes (e.g., an H3 and/or H1 subtype(s)).

In an embodiment, each of the clone 7-derived antibodies/antibody fragments is used for treating or preventing an infection with an influenza A virus HA subtype(s) belonging to phylogenetic group(s) 1 and/or 2 (e.g., two or more different influenza A virus HA subtypes belonging to phylogenetic group 1 and/or two or more different influenza A virus HA subtypes belonging to phylogenetic group 2).

In an embodiment, each of the clone 7-derived antibodies/antibody fragments is used for treating or preventing an infection with an H1, H3, H5, and/or H7 subtype(s).

In an embodiment, each of the clone 7-derived antibodies/antibody fragments is used for detecting or quantifying a membrane fusion-type influenza HA antigen(s) derived from an influenza A virus HA subtype(s) belonging to phylogenetic group(s) 1 and/or 2.

In an embodiment, each of the clone 7-derived antibodies/antibody fragments is used for detecting or quantifying a membrane fusion-type influenza HA antigen(s) derived from an H1, H3, H5, and/or H7 subtype(s).

In an embodiment, each of the clone 7-derived antibodies/antibody fragments has protective capacity against infection for at least one subtype selected from the group consisting of an influenza A virus H1, H2, H5, H6, H8, H9, H11, H12, H13, H16, H17, H18, H3, H4, H7, H10, H14, and H15 subtypes (e.g., H1, H2, H5, H6, H8, H9, H12, H13, H16, H17, H18, H3, H4, H7, H10, H14, and H15 subtypes).

In an embodiment, each of the clone 7-derived antibodies/antibody fragments is used for treating or preventing an infection with at least one subtype selected from the group consisting of an influenza A virus H1, H2, H5, H6, H8, H9, H11, H12, H13, H16, H17, H18, H3, H4, H7, H10, H14, and H15 subtypes (e.g., H1, H2, H5, H6, H8, H9, H12, H13, H16, H17, H18, H3, H4, H7, H10, H14, and H15 subtypes).

In an embodiment, each of the clone 7-derived antibodies/antibody fragments is used for detecting or quantifying a membrane fusion-type influenza HA antigen derived from at least one subtype selected from the group consisting of H1, H2, H5, H6, H8, H9, H11, H12, H13, H16, H17, H18, H3, H4, H7, H10, H14, and H15 subtypes (e.g., H1, H2, H5, H6, H8, H9, H12, H13, H16, H17, H18, H3, H4, H7, H10, H14, and H15 subtypes).

In an embodiment, the monoclonal antibody or antibody fragment thereof according to the present disclosure comprises:

(g)-1 a heavy chain variable region comprising
CDR1 comprising (or consisting of) an amino acid sequence of SEQ ID NO: 39 or an amino acid sequence in which one or two amino acid residues are independently substituted, deleted, added, or inserted in the amino acid sequence, CDR2 comprising (or consisting of) an amino acid sequence of SEQ ID NO: 40 or an amino acid sequence in which one or two amino acid residues are independently substituted, deleted, added, or inserted in the amino acid sequence, and CDR3 comprising (or consisting of) an amino acid sequence of SEQ ID NO: 41 or an amino acid sequence in which one or two amino acid residues are independently substituted, deleted, added, or inserted in the amino acid sequence; and/or

a light chain variable region comprising

CDR1 comprising (or consisting of) an amino acid sequence of SEQ ID NO: 42 or an amino acid sequence in which one or two amino acid residues are independently substituted, deleted, added, or inserted in the amino acid sequence, CDR2 comprising (or consisting of) an amino acid sequence of SEQ ID NO: 43 or an amino acid sequence in which one or two amino acid residues are independently substituted, deleted, added, or inserted in the amino acid sequence, and CDR3 comprising (or consisting of) an amino acid sequence of SEQ ID NO: 44 or an amino acid sequence in which one or two amino acid residues are independently substituted, deleted, added, or inserted in the amino acid sequence.

In an embodiment, the monoclonal antibody or antibody fragment thereof according to the present disclosure comprises:

(g)-2 a heavy chain variable region comprising

CDR1 comprising (or consisting of) an amino acid sequence of SEQ ID NO: 39 or an amino acid sequence in which one or two amino acid residues are independently substituted, deleted, added, or inserted in the amino acid sequence, CDR2 comprising (or consisting of) an amino acid sequence of SEQ ID NO: 40 or an amino acid sequence in which one or two amino acid residues are independently substituted, deleted, added, or inserted in the amino acid sequence, and CDR3 comprising (or consisting of) an amino acid sequence of SEQ ID NO: 41 or an amino acid sequence in which one or two amino acid residues are independently substituted, deleted, added, or inserted in the amino acid sequence; and

a light chain variable region comprising

CDR1 comprising (or consisting of) an amino acid sequence of SEQ ID NO: 42 or an amino acid sequence in which one or two amino acid residues are independently substituted, deleted, added, or inserted in the amino acid sequence, CDR2 comprising (or consisting of) an amino acid sequence of SEQ ID NO: 43 or an amino acid sequence in which one or two amino acid residues are independently substituted, deleted, added, or inserted in the amino acid sequence, and CDR3 comprising (or consisting of) an amino acid sequence of SEQ ID NO: 44 or an amino acid sequence in which one or two amino acid residues are independently substituted, deleted, added, or inserted in the amino acid sequence.

In an embodiment, the monoclonal antibody or antibody fragment thereof according to the present disclosure comprises:

(g')-1 a heavy chain variable region comprising

CDR1 comprising (or consisting of) an amino acid sequence of SEQ ID NO: 39,

CDR2 comprising (or consisting of) an amino acid sequence of SEQ ID NO: 40, and

CDR3 comprising (or consisting of) an amino acid sequence of SEQ ID NO: 41; and/or

a light chain variable region comprising

CDR1 comprising (or consisting of) an amino acid sequence of SEQ ID NO: 42,

CDR2 comprising (or consisting of) an amino acid sequence of SEQ ID NO: 43, and

CDR3 comprising (or consisting of) an amino acid sequence of SEQ ID NO: 44.

In an embodiment, the monoclonal antibody or antibody fragment thereof according to the present disclosure comprises:

(g')-2 a heavy chain variable region comprising

CDR1 comprising (or consisting of) an amino acid sequence of SEQ ID NO: 39,

CDR2 comprising (or consisting of) an amino acid sequence of SEQ ID NO: 40, and

CDR3 comprising (or consisting of) an amino acid sequence of SEQ ID NO: 41; and

a light chain variable region comprising

CDR1 comprising (or consisting of) an amino acid sequence of SEQ ID NO: 42,

CDR2 comprising (or consisting of) an amino acid sequence of SEQ ID NO: 43, and

CDR3 comprising (or consisting of) an amino acid sequence of SEQ ID NO: 44.

In an embodiment, the monoclonal antibody or antibody fragment thereof according to the present disclosure comprises:

(g")-1 a heavy chain variable region comprising (or consisting of) an amino acid sequence having a sequence identity of at least 90%, 95%, 97%, or 99% with an amino acid sequence of SEQ ID NO: 45 and/or a light chain variable region comprising (or consisting of) an amino acid sequence having a sequence identity of at least 90%, 95%, 97%, or 99% with an amino acid sequence of SEQ ID NO: 46.

In an embodiment, the monoclonal antibody or antibody fragment thereof according to the present disclosure comprises:

(g")-2 a heavy chain variable region comprising (or consisting of) an amino acid sequence having a sequence identity of at least 90%, 95%, 97%, or 99% with an amino acid sequence of SEQ ID NO: 45 and a light chain variable region comprising (or consisting of) an amino acid sequence having a sequence identity of at least 90%, 95%, 97%, or 99% with an amino acid sequence of SEQ ID NO: 46.

In an embodiment, the monoclonal antibody or antibody fragment thereof according to the present disclosure comprises:

(g‴)-1 a heavy chain variable region comprising (or consisting of) an amino acid sequence of SEQ ID NO: 45 and/or a light chain variable region comprising (or consisting of) an amino acid sequence of SEQ ID NO: 46.

In an embodiment, the monoclonal antibody or antibody fragment thereof according to the present disclosure comprises:

(g‴)-2 a heavy chain variable region comprising (or consisting of) an amino acid sequence of SEQ ID NO: 45 and a light chain variable region comprising (or consisting of) an amino acid sequence of SEQ ID NO: 46.

In an embodiment, the monoclonal antibody or antibody fragment thereof according to the present disclosure comprises:

(g⁗)-1 a heavy chain variable region comprising (or consisting of) an amino acid sequence of SEQ ID NO: 45 or an amino acid sequence in which one to several (e.g., 1 to 10, 1 to 5, 1 to 3) amino acid residues are independently substituted, deleted, added, or inserted in the amino acid sequence and/or a light chain variable region comprising (or consisting of) an amino acid sequence of SEQ ID NO: 46 or an amino acid sequence in which one to several (e.g., 1 to 10, 1 to 5, 1 to 3) amino acid residues are independently substituted, deleted, added, or inserted in the amino acid sequence.

In an embodiment, the monoclonal antibody or antibody fragment thereof according to the present disclosure comprises:

(g⁗)-2 a heavy chain variable region comprising (or consisting of) an amino acid sequence of SEQ ID NO: 45 or an amino acid sequence in which one to several (e.g., 1 to 10, 1 to 5, 1 to 3) amino acid residues are independently substituted, deleted, added, or inserted in the amino acid sequence and a light chain variable region comprising (or consisting of) an amino acid sequence of SEQ ID NO: 46 or an amino acid sequence in which one to several (e.g., 1 to 10, 1 to 5, 1 to 3) amino acid residues are independently substituted, deleted, added, or inserted in the amino acid sequence.

In an embodiment, each of the monoclonal antibodies or antibody fragments thereof in (g)-1 and -2, (g')-1 and -2, (g")-1 and -2, (g‴)-1 and -2, and (g⁗)-1 and -2 (hereinafter, also referred to as a clone 1-derived antibody/antibody fragment) binds to a peptide consisting of an amino acid sequence of SEQ ID NO: 3 or SEQ ID NO: 21.

In an embodiment, each of the clone 1-derived antibodies/antibody fragments binds to a peptide consisting of an amino acid sequence of SEQ ID NO: 7 or SEQ ID NO: 25.

In an embodiment, each of the clone 1-derived antibodies/antibody fragments binds to a peptide consisting of an amino acid sequence of SEQ ID NO: 3 or SEQ ID NO: 21 and/or binds to a peptide consisting of an amino acid sequence of SEQ ID NO: 7 or SEQ ID NO: 25.

In an embodiment, each of the clone 1-derived antibodies/antibody fragments has protective capacity against infection for an influenza A virus H3 and/or H7 subtype(s).

In an embodiment, each of the clone 1-derived antibodies/antibody fragments is used for treating or preventing an infection with an influenza A virus HA subtype belonging to phylogenetic group 2 (e.g., two or more different influenza A virus HA subtypes belonging to phylogenetic group 2).

In an embodiment, each of the clone 1-derived antibodies/antibody fragments is used for treating or preventing an infection with an H3 and/or H7 subtype(s).

In an embodiment, each of the clone 1-derived antibodies/antibody fragments is used for detecting or quantifying a membrane fusion-type influenza HA antigen derived from an influenza A virus HA subtype belonging to phylogenetic group 2.

In an embodiment, each of the clone 1-derived antibodies/antibody fragments is used for detecting or quantifying a membrane fusion-type influenza HA antigen(s) derived from an H3 and/or H7 subtype(s).

In an embodiment, the monoclonal antibody or antibody fragment thereof according to the present disclosure comprises:

a heavy chain variable region comprising CDR1 comprising (or consisting of) an amino acid sequence selected from the group consisting of SEQ ID NOs: 47, 55, 63, 71, 79, and 87 or an amino acid sequence in which one or two amino acid residues are independently substituted, deleted, added, or inserted in the amino acid sequence selected from the group consisting of SEQ ID NOs: 47, 55, 63, 71, 79, and 87, CDR2 comprising (or consisting of) an amino acid sequence selected from the group consisting of SEQ ID NOs: 48, 56, 64, 72, 80, and 88 or an amino acid sequence in which one or two amino acid residues are independently substituted, deleted, added, or inserted in the amino acid sequence selected from the group consisting of SEQ ID NOs: 48, 56, 64, 72, 80, and 88, and CDR3 comprising (or consisting of) an amino acid sequence selected from the group consisting of SEQ ID NOs: 49, 57, 65, 73, 81, and 89 or an amino acid sequence in which one or two amino acid residues are independently substituted, deleted, added, or inserted in the amino acid sequence selected from the group consisting of SEQ ID NOs: 49, 57, 65, 73, 81, and 89; and/or

a light chain variable region comprising CDR1 comprising (or consisting of) an amino acid sequence selected from the group consisting of SEQ ID NOs: 50, 58, 66, 74, 82, and 90 or an amino acid sequence in which one or two amino acid residues are independently substituted, deleted, added, or inserted in the amino acid sequence selected from the group consisting of SEQ ID NOs: 50, 58, 66, 74, 82, and 90, CDR2 comprising (or consisting of) an amino acid sequence selected from the group consisting of SEQ ID NOs: 51, 59, 67, 75, 83, and 91 or an amino acid sequence in which one or two amino acid residues are independently substituted, deleted, added, or inserted in the amino acid sequence selected from the group consisting of SEQ ID NOs: 51, 59, 67, 75, 83, and 91, and CDR3 comprising (or consisting of) an amino acid sequence selected from the group consisting of SEQ ID NOs: 52, 60, 68, 76, 84, and 92 or an amino acid sequence in which one or two amino acid residues are independently substituted, deleted, added, or inserted in the amino acid sequence selected from the group consisting of SEQ ID NOs: 52, 60, 68, 76, 84, and 92 (hereinafter, also referred to as a clone-derived antibody/antibody fragment).

In an embodiment, the monoclonal antibody or antibody fragment thereof according to the present disclosure comprises:

a heavy chain variable region comprising CDR1 comprising (or consisting of) an amino acid sequence selected from the group consisting of SEQ ID NOs: 39, 47, 55, 63, 71, 79, and 87 or an amino acid sequence in which one or two amino acid residues are independently substituted, deleted, added, or inserted in the amino acid sequence selected from the group consisting of SEQ ID NOs: 39, 47, 55, 63, 71, 79, and 87, CDR2 comprising (or consisting of) an amino acid sequence selected from the group consisting of SEQ ID NOs: 40, 48, 56, 64, 72, 80, and 88 or an amino acid sequence in which one or two amino acid residues are independently substituted, deleted, added, or inserted in the amino acid sequence selected from the group consisting of SEQ ID NOs: 40, 48, 56, 64, 72, 80, and 88, and CDR3 comprising (or consisting of) an amino acid sequence selected from the group consisting of SEQ ID NOs: 41, 49, 57, 65, 73, 81, and 89 or an amino acid sequence in which one or two amino acid residues are independently substituted, deleted, added, or inserted in the amino acid sequence selected from the group consisting of SEQ ID NOs: 41, 49, 57, 65, 73, 81, and 89; and/or

a light chain variable region comprising CDR1 comprising (or consisting of) an amino acid sequence selected from the group consisting of SEQ ID NOs: 42, 50, 58, 66, 74, 82, and 90 or an amino acid sequence in which one or two amino acid residues are independently substituted, deleted, added, or inserted in the amino acid sequence selected from the group consisting of SEQ ID NOs: 42, 50, 58, 66, 74, 82, and 90, CDR2 comprising (or consisting of) an amino acid sequence selected from the group consisting of SEQ ID NOs: 43, 51, 59, 67, 75, 83, and 91 or an amino acid sequence in which one or two amino acid residues are independently substituted, deleted, added, or inserted in the amino acid sequence selected from the group consisting of SEQ ID NOs: 43, 51, 59, 67, 75, 83, and 91, and CDR3 comprising (or consisting of) an amino acid sequence selected from the group consisting of SEQ ID NOs: 44, 52, 60, 68, 76, 84, and 92 or an amino acid sequence in which one or two amino acid residues are independently substituted, deleted, added, or inserted in the amino acid sequence selected from the group consisting of SEQ ID NOs: 44, 52, 60, 68, 76, 84, and 92 (hereinafter, also referred to as a clone-derived antibody/antibody fragment).

In an embodiment, the monoclonal antibody or antibody fragment thereof according to the present disclosure comprises:

a heavy chain variable region comprising CDR1 comprising an amino acid sequence selected from the group consisting of SEQ ID NOs: 47, 55, 63, 71, 79, and 87, CDR2 comprising an amino acid sequence selected from the group consisting of SEQ ID NOs: 48, 56, 64, 72, 80, and 88, and CDR3 comprising (or consisting of) an amino acid sequence selected from the group consisting of SEQ ID NOs: 49, 57, 65, 73, 81, and 89; and/or

a light chain variable region comprising CDR1 comprising an amino acid sequence selected from the group consisting of SEQ ID NOs: 50, 58, 66, 74, 82, and 90, CDR2 comprising an amino acid sequence selected from the group consisting of SEQ ID NOs: 51, 59, 67, 75, 83, and 91, and CDR3 comprising (or consisting of) an amino acid sequence selected from the group consisting of SEQ ID NOs: 52, 60, 68, 76, 84, and 92 (hereinafter, also referred to as a clone-derived antibody/antibody fragment).

In an embodiment, the monoclonal antibody or antibody fragment thereof according to the present disclosure comprises:

a heavy chain variable region comprising CDR1 comprising an amino acid sequence selected from the group consisting of SEQ ID NOs: 39, 47, 55, 63, 71, 79, and 87, CDR2 comprising an amino acid sequence selected from the group consisting of SEQ ID NOs: 40, 48, 56, 64, 72, 80, and 88, and CDR3 comprising (or consisting of) an amino acid sequence selected from the group consisting of SEQ ID NOs: 41, 49, 57, 65, 73, 81, and 89; and/or

a light chain variable region comprising CDR1 comprising an amino acid sequence selected from the group consisting of SEQ ID NOs: 42, 50, 58, 66, 74, 82, and 90, CDR2 comprising an amino acid sequence selected from the group consisting of SEQ ID NOs: 43, 51, 59, 67, 75, 83, and 91, and CDR3 comprising (or consisting of) an amino acid sequence selected from the group consisting of SEQ ID NOs: 44, 52, 60, 68, 76, 84, and 92 (hereinafter, also referred to as a clone-derived antibody/antibody fragment).

In an embodiment, the monoclonal antibody or antibody fragment thereof according to the present disclosure comprises:

a heavy chain variable region comprising CDR1 comprising (or consisting of) an amino acid sequence selected from the group consisting of SEQ ID NOs: 47, 55, and 63, CDR2 comprising (or consisting of) an amino acid sequence selected from the group consisting of SEQ ID NOs: 48, 56, and 64, and CDR3 comprising (or consisting of) an amino acid sequence selected from the group consisting of SEQ ID NOs: 49, 57, and 65; and/or

a light chain variable region comprising CDR1 comprising (or consisting of) an amino acid sequence selected from the group consisting of SEQ ID NOs: 50, 58, and 66, CDR2 comprising (or consisting of) an amino acid sequence selected from the group consisting of SEQ ID NOs: 51, 59, and 67, and CDR3 comprising (or consisting of) an amino acid sequence selected from the group consisting of SEQ ID NOs: 52, 60, and 68 (hereinafter, also referred to as a clone-derived antibody/antibody fragment).

In an embodiment, the monoclonal antibody or antibody fragment thereof according to the present disclosure comprises:

a heavy chain variable region comprising CDR1 comprising (or consisting of) an amino acid sequence selected from the group consisting of SEQ ID NOs: 39, 47, 55, and 63, CDR2 comprising (or consisting of) an amino acid sequence selected from the group consisting of SEQ ID NOs: 40, 48, 56, and 64, and CDR3 comprising (or consisting of) an amino acid sequence selected from the group consisting of SEQ ID NOs: 41, 49, 57, and 65; and/or

a light chain variable region comprising CDR1 comprising (or consisting of) an amino acid sequence selected from the group consisting of SEQ ID NOs: 42, 50, 58, and 66, CDR2 comprising (or consisting of) an amino acid sequence selected from the group consisting of SEQ ID NOs: 43, 51, 59, and 67, and CDR3 comprising (or consisting of) an amino acid sequence selected from the group consisting of SEQ ID NOs: 44, 52, 60, and 68 (hereinafter also referred to as a clone-derived antibody/antibody fragment).

In an embodiment, the monoclonal antibody or antibody fragment thereof according to the present disclosure comprises:

a heavy chain variable region comprising CDR1 comprising (or consisting of) an amino acid sequence selected from the group consisting of SEQ ID NOs: 71, 79, and 87, CDR2 comprising (or consisting of) an amino acid sequence selected from the group consisting of SEQ ID NOs: 72, 80, and 88, and CDR3 comprising (or consisting of) an amino acid sequence selected from the group consisting of SEQ ID NOs: 73, 81, and 89; and/or

a light chain variable region comprising CDR1 comprising (or consisting of) an amino acid sequence selected from the group consisting of SEQ ID NOs: 74, 82, and 90, CDR2 comprising (or consisting of) an amino acid sequence selected from the group consisting of SEQ ID NOs: 75, 83, and 91, and CDR3 comprising (or consisting of) an amino acid sequence selected from the group consisting of SEQ ID NOs: 76, 84, and 92 (hereinafter, also referred to as a clone-derived antibody/antibody fragment).

Herein, the meaning of "comprising" a specific amino acid sequence includes "consisting of" the specific amino acid sequence and "consisting of" an amino acid sequence in which one or more amino acid residues are added to the specific amino acid sequence.

Herein, "substitution" may be conservative substitution or nonconservative substitution, and is preferably conservative substitution. Conservative substitution is known to those skilled in the art, and the term refers to substitution that does not cause any alteration in biological activity in the resulting molecule. Examples of conservative amino acid substitution include substitution from alanine to glycine or serine, substitution from arginine to lysine or histidine, substitution from asparagine to glutamine or histidine, substitution from aspartic acid to glutamic acid or asparagine, substitution from cysteine to serine or alanine, substitution from glutamine to asparagine, substitution from glutamic acid to aspartic acid or glutamine, substitution from glycine to alanine, substitution from histidine to asparagine or glutamine, substitution from isoleucine to leucine or valine, substitution from leucine to isoleucine or valine, substitution from lysine to arginine or histidine, substitution from methionine to leucine, isoleucine, or tyrosine, substitution from phenylalanine to tyrosine, methionine, or leucine, substitution from proline to alanine, substitution from serine to threonine, substitution from threonine to serine, substitution from tryptophan to tyrosine or phenylalanine, substitution from tyrosine to tryptophan or phenylalanine, and substitution from valine to isoleucine or leucine.

Herein, the term "sequence identity" refers to the proportion of amino acid residues matching between two sequences to be compared that have been aligned in an optimum state (in a state with the maximum matching) over the entire regions of the sequences. In the optimum alignment of two sequences, one sequence may have an addition or a deletion (e.g., a gap). Sequence identity can be calculated by producing alignment, for example, with use of a Clustal W algorithm (Nucleic Acid Res., 22(22): 4673-4680 (1994)). Sequence identity can be calculated with sequence analysis software such as Vector NTI and GENETYX-MAC, or an analysis tool provided by a public database (e.g., http://www.ddbj. nig.ac.jp).

In an embodiment, the monoclonal antibody or antibody fragment thereof according to the present disclosure is a monoclonal antibody or antibody fragment thereof that competes with any of the clone 1-derived antibodies/antibody fragments, clone 2-derived antibodies/antibody fragments, clone 3-derived antibodies/antibody fragments, clone 4-derived antibodies/antibody fragments, clone 5-derived antibodies/antibody fragments, clone 6-derived antibodies/antibody fragments, clone 7-derived antibodies/antibody fragments, clone 8-derived antibodies/antibody fragments, and clone-derived antibodies/antibody fragments for binding to an HA stem region (or LAH region).

In an embodiment, the monoclonal antibody or antibody fragment thereof according to the present disclosure is a monoclonal antibody or antibody fragment thereof that competes with any of the clone 1-derived antibodies/antibody fragments, clone 2-derived antibodies/antibody fragments, clone 3-derived antibodies/antibody fragments, clone 4-derived antibodies/antibody fragments, clone 5-derived antibodies/antibody fragments, clone 6-derived antibodies/antibody fragments, clone 7-derived antibodies/antibody fragments, clone 8-derived antibodies/antibody fragments, and clone-derived antibodies/antibody fragments for binding to a polypeptide consisting of an amino acid sequence selected from the amino acid sequences of SEQ ID NOs: 1 to 18 (or SEQ ID NOs: 19 to 36).

Herein, the term "compete" means that a monoclonal antibody or an antibody fragment thereof competes with a reference antibody (e.g., any of the clone 1-derived antibodies/antibody fragments, clone 2-derived antibodies/antibody fragments, clone 3-derived antibodies/antibody fragments, clone 4-derived antibodies/antibody fragments, clone 5-derived antibodies/antibody fragments, clone 6-derived antibodies/antibody fragments, clone 7-derived antibodies/antibody fragments, and clone 8-derived antibodies/antibody fragments) in binding assay using a polypeptide comprising a part or the whole of an HA stem region (or LAH region) (e.g., a polypeptide consisting of an amino acid sequence selected from the amino acid sequences of SEQ ID NOs: 1 to 18 or 19 to 36). For example, if a test antibody or an antibody fragment thereof reduces the binding of a reference antibody to a polypeptide comprising a part or the whole of an HA stem region (or LAH region) in binding assay, the test antibody or antibody fragment "competes" with the reference antibody. An antibody or antibody fragment that competes with a reference antibody reduces the binding of the reference antibody to an antigen by 10%, 20%, 30%, 40%, 50%, 60%, 70%, 80%, 90%, or 95% or more. Competition binding assay can be performed with a common method, and examples thereof include ELISA, flow cytometry, an SPR (surface plasmon resonance) method, and a BLI (Bio-Layer Interferometry) method.

Confirmation of binding capacity to an antigen can be performed with an assay method of any type such as a surface plasmon resonance (SPR) method, Western blotting, direct or indirect sandwich assay, flow cytometry, and immunoprecipitation assay (Zola, Monoclonal Antibodies: A Manual of Techniques, CRC Press, Inc. 1987, pp. 147-158).

Antigen avidity can be determined through a common ELISA method with addition of one step (a step of dissociating antigen-antibody binding). Thiocyanate is used for dissociating antigen-antibody binding, and the level of avidity is determined with reference to the thiocyanate concentration that causes dissociation. The antibody is used in a concentration range having high linearity and sensitivity to changes around the midpoint of a reaction curve in common ELISA. For thiocyanate concentration, there are a method of calculating thiocyanate resistance rates (%) as compared with an untreated sample at one or two steps of concentration, and a method of examining multiple concentrations to calculate concentration at which 50% inhibition is achieved (AI50). To the same epitope or antigen, higher avidity is generally predicted to indicate higher biological activity. For the antibody according to the present invention, the present test is effective for predicting the efficacy to virus strains that exhibit cross-reaction.

Binding affinity can be determined by using an SPR method. For apparatuses to measure interaction between substances with use of SPR, the Kretschmann configuration with a prism is typically employed. When a surface of a metal thin film of a sensor chip is irradiated with polarized light to cause total reflection on the surface, an SPR signal with reduced reflected light intensity is observed as part of reflected light. The angle of the SPR signal generated depends on the mass on the sensor chip, and hence varies through association or dissociation of molecules fixed on the sensor chip (ligand) and molecules added (analyte). Measurement results are acquired as a sensorgram showing the angle variation of the SPR signal and the response unit (RU) obtained by conversion from the angle variation over time. By fitting the sensorgram acquired for association and dissociation to a theoretical curve, the association rate constant (ka), dissociation rate constant (kd), and dissociation constant (KD = kd / ka) of the antibody to the antigen can be determined. In a certain embodiment, the SPR method is performed under conditions described in Examples.

In using as a medical antibody, the monoclonal antibody or antibody fragment thereof according to the present disclosure preferably has a reduced risk of exhibiting antigenicity when administered to a human. The monoclonal antibody or antibody fragment thereof can be a fully human antibody, humanized antibody, or chimeric antibody (e.g., a mouse-human chimeric antibody), or a fragment thereof. In using for quality control application in vaccine production, the monoclonal antibody or antibody fragment thereof according to the present disclosure can be a mouse antibody, chimeric antibody (e.g., a mouse-human chimeric antibody), humanized antibody, or fully human antibody, or a fragment thereof.

The monoclonal antibody or antibody fragment thereof according to the present disclosure may be a multispecific antibody or fragment thereof that binds to a polypeptide consisting of an amino acid sequence of any of SEQ ID NOs: 1 to 18 or 19 to 36 and to an antigen differing from this polypeptide.

A drug may have been bound to the monoclonal antibody or antibody fragment thereof. The drug can be, for example, a low-molecular-weight compound, a peptide, or a nucleic acid.

In the present disclosure, "a pharmaceutical composition comprising a monoclonal antibody or an antibody fragment thereof' is a pharmaceutical composition comprising at least one monoclonal antibody or at least one antibody fragment thereof, and such pharmaceutical compositions include a pharmaceutical composition comprising one monoclonal antibody, a pharmaceutical composition comprising two or more monoclonal antibodies, a pharmaceutical composition comprising one antibody fragment, a pharmaceutical composition comprising two or more antibody fragments, a pharmaceutical composition comprising one monoclonal antibody and one antibody fragment, and a pharmaceutical composition comprising two or more monoclonal antibodies and two or more antibody fragments.

In addition to the monoclonal antibody or antibody fragment thereof, the pharmaceutical composition may comprise a pharmaceutically acceptable carrier or excipient such as sterile water, physiological saline, a stabilizer, a diluent, an antioxidant, a buffering agent, a preservative, and a chelating agent.

The dosage form and route of administration of the monoclonal antibody or antibody fragment thereof to a subject can be appropriately determined with considering the purpose of administration, the condition of the subject, and others. Examples of the dosage form include an injection, a transdermal agent, an inhalant, a nasal drop, and an oral agent. In a certain embodiment, the dosage form is an injection. Examples of the route of administration include intravenous, intraarterial, subcutaneous, intramuscular, transdermal, transnasal, and oral administrations. In a certain embodiment, the route of administration is intravenous administration (e.g., from the cubital fossa or any other peripheral vein) or subcutaneous administration.

When being administered to a subject, the monoclonal antibody or antibody fragment thereof may be administered singly, or as a mixture of multiple components such as antibodies that bind to HA stem regions (or LAH regions) of two or more different influenza A virus HA subtypes belonging to phylogenetic group 1, antibodies that bind to HA stem regions (or LAH regions) of two or more different influenza A virus HA subtypes belonging to phylogenetic group 1, or a combination of these antibodies.

The dose of the monoclonal antibody or antibody fragment thereof can be appropriately adjusted in view of the purpose of administration, and the age and body weight of a subject, and others. For the dose, for example, a dose per administration selected from the range of 0.0001 mg to 1,000 mg/kg body weight can be employed, and a dose per patient selected from the range of 0.001 to 100,000 mg can be employed, though the dose is not limited thereto.

Examples of the subject to receive administration in the present disclosure include a mammal (e.g., a human, a mouse, a rat, a hamster, a guinea pig, a rabbit, a cat, a dog, a pig, a bovine, a horse, a sheep, a monkey), and preferably a human.

The schedule for administration of the monoclonal antibody or antibody fragment thereof can be appropriately adjusted in view of the purpose of administration, the age and body weight of a subject, and others. For example, the frequency of administration is typically in the range of once per week to once per 3 months, and more preferably in the range of once per 2 weeks to once per 10 weeks, for example, once per 4 to 8 weeks. For prophylactic treatment, typically, it is preferable to administer at a frequency of once per month to once per 2 to 3 months, or at a less frequency. The number of administrations may be one, and administration may be performed multiple times (e.g., 2 to 5 times) at intervals of several days or several weeks.

The present disclosure provides, in an embodiment, a monoclonal antibody or antibody fragment that binds to an epitope consisting of several to about a dozen of amino acids having common or similar characteristics in the amino acid sequence of an HA stem region (e.g., any of SEQ ID NOs: 1 to 18) of an influenza virus or a Long alpha helix region (LAH region, any of SEQ ID NOs: 19 to 36) in an HA stem region (e.g., any of SEQ ID NOs: 1 to 18) of an influenza virus.

The present disclosure provides, in an embodiment, a therapeutic agent or prophylactic agent for an influenza virus infection, the therapeutic agent or prophylactic agent comprising a monoclonal antibody or antibody fragment that binds to an epitope consisting of several to about a dozen of amino acids having common or similar characteristics in an HA stem region of an influenza virus or an LAH amino acid sequence in an HA stem region of an influenza virus.

The present disclosure provides, in an embodiment, a method for detecting or quantifying an HA-derived influenza virus vaccine antigen, the method including bringing a monoclonal antibody or antibody fragment that binds to a polypeptide consisting of several to about a dozen of amino acids having common or similar characteristics in an HA stem region of an influenza virus or an LAH amino acid sequence in an HA stem region of an influenza virus into contact with a sample.

The present disclosure relates to, in an embodiment, a method for measuring human blood anti-LAH antibody titer to be developed with the monoclonal antibody or chimeric antibody according to the present invention as a positive control.

The monoclonal antibody or antibody fragment thereof according to the present disclosure enables detection or quantification of an influenza HA antigen (e.g., a membrane fusion-type influenza HA antigen), and can be useful for quality control, for example, for an influenza vaccine comprising this or an intermediate product in production thereof. The quality control includes standard management and process management. The present disclosure provides, in an aspect, a method for detecting or quantifying a membrane fusion-type influenza HA antigen, the method including bringing the monoclonal antibody or antibody fragment thereof according to the present disclosure into contact with a sample. The sample can be, for example, an influenza vaccine comprising a membrane fusion-type influenza HA antigen or a sample obtained through a production process therefor. For example, the detection or quantification can be performed by ELISA or slot blotting. The membrane fusion-type influenza HA antigen contains the entire range of a stem region, and is an antigen obtained by subjecting an influenza A HA antigen to acidic treatment. For example, a stem region including an LAH region can be detected by using an antibody that recognizes the LAH region as a part of the membrane fusion-type influenza HA antigen.

In an embodiment, the membrane fusion-type influenza HA antigen can be produced in accordance with WO 2020/179797.

In an embodiment, the membrane fusion-type influenza HA antigen is obtained by treating an influenza A whole particle vaccine with ether to remove fat components that serve as pyrogens to give an influenza A HA antigen, and subjecting the resulting influenza A HA antigen to acidic treatment and then optionally to formalin treatment.

In an embodiment, the membrane fusion-type influenza HA antigen is in a shape with an HA stem region externally exposed.

The amount of an influenza HA antigen (not of membrane fusion-type) contained in a common inactivated influenza vaccine is quantified by SRD test (single radial immunodiffusion). In the SRD test, when an antigen is added into a hole that has been opened in a slab gel containing an antibody, the antigen radially diffuses from the well, and a precipitation ring is formed at an equilibrium point between the antibody and the antigen. The concentration of the antigen is determined by measuring the diameter of the ring. The SRD test is a method commonly employed as a titer test for inactivated influenza vaccines, and quantification is performed with a commercially available standard antigen or reference antiserum; however, the membrane fusion-type influenza HA antigen has a three-dimensional structure differing from those of normal influenza HA antigens and has reactivity differing from that of reference antiserum, and hence it is expected to be impossible to accurately measure the amount of the membrane fusion-type influenza HA antigen contained by the SRD test.

The monoclonal antibody or antibody fragment thereof according to the present invention binds to an epitope consisting of several to about a dozen of amino acids having common or similar characteristics in an LAH amino acid sequence of an HA stem region, and hence is suitable for quantification of the amount of a membrane fusion-type influenza HA antigen contained by Slot Blot test or ELISA test, thus being applicable as titer test for membrane fusion-type influenza HA antigens.

In an embodiment, the membrane fusion-type influenza HA antigen is obtained by a method including the following steps:

(1) treating an influenza A whole particle with ether to give an influenza A HA antigen;
(2) subjecting the resulting influenza A HA antigen to acidic treatment; and then
(3) optionally subjecting the resultant to formalin treatment.

In an embodiment, the membrane fusion-type influenza HA antigen is obtained by a method including the following steps:

(1) subjecting an influenza A whole particle to treatment for removing fat components that serve as pyrogens to give an influenza A HA antigen;
(2) subjecting the resulting influenza A HA antigen to acidic treatment; and then
(3) optionally subjecting the resultant to formalin treatment.

In the acidic treatment, the pH is not limited, and, for example, 2.0 to 6.5, preferably 3.0 to 6.5, more preferably 4.0 to 6.0, and further preferably 4.4 to 5.8. Specifically, for example, the pH is 2.0 to 2.9, 2.0 to 4.0, 2.0 to 5.0, 2.0 to 6.0, 3.0 to 4.0, 3.0 to 5.0, 3.0 to 6.0, 4.0 to 5.8, 4.0 to 6.5, 5.0 to 6.5, or 6.0 to 6.5. The acid to be used for the acidic treatment is not limited; for example, phosphoric acid, citric acid, maleic acid, or hydrochloric acid can be used.

The temperature in the acidic treatment is, for example, 0°C to 75°C, preferably 10°C to 60°C, more preferably 20°C to 45°C, and further preferably 25°C to 42°C. Specifically, for example, the temperature is 0°C to 20°C, 5°C to 25°C, 10°C to 30°C, 15°C to 35°C, 20°C to 37°C, 25°C to 37°C, 30°C to 50°C, 38°C to 55°C, 38°C to 60°C, 38°C to 65°C, 38°C to 70°C, 38°C to 75°C, 40°C to 55°C, 40°C to 60°C, 40°C to 65°C, 40°C to 70°C, 40°C to 75°C, 42°C to 55°C, 42°C to 60°C, 42°C to 65°C, 42°C to 70°C, 42°C to 75°C, 45°C to 55°C, 45°C to 60°C, 45°C to 65°C, 45°C to 70°C, or 45°C to 75°C. The treatment time is, for example, 5 minutes to 120 minutes, preferably 15 minutes to 60 minutes, and further preferably 20 minutes to 45 minutes. Specifically, for example, the treatment time is 5 minutes to 60 minutes, 20 minutes to 60 minutes, 15 minutes to 120 minutes, 15 minutes to 45 minutes, 20 minutes to 60 minutes, 20 minutes to 120 minutes, 45 minutes to 120 minutes, or 60 minutes to 120 minutes.

In measuring antibody titer or the avidity of an antibody in blood after vaccine administration, the monoclonal antibody or antibody fragment thereof according to the present disclosure can be used for construction of a test system for antibody titer or avidity, and as a positive control in measurement.

In this case, the monoclonal antibody or antibody fragment thereof according to the present disclosure is preferably one that binds to an LAH region.

A method for measuring human antibody titer for an LAH peptide, which is a membrane fusion-type HA antigenic moiety essential for clinical development of vaccines, is established, and this can lead to successful quantification thereof. In particular, development of a method for measuring blood antibody titer is limited by the current technical level, which lacks positive control serum, whereas use of the human monoclonal antibody or chimeric antibody according to the present invention as a positive control enables quantitative evaluation, and can reduce obstacles to development.

Examples

The following describes the present invention in detail with examples, but the present invention is by no means limited to them.

1. Production of mouse monoclonal antibody against H3N2

With use of a mouse infected with A/H3N2 influenza virus strain X31 and subjected to vaccination, mouse B cells and myeloma cells were fused by means of a hybridoma method to isolate clone 1 and clone 2.

2. Production of mouse monoclonal antibody against H1N1

From the lung tissue of a mouse infected with A/H1N1 influenza virus strain A/Narita/1/2009, HA-binding B cells were isolated by using a flow cytometer. A mouse antibody gene was cloned from the isolated B cells to produce monoclonal antibody clones 3 and 4.

3. Production of human monoclonal antibodies

From peripheral blood mononuclear cells of a healthy human, HA-binding memory B cells were isolated by using a flow cytometer. In production of clone 5, a human antibody gene was cloned from the isolated cells to produce a monoclonal antibody. In production of clone 6 and clone 7, a single-cell culture system was applied, and a human antibody gene was cloned from the cells after culture to produce a monoclonal antibody.

4. Production of chimeric antibody

On the basis of the amino acid sequences of the variable regions of the heavy chain and light chain of clone 4 antibody, which is a mouse monoclonal antibody, and information on the amino acid sequences of the constant regions of the heavy chain and light chain of a human antibody registered in a gene bank and having known sequences (heavy chain: registration No. QDA39867.1, light chain: registration No. P01834.2), a gene for a chimeric antibody in which the variable regions were derived from clone 4 and the constant regions from the human antibody was produced, and the chimeric antibody (clone 8) was expressed in CHO cells with a plasmid and then purified.

5. Measurement of antibody titer by ELISA method

The LAH antibody titers of the human or mouse monoclonal antibodies (clones 1 to 7) were measured by an ELISA (Enzyme-Linked ImmunoSorbent Assay) method through a procedure shown in the following. Recombinant HA antigens (A/X-31(H3N2), A/Victoria/361/2011 (H3N2), A/Puerto Rico/8/1934 (H1N1), A/Narita/1/2009 (H1N1pdm09), A/NIBRG-14 (H5N1), and A/Anhui/1/2013 (H7N9) were produced with reference to Onodera T et al. PNAS. 109 (7) 2485-2490. (2012); A/Singapore/INFIMH-16-0019/2016 (H3N2), A/Kansas/14/2017 (H3N2), A/Michigan/45/2015 (H1N1pdm09), and A/Brisbane/02/2018 (H1N1pdm09) were produced with reference to Onodera T et al. Immunity. 54 (10): 2385-2398.e10. (2021)) were each dissolved in phosphate buffered saline at 5 μg/ml, and added to a 96-well plate and incubated. Subsequently, each well was washed with phosphate buffered saline, phosphate buffered saline containing 1% bovine serum albumin was then added thereto, and the resultant was incubated. To the wells of the plate, a human or mouse monoclonal antibody (any of clones 1 to 7) in known concentrations obtained by serial dilution with phosphate buffer containing 0.05% Tween 20 and 1% bovine serum albumin was added, and the resultant was incubated. Each well of the plate was washed with phosphate buffered saline (containing 0.05% Tween 20), a peroxidase-labeled anti-human or anti-mouse IgG antibody (both from SouthernBiotech) diluted with phosphate buffered saline containing 0.05% Tween 20 and 1% bovine serum albumin was then added to each well, and the resultant was incubated. Each well of the plate was washed with phosphate buffered saline (containing 0.05% Tween 20), a product prepared by adding an o-phenylendiamine tablet (Sigma-Aldrich Co. LLC) and hydrogen peroxide to citrate buffer (pH 5.0) was added as a substrate to each well, the resultant was incubated, after that the reaction was terminated with 2 N sulfuric acid, and the absorbance value at 490 nm was measured with a microplate reader (Bio-Rad Laboratories, Inc.).

6-1. Confirmation of cross-reactivities of monoclonal antibodies against LAH region

Through the procedure described above in 5., the antibody titers of the human monoclonal antibodies (clones 5 to 7) and mouse monoclonal antibodies (clones 1 to 4) were measured by an ELISA method. A graph with the ordinate representing ELISA measurements (absorbance) and the abscissa representing the reciprocals of antibody concentrations was drawn for each antibody, and the area under the curve (AUC) of each curve was calculated. Figure 1 shows the results.

Clone 6 and clone 7, which are each a human monoclonal antibody, were found to bind to the H1N1, H3N2, H5N1, and H7N9 antigens; thus, it was suggested that clone 6 and clone 7 widely bind to both of phylogenetic groups 1 and 2.

Clone 5, which is a human monoclonal antibody, was found to bind to H3N2 and H7N9; thus, it was suggested that clone 5 binds to phylogenetic group 2.

Clone 1 and clone 2, which are each a mouse monoclonal antibody, were found to bind to H3N2 and weakly bind to H7N9; thus, it was suggested that clone 1 and clone 2 bind to phylogenetic group 2.

Clone 3 and clone 4, which are each a mouse monoclonal antibody, were found to bind to H1N1 and H5N1; thus, it was suggested that clone 3 and clone 4 bind to phylogenetic group 1.

The following shows the sequences of the LAH moieties of the antigens corresponding to parts of the stem moieties (long alpha helix) used for the confirmation of cross-reactivity, and the strains from which the LAHs were derived.

| Strain | Sequence of LAH |
|---|---|
| A/X-31(H3N2) | RIQDLEKYVEDTKIDLWSYNAELLVALENQHTIDL TDSEMNKLFEKTRRQLRENA (SEQ ID NO: 21) |
| A/Victoria/361/2011 (H3N2) | RIQDLEKYVEDTKIDLWSYNAELLVALENQHTIDL TDSEMNKLFEKTKKQLRENA (SEQ ID NO: 37) |
| A/Singapore/INFIMH-16-0019/2016 (H3N2) | RVQDLEKYVEDTKIDLWSYNAELLVALENQHTID LTDSEMNKLFEKTKKQLRENA (SEQ ID NO: 38) |
| A/Kansas/14/2017 (H3N2) | RIQDLEKYVEDTKIDLWSYNAELLVALENQHTIDL TDSEMNKLFEKTKKQLRENA (SEQ ID NO: 95) |
| A/Puerto Rico/8/1934 (H1N1) | RMENLNKKVDDGFLDIWTYNAELLVLLENERTLD FHDSNVKNLYEKVKSQLKNNA (SEQ ID NO: 96) |
| A/Narita/1/2009 (H1N1pdm09) | RIENLNKKVDDGFLDIWTYNAELLVLLENERTLDY HDSNVKNLYEKVRSQLKNNA (SEQ ID NO: 19) |

(continued)

| Strain | Sequence of LAH |
|---|---|
| A/Michigan/45/2015 (H1N1pdm09) | RIENLNKKVDDGFLDIWTYNAELLVLLENERTLDY HDSNVKNLYEKVRNQLKNNA (SEQ ID NO: 97) |
| A/Brisbane/02/2018 (H1N1pdm09) | RIENLNKKVDDGFLDIWTYNAELLVLLENERTLDY HDSNVKNLYEKVRNQLKNNA (SEQ ID NO: 98) |
| A/NIBRG-14 (H5N1) | RIENLNKKMEDGFLDVWTYNAELLVLMENERTLD FHDSNVKNLYDKVRLQLRDNA (SEQ ID NO: 23) |
| A/Anhui/1/2013 (H7N9) | QIGNVINWTRDSITEVWSYNAELLVAMENQHTIDL ADSEMDKLYERVKRQLRENA (SEQ ID NO: 25) |

6-2. Analysis of binding capacity between monoclonal antibody and antigen protein

Through the procedure described above in 5., the antibody titers of the human monoclonal antibodies (clones 6 and 7) were measured by an ELISA method with 19 recombinant HA antigens. For comparing the amounts of recombinant monoclonal antibodies bound to the different HA antigens, the AUC values were calculated, and a heat map representing the results was shown as a drawing. Figure 2 and the following table show the results.

| | Clone 6 AUC value | Clone 7 AUC value |
|---|---|---|
| H1 | 3356 | 3323 |
| pH1 | 2485 | 3348 |
| H2 | 3312 | 3305 |
| H5 | 3242 | 3294 |
| H6 | 2803 | 3218 |
| H8 | 474 | 3323 |
| H9 | 632.2 | 3237 |
| H11 | 757.4 | 299.9 |
| H12 | 98.53 | 3343 |
| H13 | 117.3 | 1559 |
| H16 | 289.6 | 3199 |
| H17 | 3367 | 3349 |
| H18 | 3241 | 3165 |
| H3 | 3305 | 3196 |
| H4 | 3311 | 3254 |
| H7 | 3317 | 3263 |
| H10 | 3328 | 3225 |
| H14 | 3358 | 3316 |
| H15 | 3355 | 3270 |

Clone 6, which is a human monoclonal antibody, was found to bind to the H1N1, H2N2, H3N2, H4N6, H5N1, H6N1, H7N9, H8N4, H9N2, H10N7, H11N9, H14N5, H15N9, H16N3, H17N10, and H18N11 antigens; thus, it was suggested that clone 6 widely binds to both of phylogenetic groups 1 and 2.

Clone 7, which is a human monoclonal antibody, was found to bind to the H1N1, H2N2, H3N2, H4N6, H5N1, H6N1, H7N9, H8N4, H9N2, H10N7, H11N9, H12N5, H13N6, H14N5, H15N9, H16N3, H17N10, and H18N11 antigens;

thus, it was suggested that clone 7 widely binds to both of phylogenetic groups 1 and 2.

Having a wide range of binding activity to the H1 to H18 antigens, clones 6 and 7 are expected to exhibit protective activity against a wide range of antigens.

The 19 recombinant HA antigens used for the measurement are listed in a table shown below. The 19 recombinant HA antigens were all produced with reference to Saito S et al. PLoS Pathog 15(1): e1007427.

| H1 | A/South Carolina/1/1918 | H1N1 | Group 1 |
| pH1 | A/Narita/1/2009 | H1N1 | Group 1 |
| H2 | A/Japan/305/57 | H2N2 | Group 1 |
| H3 | A/Hong Kong/1/68 | H3N2 | Group 2 |
| H4 | A/duck/Czechoslovakia/1956 | H4N6 | Group 2 |
| H5 | A/Laos/JP127/2004 | H5N1 | Group 1 |
| H6 | Almallard/Sweden/81/2002 | H6N1 | Group 1 |
| H7 | A/Anhui/1/2013 | H7N9 | Group 2 |
| H8 | A/mallard/Sweden/24/2002 | H8N4 | Group 1 |
| H9 | A/guinea fowl/Hong Kong/WF10/99 | H9N2 | Group 1 |
| H10 | A/mallard/Interior Alaska/10BM01929R0/2010 | H10N7 | Group 2 |
| H11 | A/northern shoveler/Netherlands/18/1999 | H11N9 | Group 1 |
| H12 | A/mallard/Interior Alaska/7MP0167/2007 | H12N5 | Group 1 |
| H13 | A/black-headed gull/Sweden/1/1999 | H13N6 | Group 1 |
| H14 | A/mallard/Astrakhan/263/1982 | H14N5 | Group 2 |
| H15 | A/shearwater/Australia/2576/1979 | H15N9 | Group 2 |
| H16 | A/black-headed gull/Sweden/5/99 | H16N3 | Group 1 |
| H17 | A/little yellow-shouldered bat/Guatemala/060/2010 | H17N10 | Group 1 |
| H18 | A/flat-faced bat/Peru/033/2010 | H18N11 | Group 1 |

7. Method for quantifying anti-LAH human antibody titers by ELISA

It is desirable in measuring human blood antibody titers to use standard serum, as a positive control, having a known antibody titer. However, in the current situation lacking human LAH immune serum, there is no anti-LAH human standard serum, and no immune serum to be measured, likewise. Even under such limited conditions, a model system using monoclonal antibodies confirmed the feasibility of quantification of human anti-LAH antibody titers. Arbitrary titers (AU) for H1 LAH and H3 LAH in ELISA were set with clone 6, which is a human monoclonal antibody, as a reference standard, and the anti-H1 LAH and anti-H3 LAH antibody titers of clone 8, which is a chimeric antibody against H1 LAH, and clone 5, which is a human monoclonal antibody against H3 LAH, as specimens were quantified by a calibration curve method.

(1) Setting of arbitrary titer (AU) in ELISA with clone 6

Data were plotted on a graph with the ordinate representing absorbance and the abscissa representing logarithmic values of antibody concentration, and the antibody concentration at the midpoint (IC50) of the resulting sigmoid curve was set to 1 arbitrary titer (AU/mL) (clone 6 anti-H1-LAH antibody: 4.96 ng clone 6/AU; clone 6 anti-H3 LAH antibody: 11.08 ng clone 6/mL) (Figure 3).

(2) Preparation of calibration curve with clone 6 and quantification of specimens

Data were plotted on a graph with the ordinate representing absorbance and the abscissa representing logarithmic values of titer, and the resulting sigmoid curve was used for calculation of quantified titer values. For each of specimens (clone 8 (anti-H1 LAH antibody) and clone 5 (anti-H3 LAH antibody)), the absorbance was measured at multiple antibody concentrations, and a measurement (clone 8: 0.538; clone 5: 0.877) closest to the absorbance corresponding to the midpoint (IC50) of the calibration sigmoid curve (anti-H1 LAH antibody titer: 0.743, anti-H3 LAH antibody titer: 0.908) was employed, and the titer (clone 8: 0.589 AU/mL; clone 5: 0.920 AU/mL) at the antibody concentration (clone 8: 3.906 ng/mL; clone 5: 15.625 ng/mL) was calculated from the expression of the calibration curve (Figure 4).

The model system using monoclonal antibodies was confirmed to allow quantification of human antibody titer by the calibration curve method.

(3) Quantification of serum specimen with calibration curve for clone 6

Antibody titer measurement for an actual clinical specimen is performed by adding serum specimens obtained by appropriately diluting to three concentrations (e.g., 400-fold, 800-fold, 1200-fold dilution) to the same ELISA plate that receives a dilution series of a clone 6 reference standard having a known titer. Among the absorbance measurements obtained, a measurement closest to the absorbance corresponding to the midpoint (IC50) of the calibration sigmoid curve is employed, and the titer at the specimen concentration is calculated from the expression of the calibration curve, and the result is multiplied by the dilution rate to give the serum antibody titer.

While measurement with this system uses a monoclonal antibody as a reference standard, it is also acceptable in measurement of actual blood antibody titer to set standard serum and perform the calculation with the abscissa representing the logarithm of the dilution factor of the standard serum.

8. Protective capacity of passive immunity caused by monoclonal antibody against infection

Whether administration of a monoclonal antibody imparts protective capacity against infection was examined by using mice.

(1) Protective capacity against infection for H1N1 virus strain

Mice were assigned to a PBS administration group, as a control group, and seven groups (4 mice/group), as test groups, to each of which one of four monoclonal antibodies (clone 3, clone 4, clone 6, clone 7) was to be administered at 5 mg/kg body weight or 10 mg/kg body weight (for clone 6, only a group at 10 mg/kg body weight), were provided, and subjected to intraperitoneal administration 24 hours before infection and then to challenge trial with strain A/Guangdong-Maonan/SWL1536/2019 (H1N1pdm09). The efficacy of each antibody was evaluated on the basis of the body weight variation rates (Figure 5) and survival rates (Figure 6) of the mice to the challenge trial.

In the PBS administration group as a negative control, body weight loss began on day 3 after virus inoculation, the loss was significant until day 8, showing a loss of 20% or more, and three mice died in 6 or 7 days after the inoculation. The surviving mouse in turn gained body weight after that, but the body weight recovered only to 90% of that before the inoculation even after 12 days. All of the antibody administration groups exhibited about 5% of body weight loss on day 3 after virus inoculation, but in turn body weight gain after day 5, and all the mice survived; thus, protective effect against infection was found.

(2) Protective capacity against infection for H3N2 virus strain

Mice were assigned to a PBS administration group, as a control group, and three groups, as test groups, to each of which one of two monoclonal antibodies (clone 6, clone 7) was to be administered at 4 mg/kg body weight, were provided, and subjected to intraperitoneal administration 24 hours before infection and then to challenge trial with strain A/X31 (H3N2). The efficacy of each antibody was evaluated on the basis of the body weight variation rates (Figure 7) and survival rates (Figure 8) of the mice to the challenge trial.

The PBS administration group as a negative control exhibited significant body weight loss, a loss of 30%, from day 6 to day 9 after virus inoculation, and all the mice had died on day 8. In the antibody administration groups, body weight loss began on day 5 after virus inoculation, and continued until day 7 for clone 6 and until day 8 for clone 7. After those, the antibody administration groups in turn exhibited body weight gain, and all the mice survived; thus, protective effect against infection was found.

9. Measurement of avidity

The avidity between an LAH peptide antigen and an antibody is measured through steps of common ELISA with addition of one step, a step of dissociating the antibody from the antigen.

An antigen-immobilized plate is prepared in the same manner as in common ELISA, and reacted with a specimen containing a test antibody. Before use, the specimen has been diluted to a proper concentration that develops color at an OD490 of 0.7 ± 0.3 in common ELISA.

Treatment with sodium thiocyanate (NaSCN) solution follows to dissociate the antibody.

The plate is washed three times with PBS containing 0.05% Tween 20, PBS containing 1 mol/L or 2 mol/L sodium thiocyanate (NaSCN) is added at a liquid volume of 100 μL/well to treat at room temperature for 30 minutes, and the plate is then washed three times with PBS containing 0.05% Tween 20.

The amount of the antibody remaining in the plate without dissociation is measured by using a labeled secondary antibody and a chromogenic substrate in the same manner as in a common method, and the proportions of antibody molecules with high avidity, intermediate avidity, and low avidity as compared with a control untreated with NaSCN are calculated from the following expressions.

Proportion with high avidity: (OD490 of serum treated with 2 mol/L / OD490 of untreated serum) × 100% Proportion with intermediate avidity: (OD490 of serum treated with 1 mol/L / OD490 of serum treated with 2 mol/L) × 100% Proportion with low avidity: (OD490 of untreated serum / OD490 of serum treated with 1 mol/L) × 100%

10. CDR analysis

The nucleotide sequences of genes for clones 1 to 8 were acquired with reference to previous articles (Tiller et al., J. Immunol. Methods., 2008 and Tiller et al., J. Immunol. Methods., 2009). RNA was extracted from HA-binding B cells, and cDNA was synthesized through reverse transcription reaction. With use of the cDNA synthesized as a template, human/mouse IgG heavy chain/light chain regions were amplified by a PCR method. The amplified PCR products were subjected to Sanger's sequencing method to identify the nucleotide sequences for the antibodies. Information on CDRs in clones 1 to 8 was acquired by comparing the nucleotide sequences identified with an antibody gene database (IMGT: International ImMunoGeneTics database).

The amino acid sequences of the heavy chain CDR1 to 3 (H-CDR1 to 3), light chain CDR1 to 3 (L-CDR1 to 3), heavy chain variable region ($V_H$), and light chain variable region ($V_L$) of the monoclonal antibodies (clones 1 to 8) are shown in Tables 1 to 7.

[Table 1]

| Table 1 Clone name | Sequence information | | SEQ ID NO: |
|---|---|---|---|
| Clone 1 | H-CDR1 | GYSITSDYA | 39 |
| | H-CDR2 | ISYSGST | 40 |
| | H-CDR3 | TKNLIYYYGRPYYFDY | 41 |
| | L-CDR1 | QGISNY | 42 |
| | L-CDR2 | YTS | 43 |
| | L-CDR3 | QQYSKLPFT | 44 |
| | VH | EVQLQESGPGLVKPSQSLSLTCTVTGYSITSDYAWNWIRQFPGNKLEWMGYISYSGSTSYNPSLKSRISITRDTSKNQFFLQLNSVTTEDTATYYCTKNLIYYYGRPYYFDYWGQGTTLTVSS | 45 |
| | VL | DIQMTQTTSSLSASLGDRVTITCSASQGISNYLGWYQQKPDGTVKLLIYYTSNLHSGVPSRFSGSGSGTDYSLTISNLEPEDIATYYCQQYSKLPFTFGSGTKLEIK | 46 |

[Table 2]

| Table 2 Clone name | Sequence information | | SEQ ID NO: |
|---|---|---|---|
| Clone 2 | H-CDR1 | GYKFSSYW | 47 |
| | H-CDR2 | ILPGSGST | 48 |
| | H-CDR3 | ARSNWEAY | 49 |
| | L-CDR1 | QSLVHSNGNTY | 50 |
| | L-CDR2 | KVS | 51 |
| | L-CDR3 | SQSTHVPPT | 52 |
| | VH | EVQLQQSGAELMKPGASVKISCKATGYKFSS YWIEWVKQRPGHGLEWIGKILPGSGSTNYNE KLKGKATFTADTSSNTAYMQLSSLTSEDSAV YYCARSNWEAYWGQGTLVTVSA | 53 |
| | VL | DVVMTQTPLSLPVSLGDQASISCRSSQSLVHS NGNTYLHWYLQKPGQSPKLLIYKVSNRFSGV PDRFSGSGSGTDFTLKISRVEAEDLGVYFCSQ STHVPPTFGGGTKLEIK | 54 |

[Table 3]

| Table 3 Clone name | Sequence information | | SEQ ID NO: |
|---|---|---|---|
| Clone 3 | H-CDR1 | GYTFTSYW | 55 |
| | H-CDR2 | IYPSNGRT | 56 |
| | H-CDR3 | TIHRYDFDH | 57 |
| | L-CDR1 | QGINNY | 58 |
| | L-CDR2 | YTS | 59 |
| | L-CDR3 | QQYSKLPHT | 60 |
| | VH | QVQLQQSGTELVKPGASVKLSCKASGYTFTS YWIHWVKQRPGQGLEWIGEIYPSNGRTNYNE KFMSKATLTVDKSSSTAYMQLSSLTSEDSAV YYCTIHRYDFDHWGQGTTLTVSS | 61 |
| | VL | DIEMTQTTSSLSASLGDRVTISCSASQGINNYL NWYQQKPDGTVKLLIYYTSSLHSGVPSRFSGS GSGTDYSLTISILEPEDIATYYCQQYSKLPHTF GGGTKLEIK | 62 |

[Table 4]

| Table 4 Clone name | Sequence information | | SEQ ID NO: |
|---|---|---|---|
| Clones 4 and 8 | H-CDR1 | GFTFSRYY | 63 |
| | H-CDR2 | INNNGGST | 64 |
| | H-CDR3 | TSHGPWFTY | 65 |
| | L-CDR1 | QDINSH | 66 |
| | L-CDR2 | RAN | 67 |
| | L-CDR3 | LQYDEFPPT | 68 |
| | VH | EVQLVESGGGLVKLGGSLKLSCAASGFTFSR YYMSWVRQTPEKRLELVAAINNNGGSTYYP DTVKGRFTISRDNAKNTLYLQMSSLKSEDTA LYYCTSHGPWFTYWGQGTLVTVSA | 69 |
| | VL | DIKMTQSPSSMYASLGERVTITCKASQDINSH LSWFQQKPGKSPKTLIYRANRLVDGVPSRFSG SGSGQDYSLTISSLEYEDLGIYYCLQYDEFPPT FGGGTKLEIK | 70 |

[Table 5]

| Table 5 Clone name | Sequence information | | SEQ ID NO: |
|---|---|---|---|
| Clone 5 | H-CDR1 | GVSMNSNH | 71 |
| | H-CDR2 | IYYTGKT | 72 |
| | H-CDR3 | ARRAMASVQRGILRVFDL | 73 |
| | L-CDR1 | QIIDKW | 74 |
| | L-CDR2 | EAS | 75 |
| | L-CDR3 | QQYKSYLPYT | 76 |
| | VH | QVQLQESGPGLVKPSETLSLTCTVSGVSMNS NHWSWIRQAPGKELEWIGYIYYTGKTFYNPS LQSRVTVSLDTSKNQFYLKLTSVTAADTAVY YCARRAMASVQRGILRVFDLWGQGKMVTVS S | 77 |
| | VL | DIQMTQSPSTLSASVGDRVTITCRASQIIDKWL AWYQHKPGKAPKLLIYEASNLETGVPSRFSG SGSGTEFTLTISSLQPDDFATYFCQQYKSYLPY TFGQGTKLEIK | 78 |

[Table 6]

| Table 6 Clone name | Sequence information | | SEQ ID NO: |
|---|---|---|---|
| Clone 6 | H-CDR1 | GPMFSRSA | 79 |
| | H-CDR2 | IIPTVDLK | 80 |
| | H-CDR3 | ARMGSGSSYYGMDV | 81 |
| | L-CDR1 | QSLLQSNGNNY | 82 |
| | L-CDR2 | LGS | 83 |
| | L-CDR3 | LQARQSSVLT | 84 |
| | VH | QVQLEQSGAEVKKPGSSVKVSCKASGPMFSR SAFSWVRQAPGQGLEWMGRIIPTVDLKNYAQ KFQGRVTFTADKSTATSYMELRSLKSEDTAV YYCARMGSGSSYYGMDVWGLGTTVTVSS | 85 |
| | VL | DIVMTQSPLSLPVIPGEPASISCRSSQSLLQSNG NNYLDWYLQKPGQSPQLLIYLGSNRASGVPD RFSGSGSGTDFTLTISRVEAEDVGVYYCLQAR QSSVLTFGGGTKLEIK | 86 |

[Table 7]

| Table 7 Clone name | Sequence information | | SEQ ID NO: |
|---|---|---|---|
| Clone 7 | H-CDR1 | GGPLTSSTYF | 87 |
| | H-CDR2 | INFRGTT | 88 |
| | H-CDR3 | ARHTQRLTVFTVVHGFDL | 89 |
| | L-CDR1 | TGDIGVSDF | 90 |
| | L-CDR2 | DVT | 91 |
| | L-CDR3 | CLYSTRSRDWE | 92 |
| | VH | QVQLQESGPGLVKPSETLSLTCSVSGGPLTSS TYFWGWIRQPPGKGLEWLGSINFRGTTDNAP SLKSRVTLSIDTSTHQFSLRLSSVTAKDTAVY YCARHTQRLTVFTVVHGFDLWGQGTVVSVS S | 93 |
| | VL | QSALTQPASVSGSPGQSITISCTGATGDIGVSD FVSWYQQHPGKAPKLIIFDVTNRPSGVSNRFS GSKSVNTASLTISGLQAEDEADYYCLYSTRS RDWEFGGGTKLTVL | 94 |

11. Quality control of influenza vaccine (slot blotting)

(1) Production of membrane fusion-type influenza vaccine

1. Preparation of HA split vaccine

Tween 80 was added to particles of H3N2 influenza virus (strain X31) or particles of H1N1 influenza virus (strain A/Puerto Rico/8/34) suspended in phosphate buffered saline to a final concentration of 0.2%, and suspended therein. Diethyl ether was added and further suspended, and the suspension was left to stand until an aqueous layer and a diethyl ether layer were completely separated, and then the diethyl ether layer was removed. After repeating this ether extraction, diethyl ether remaining in the recovered aqueous layer was distilled off at normal pressure to obtain an HA split vaccine.

2. Acidic treatment

The HA split vaccine was suspended in phosphate buffered saline, and an acidic treatment was then performed by adding 0.15 M citrate buffer (pH 3.5) to bring the pH to 5.0. After standing at room temperature for 30 minutes, 1 M Tris buffer (pH S.0) was added so that the pH was returned to 7.3. Thereafter, centrifugation was performed to obtain a membrane fusion-type HA split vaccine. Formalin was added to the membrane fusion-type HA split vaccine thus prepared to a final concentration of 0.05%, and the resultant was left to stand for several days.

(2) Detection and quantification of membrane fusion-type influenza vaccine

The H1 membrane fusion-type influenza HA antigen produced in (1) was detected and quantified by slot blotting as follows. To a membrane (Immobilon-P transfer membrane, Merck KGaA) installed in a 48-well slot blotting apparatus, diluted solutions containing the H1 membrane fusion-type influenza HA antigen in phosphate buffer (obtained by serially diluting 300 ng/mL solution at a common ratio of 1.6; 45.8 ng/mL to 300 ng/mL) and a diluted solution containing a sample to be tested in phosphate buffer (approximately 150 ng/mL) were adhered, and then blocked with Blocker (TM) BSA (10×) (Thermo Fisher Scientific) 10-fold-diluted with phosphate buffer. The membrane was incubated with clone 3 (1 $\mu$g/ml), which is a mouse monoclonal antibody, diluted with phosphate buffer to which 0.05% Tween 20 had been added, and after that washed with phosphate buffer to which 0.05% Tween 20 had been added. The membrane was proved with a fluorescence-labeled secondary antibody (Thermo Fisher Scientific), and then washed with phosphate buffer to which 0.05% Tween 20 had been added, and visualization was performed with ChemiDoc Totch MP (Bio-Rad Laboratories, Inc.). As shown in Figure 9, the H1 membrane fusion-type influenza HA antigen reacted with clone 3, which is a mouse monoclonal antibody, in a concentration-dependent manner as assayed by slot blotting, and this result demonstrated that the H1 membrane fusion-type influenza HA antigen can be detected and quantified by using the monoclonal antibody. Likewise, as shown in Figure 10, it was demonstrated that the H3 membrane fusion-type influenza HA antigen, which was produced in (1), can be detected and quantified by using clone 1, which is a mouse monoclonal antibody.

12. Quality control of influenza vaccine (ELISA)

The H1 membrane fusion-type influenza HA antigen produced above in 11. was detected and quantified by ELISA as follows. To a 96-well plate, clone 3, which is a mouse monoclonal antibody, diluted with carbonate buffer (5 $\mu$g/mL) was adhered, diluted solutions containing the H1 membrane fusion-type influenza HA antigen in phosphate buffer (obtained by serially diluting 2000 ng/mL solution at a common ratio of 3; 34 pg/mL to 2000 ng/mL) and a diluted solution containing a sample to be tested in phosphate buffer (approximately 50 ng/mL) were added, and then blocked with Blocker (TM) BSA (10×) (Thermo Fisher Scientific) 10-fold-diluted with phosphate buffer. An anti-H1 membrane fusion-type influenza HA antigen polyclonal antibody prepared from rabbit antiserum was 2000-fold-diluted with phosphate buffer to which 0.05% Tween 20 had been added, and the resultant was added to the blocked plate, which was then incubated. The plate was washed with phosphate buffer to which 0.05% Tween 20 had been added, a secondary antibody (Anti rabbit IgG, HRP-Linked Antibody, Cell Signaling Technology, Inc.) 1000-fold-diluted with phosphate buffer to which 0.05% Tween 20 had been added was then added, and the resultant was incubated. Subsequently, the plate was washed with phosphate buffer to which 0.05% Tween 20 had been added, substrate solution (TMB 1-Component Microwell Peroxidase Substrate, SureBlue Reserve, Sera care Life Sciences, Inc.) was then added, and the resultant was incubated. To this solution, reaction-terminating solution (TMB Stop Solution, Sera care Life Sciences, Inc.) was added, and products of the enzymatic reaction were measured with a microplate reader. As shown in Figure 11, the H1 membrane fusion-type influenza HA antigen reacted with clone 3, which is a mouse monoclonal antibody, through ELISA in a concentration-dependent manner, and this result demonstrated that the H1 membrane fusion-type influenza HA antigen can be detected and quantified by using the monoclonal antibody. Likewise, as shown in Figure 12, it was demonstrated that the H3 membrane fusion-type influenza HA antigen, which was produced above in 11., can be detected and quantified by using clone 1, which is a mouse monoclonal antibody.

The methods shown above in 11 and 12 are expected to allow detection and quantification of the H1 membrane fusion-type influenza HA antigen not only with clone 3, which has binding capacity to H1 subtype, but also with any of clones 4, 6, 7, and 8. The methods are expected to allow detection and quantification of the H3 membrane fusion-type influenza HA antigen not only with clone 1, which has binding capacity to H3 subtype, but also with any of clones 2, 5, 6, and 7.

The membrane fusion-type influenza HA antigens have three-dimensional structures differing from those of conventional influenza HA antigens and have reactivity differing from that of reference antiserum in SRD test, which is a common quantification test, and hence it is expected to be difficult to quantify with a common quantification test, whereas the test results in 11 and 12 revealed that use of the monoclonal antibody or antibody fragment according to the present application for slot blotting test or ELISA test enables quantification of membrane fusion-type influenza HA antigens.

Industrial Applicability

The monoclonal antibody or antibody fragment according to the present disclosure is useful for treating or preventing an influenza infection, or quality control in production of an influenza vaccine.

**Claims**

1. A monoclonal antibody or an antibody fragment thereof, wherein the monoclonal antibody or antibody fragment thereof binds to a hemagglutinin stem region (HA stem region) of an influenza A virus.

2. The monoclonal antibody or antibody fragment thereof according to claim 1, wherein the monoclonal antibody or antibody fragment thereof binds to at least one peptide consisting of an amino acid sequence selected from the group consisting of SEQ ID NOs: 1 to 18.

3. The monoclonal antibody or antibody fragment thereof according to claim 1 or 2, wherein the monoclonal antibody or antibody fragment thereof binds to an LAH (long alpha helix) region of the HA stem region.

4. The monoclonal antibody or antibody fragment thereof according to any one of claims 1 to 3, wherein the monoclonal antibody or antibody fragment thereof binds to at least one peptide consisting of an amino acid sequence selected from the group consisting of SEQ ID NOs: 19 to 36.

5. The monoclonal antibody or antibody fragment thereof according to any one of claims 1 to 4, wherein the monoclonal antibody or antibody fragment thereof comprises:

   a heavy chain variable region comprising CDR1 comprising an amino acid sequence selected from the group consisting of SEQ ID NOs: 47, 55, 63, 71, 79, and 87 or an amino acid sequence in which one or two amino acid residues are independently substituted, deleted, added, or inserted in the amino acid sequence selected from the group consisting of SEQ ID NOs: 47, 55, 63, 71, 79, and 87, CDR2 comprising an amino acid sequence selected from the group consisting of SEQ ID NOs: 48, 56, 64, 72, 80, and 88 or an amino acid sequence in which one or two amino acid residues are independently substituted, deleted, added, or inserted in the amino acid sequence selected from the group consisting of SEQ ID NOs: 48, 56, 64, 72, 80, and 88, and CDR3 comprising an amino acid sequence selected from the group consisting of SEQ ID NOs: 49, 57, 65, 73, 81, and 89 or an amino acid sequence in which one or two amino acid residues are independently substituted, deleted, added, or inserted in the amino acid sequence selected from the group consisting of SEQ ID NOs: 49, 57, 65, 73, 81, and 89; and/or
   a light chain variable region comprising CDR1 comprising an amino acid sequence selected from the group consisting of SEQ ID NOs: 50, 58, 66, 74, 82, and 90 or an amino acid sequence in which one or two amino acid residues are independently substituted, deleted, added, or inserted in the amino acid sequence selected from the group consisting of SEQ ID NOs: 50, 58, 66, 74, 82, and 90, CDR2 comprising an amino acid sequence selected from the group consisting of SEQ ID NOs: 51, 59, 67, 75, 83, and 91 or an amino acid sequence in which one or two amino acid residues are independently substituted, deleted, added, or inserted in the amino acid sequence selected from the group consisting of SEQ ID NOs: 51, 59, 67, 75, 83, and 91, and CDR3 comprising an amino acid sequence selected from the group consisting of SEQ ID NOs: 52, 60, 68, 76, 84, and 92 or an amino acid sequence in which one or two amino acid residues are independently substituted, deleted, added, or inserted in the amino acid sequence selected from the group consisting of SEQ ID NOs: 52, 60, 68, 76, 84, and 92.

6. The monoclonal antibody or antibody fragment thereof according to any one of claims 1 to 5, wherein the monoclonal antibody or antibody fragment thereof comprises:

   a heavy chain variable region comprising CDR1 comprising an amino acid sequence selected from the group consisting of SEQ ID NOs: 47, 55, 63, 71, 79, and 87, CDR2 comprising an amino acid sequence selected from the group consisting of SEQ ID NOs: 48, 56, 64, 72, 80, and 88, and CDR3 comprising an amino acid sequence selected from the group consisting of SEQ ID NOs: 49, 57, 65, 73, 81, and 89; and/or
   a light chain variable region comprising CDR1 comprising an amino acid sequence selected from the group consisting of SEQ ID NOs: 50, 58, 66, 74, 82, and 90, CDR2 comprising an amino acid sequence selected from the group consisting of SEQ ID NOs: 51, 59, 67, 75, 83, and 91, and CDR3 comprising an amino acid sequence selected from the group consisting of SEQ ID NOs: 52, 60, 68, 76, 84, and 92.

7. The monoclonal antibody or antibody fragment thereof according to any one of claims 1 to 6, wherein the monoclonal antibody or antibody fragment thereof comprises:

a heavy chain variable region comprising CDR1 comprising an amino acid sequence selected from the group consisting of SEQ ID NOs: 47, 55, and 63, CDR2 comprising an amino acid sequence selected from the group consisting of SEQ ID NOs: 48, 56, and 64, and CDR3 comprising an amino acid sequence selected from the group consisting of SEQ ID NOs: 49, 57, and 65; and/or
a light chain variable region comprising CDR1 comprising an amino acid sequence selected from the group consisting of SEQ ID NOs: 50, 58, and 66, CDR2 comprising an amino acid sequence selected from the group consisting of SEQ ID NOs: 51, 59, and 67, and CDR3 comprising an amino acid sequence selected from the group consisting of SEQ ID NOs: 52, 60, and 68.

8. The monoclonal antibody or antibody fragment thereof according to claims 1 to 6, wherein the monoclonal antibody or antibody fragment thereof comprises:

a heavy chain variable region comprising CDR1 comprising an amino acid sequence selected from the group consisting of SEQ ID NOs: 71, 79, and 87, CDR2 comprising an amino acid sequence selected from the group consisting of SEQ ID NOs: 72, 80, and 88, and CDR3 comprising an amino acid sequence selected from the group consisting of SEQ ID NOs: 73, 81, and 89; and/or
a light chain variable region comprising CDR1 comprising an amino acid sequence selected from the group consisting of SEQ ID NOs: 74, 82, and 90, CDR2 comprising an amino acid sequence selected from the group consisting of SEQ ID NOs: 75, 83, and 91, and CDR3 comprising an amino acid sequence selected from the group consisting of SEQ ID NOs: 76, 84, and 92.

9. The monoclonal antibody or antibody fragment thereof according to any one of claims 1 to 5, wherein the monoclonal antibody or antibody fragment thereof comprises:

(a) a heavy chain variable region comprising

CDR1 comprising an amino acid sequence of SEQ ID NO: 47 or an amino acid sequence in which one or two amino acid residues are independently substituted, deleted, added, or inserted in the amino acid sequence, CDR2 comprising an amino acid sequence of SEQ ID NO: 48 or an amino acid sequence in which one or two amino acid residues are independently substituted, deleted, added, or inserted in the amino acid sequence, and
CDR3 comprising an amino acid sequence of SEQ ID NO: 49 or an amino acid sequence in which one or two amino acid residues are independently substituted, deleted, added, or inserted in the amino acid sequence, and/or
a light chain variable region comprising
CDR1 comprising an amino acid sequence of SEQ ID NO: 50 or an amino acid sequence in which one or two amino acid residues are independently substituted, deleted, added, or inserted in the amino acid sequence, CDR2 comprising an amino acid sequence of SEQ ID NO: 51 or an amino acid sequence in which one or two amino acid residues are independently substituted, deleted, added, or inserted in the amino acid sequence, and
CDR3 comprising an amino acid sequence of SEQ ID NO: 52 or an amino acid sequence in which one or two amino acid residues are independently substituted, deleted, added, or inserted in the amino acid sequence;

(b) a heavy chain variable region comprising

CDR1 comprising an amino acid sequence of SEQ ID NO: 55 or an amino acid sequence in which one or two amino acid residues are independently substituted, deleted, added, or inserted in the amino acid sequence, CDR2 comprising an amino acid sequence of SEQ ID NO: 56 or an amino acid sequence in which one or two amino acid residues are independently substituted, deleted, added, or inserted in the amino acid sequence, and
CDR3 comprising an amino acid sequence of SEQ ID NO: 57 or an amino acid sequence in which one or two amino acid residues are independently substituted, deleted, added, or inserted in the amino acid sequence, and/or

a light chain variable region comprising

CDR1 comprising an amino acid sequence of SEQ ID NO: 58 or an amino acid sequence in which one or two amino acid residues are independently substituted, deleted, added, or inserted in the amino acid sequence, CDR2 comprising an amino acid sequence of SEQ ID NO: 59 or an amino acid sequence in which one or two amino acid residues are independently substituted, deleted, added, or inserted in the amino acid sequence, and

CDR3 comprising an amino acid sequence of SEQ ID NO: 60 or an amino acid sequence in which one or two amino acid residues are independently substituted, deleted, added, or inserted in the amino acid sequence;

(c) a heavy chain variable region comprising

CDR1 comprising an amino acid sequence of SEQ ID NO: 63 or an amino acid sequence in which one or two amino acid residues are independently substituted, deleted, added, or inserted in the amino acid sequenc , CDR2 comprising an amino acid sequence of SEQ ID NO: 64 or an amino acid sequence in which one or two amino acid residues are independently substituted, deleted, added, or inserted in the amino acid sequence, and

CDR3 comprising an amino acid sequence of SEQ ID NO: 65 or an amino acid sequence in which one or two amino acid residues are independently substituted, deleted, added, or inserted in the amino acid sequence, and/or

a light chain variable region comprising

CDR1 comprising an amino acid sequence of SEQ ID NO: 66 or an amino acid sequence in which one or two amino acid residues are independently substituted, deleted, added, or inserted in the amino acid sequence, CDR2 comprising an amino acid sequence of SEQ ID NO: 67 or an amino acid sequence in which one or two amino acid residues are independently substituted, deleted, added, or inserted in the amino acid sequence, and

CDR3 comprising an amino acid sequence of SEQ ID NO: 68 or an amino acid sequence in which one or two amino acid residues are independently substituted, deleted, added, or inserted in the amino acid sequence;

(d) a heavy chain variable region comprising

CDR1 comprising an amino acid sequence of SEQ ID NO: 71 or an amino acid sequence in which one or two amino acid residues are independently substituted, deleted, added, or inserted in the amino acid sequence, CDR2 comprising an amino acid sequence of SEQ ID NO: 72 or an amino acid sequence in which one or two amino acid residues are independently substituted, deleted, added, or inserted in the amino acid sequence, and

CDR3 comprising an amino acid sequence of SEQ ID NO: 73 or an amino acid sequence in which one or two amino acid residues are independently substituted, deleted, added, or inserted in the amino acid sequence, and/or

a light chain variable region comprising

CDR1 comprising an amino acid sequence of SEQ ID NO: 74 or an amino acid sequence in which one or two amino acid residues are independently substituted, deleted, added, or inserted in the amino acid sequence, CDR2 comprising an amino acid sequence of SEQ ID NO: 75 or an amino acid sequence in which one or two amino acid residues are independently substituted, deleted, added, or inserted in the amino acid sequence, and

CDR3 comprising an amino acid sequence of SEQ ID NO: 76 or an amino acid sequence in which one or two amino acid residues are independently substituted, deleted, added, or inserted in the amino acid sequence;

(e) a heavy chain variable region comprising

CDR1 comprising an amino acid sequence of SEQ ID NO: 79 or an amino acid sequence in which one or two amino acid residues are independently substituted, deleted, added, or inserted in the amino acid sequence, CDR2 comprising an amino acid sequence of SEQ ID NO: 80 or an amino acid sequence in which one or two amino acid residues are independently substituted, deleted, added, or inserted in the amino acid sequence, and

CDR3 comprising an amino acid sequence of SEQ ID NO: 81 or an amino acid sequence in which one or two amino acid residues are independently substituted, deleted, added, or inserted in the amino acid sequence,

and/or
a light chain variable region comprising
CDR1 comprising an amino acid sequence of SEQ ID NO: 82 or an amino acid sequence in which one or two amino acid residues are independently substituted, deleted, added, or inserted in the amino acid sequence,
CDR2 comprising an amino acid sequence of SEQ ID NO: 83 or an amino acid sequence in which one or two amino acid residues are independently substituted, deleted, added, or inserted in the amino acid sequence, and
CDR3 comprising an amino acid sequence of SEQ ID NO: 84 or an amino acid sequence in which one or two amino acid residues are independently substituted, deleted, added, or inserted in the amino acid sequence;
or

(f) a heavy chain variable region comprising

CDR1 comprising an amino acid sequence of SEQ ID NO: 87 or an amino acid sequence in which one or two amino acid residues are independently substituted, deleted, added, or inserted in the amino acid sequence,
CDR2 comprising an amino acid sequence of SEQ ID NO: 88 or an amino acid sequence in which one or two amino acid residues are independently substituted, deleted, added, or inserted in the amino acid sequence, and
CDR3 comprising an amino acid sequence of SEQ ID NO: 89 or an amino acid sequence in which one or two amino acid residues are independently substituted, deleted, added, or inserted in the amino acid sequence, and/or
a light chain variable region comprising
CDR1 comprising an amino acid sequence of SEQ ID NO: 90 or an amino acid sequence in which one or two amino acid residues are independently substituted, deleted, added, or inserted in the amino acid sequence,
CDR2 comprising an amino acid sequence of SEQ ID NO: 91 or an amino acid sequence in which one or two amino acid residues are independently substituted, deleted, added, or inserted in the amino acid sequence, and
CDR3 comprising an amino acid sequence of SEQ ID NO: 92 or an amino acid sequence in which one or two amino acid residues are independently substituted, deleted, added, or inserted in the amino acid sequence.

10. The monoclonal antibody or antibody fragment thereof according to any one of claims 1 to 6, wherein the monoclonal antibody or antibody fragment thereof comprises:

(a') a heavy chain variable region comprising

CDR1 comprising an amino acid sequence of SEQ ID NO: 47,
CDR2 comprising an amino acid sequence of SEQ ID NO: 48, and
CDR3 comprising an amino acid sequence of SEQ ID NO: 49, and/or
a light chain variable region comprising
CDR1 comprising an amino acid sequence of SEQ ID NO: 50,
CDR2 comprising an amino acid sequence of SEQ ID NO: 51, and
CDR3 comprising an amino acid sequence of SEQ ID NO: 52;

(b') a heavy chain variable region comprising

CDR1 comprising an amino acid sequence of SEQ ID NO: 55,
CDR2 comprising an amino acid sequence of SEQ ID NO: 56, and
CDR3 comprising an amino acid sequence of SEQ ID NO: 57, and/or
a light chain variable region comprising
CDR1 comprising an amino acid sequence of SEQ ID NO: 58,
CDR2 comprising an amino acid sequence of SEQ ID NO: 59, and
CDR3 comprising an amino acid sequence of SEQ ID NO: 60;

(c') a heavy chain variable region comprising

CDR1 comprising an amino acid sequence of SEQ ID NO: 63,
CDR2 comprising an amino acid sequence of SEQ ID NO: 64, and
CDR3 comprising an amino acid sequence of SEQ ID NO: 65, and/or

a light chain variable region comprising
CDR1 comprising an amino acid sequence of SEQ ID NO: 66,
CDR2 comprising an amino acid sequence of SEQ ID NO: 67, and
CDR3 comprising an amino acid sequence of SEQ ID NO: 68;

(d') a heavy chain variable region comprising

CDR1 comprising an amino acid sequence of SEQ ID NO: 71,
CDR2 comprising an amino acid sequence of SEQ ID NO: 72, and
CDR3 comprising an amino acid sequence of SEQ ID NO: 73, and/or
a light chain variable region comprising
CDR1 comprising an amino acid sequence of SEQ ID NO: 74,
CDR2 comprising an amino acid sequence of SEQ ID NO: 75, and
CDR3 comprising an amino acid sequence of SEQ ID NO: 76;

(e') a heavy chain variable region comprising

CDR1 comprising an amino acid sequence of SEQ ID NO: 79,
CDR2 comprising an amino acid sequence of SEQ ID NO: 80, and
CDR3 comprising an amino acid sequence of SEQ ID NO: 81, and/or
a light chain variable region comprising
CDR1 comprising an amino acid sequence of SEQ ID NO: 82,
CDR2 comprising an amino acid sequence of SEQ ID NO: 83, and
CDR3 comprising an amino acid sequence of SEQ ID NO: 84; or

(f) a heavy chain variable region comprising

CDR1 comprising an amino acid sequence of SEQ ID NO: 87,
CDR2 comprising an amino acid sequence of SEQ ID NO: 88, and
CDR3 comprising an amino acid sequence of SEQ ID NO: 89, and/or
a light chain variable region comprising
CDR1 comprising an amino acid sequence of SEQ ID NO: 90,
CDR2 comprising an amino acid sequence of SEQ ID NO: 91, and
CDR3 comprising an amino acid sequence of SEQ ID NO: 92.

11. The monoclonal antibody or antibody fragment thereof according to any one of claims 1 to 10, wherein the monoclonal antibody or antibody fragment thereof comprises:

(a") a heavy chain variable region comprising an amino acid sequence having a sequence identity of at least 90% with an amino acid sequence of SEQ ID NO: 53 and/or a light chain variable region comprising an amino acid sequence having a sequence identity of at least 90% with an amino acid sequence of SEQ ID NO: 54;
(b") a heavy chain variable region comprising an amino acid sequence having a sequence identity of at least 90% with an amino acid sequence of SEQ ID NO: 61 and/or a light chain variable region comprising an amino acid sequence having a sequence identity of at least 90% with an amino acid sequence of SEQ ID NO: 62;
(c") a heavy chain variable region comprising an amino acid sequence having a sequence identity of at least 90% with an amino acid sequence of SEQ ID NO: 69 and/or a light chain variable region comprising an amino acid sequence having a sequence identity of at least 90% with an amino acid sequence of SEQ ID NO: 70;
(d") a heavy chain variable region comprising an amino acid sequence having a sequence identity of at least 90% with an amino acid sequence of SEQ ID NO: 77 and/or a light chain variable region comprising an amino acid sequence having a sequence identity of at least 90% with an amino acid sequence of SEQ ID NO: 78;
(e") a heavy chain variable region comprising an amino acid sequence having a sequence identity of at least 90% with an amino acid sequence of SEQ ID NO: 85 and/or a light chain variable region comprising an amino acid sequence having a sequence identity of at least 90% with an amino acid sequence of SEQ ID NO: 86; or
(f') a heavy chain variable region comprising an amino acid sequence having a sequence identity of at least 90% with an amino acid sequence of SEQ ID NO: 93 and/or a light chain variable region comprising an amino acid sequence having a sequence identity of at least 90% with an amino acid sequence of SEQ ID NO: 94.

12. The monoclonal antibody or antibody fragment thereof according to any one of claims 1 to 11, wherein the monoclonal antibody or antibody fragment thereof comprises:

(a‴) a heavy chain variable region comprising an amino acid sequence of SEQ ID NO: 53 and/or a light chain variable region comprising an amino acid sequence of SEQ ID NO: 54;

(b‴) a heavy chain variable region comprising an amino acid sequence of SEQ ID NO: 61 and/or a light chain variable region comprising an amino acid sequence of SEQ ID NO: 62;

(c‴) a heavy chain variable region comprising an amino acid sequence of SEQ ID NO: 69 and/or a light chain variable region comprising an amino acid sequence of SEQ ID NO: 70;

(d‴) a heavy chain variable region comprising an amino acid sequence of SEQ ID NO: 77 and/or a light chain variable region comprising an amino acid sequence of SEQ ID NO: 78;

(e‴) a heavy chain variable region comprising an amino acid sequence of SEQ ID NO: 85 and/or a light chain variable region comprising an amino acid sequence of SEQ ID NO: 86; or

(f‴) a heavy chain variable region comprising an amino acid sequence of SEQ ID NO: 93 and/or a light chain variable region comprising an amino acid sequence of SEQ ID NO: 94.

13. A monoclonal antibody or an antibody fragment thereof, wherein the monoclonal antibody or antibody fragment thereof competes with the monoclonal antibody or antibody fragment thereof according to any one of claims 5 to 12 for binding to the HA stem region.

14. The monoclonal antibody or antibody fragment thereof according to any one of claims 1 to 13, wherein the monoclonal antibody or antibody fragment thereof binds to HA stem regions of two or more influenza virus HA subtypes.

15. The monoclonal antibody or antibody fragment thereof according to any one of claims 1 to 14, wherein the monoclonal antibody or antibody fragment thereof binds to an HA stem region of an influenza A virus HA subtype belonging to phylogenetic group 1, and binds to an HA stem region of an influenza A virus HA subtype belonging to phylogenetic group 2.

16. The monoclonal antibody or antibody fragment thereof according to any one of claims 1 to 15, wherein the monoclonal antibody or antibody fragment thereof has protective capacity against infection for an influenza A virus.

17. The monoclonal antibody or antibody fragment thereof according to any one of claims 1 to 16, wherein the monoclonal antibody or antibody fragment thereof is a mouse antibody, chimeric antibody, humanized antibody, or fully human antibody, or a fragment thereof.

18. The monoclonal antibody or antibody fragment thereof according to any one of claims 1 to 7 and 9 to 17, wherein the monoclonal antibody or antibody fragment thereof is a mouse antibody or a fragment thereof.

19. The monoclonal antibody or antibody fragment thereof according to any one of claims 1 to 7 and 9 to 17, wherein the monoclonal antibody or antibody fragment thereof is a chimeric antibody or a fragment thereof.

20. The monoclonal antibody or antibody fragment thereof according to any one of claims 1 to 17, wherein the monoclonal antibody or antibody fragment thereof is a fully human antibody or a fragment thereof.

21. The monoclonal antibody or antibody fragment thereof according to any one of claims 1 to 20, comprising a modified constant region.

22. The monoclonal antibody or antibody fragment thereof according to any one of claims 1 to 21, wherein a drug has been bound to the monoclonal antibody or antibody fragment thereof.

23. A pharmaceutical composition for treating or preventing an influenza A virus infection, the pharmaceutical composition comprising the monoclonal antibody or antibody fragment thereof according to any one of claims 1 to 22.

24. The pharmaceutical composition according to claim 23, wherein the monoclonal antibody or antibody fragment thereof is the monoclonal antibody or antibody fragment thereof according to any one of claims 1 to 17 and 20 to 22.

25. The pharmaceutical composition according to claim 23, wherein the monoclonal antibody or antibody fragment thereof is the monoclonal antibody or antibody fragment thereof according to claim 8.

26. A method for detecting or quantifying a membrane fusion-type influenza HA antigen, the method comprising bringing the monoclonal antibody or antibody fragment thereof according to any one of claims 1 to 22 into contact with a sample.

27. The method according to claim 26, wherein the monoclonal antibody or antibody fragment thereof is the monoclonal antibody or antibody fragment thereof according to any one of claims 1 to 4, comprising:

(a) a heavy chain variable region comprising

CDR1 comprising an amino acid sequence of SEQ ID NO: 47 or an amino acid sequence in which one or two amino acid residues are independently substituted, deleted, added, or inserted in the amino acid sequence, CDR2 comprising an amino acid sequence of SEQ ID NO: 48 or an amino acid sequence in which one or two amino acid residues are independently substituted, deleted, added, or inserted in the amino acid sequence, and

CDR3 comprising an amino acid sequence of SEQ ID NO: 49 or an amino acid sequence in which one or two amino acid residues are independently substituted, deleted, added, or inserted in the amino acid sequence, and/or
a light chain variable region comprising
CDR1 comprising an amino acid sequence of SEQ ID NO: 50 or an amino acid sequence in which one or two amino acid residues are independently substituted, deleted, added, or inserted in the amino acid sequence, CDR2 comprising an amino acid sequence of SEQ ID NO: 51 or an amino acid sequence in which one or two amino acid residues are independently substituted, deleted, added, or inserted in the amino acid sequence, and

CDR3 comprising an amino acid sequence of SEQ ID NO: 52 or an amino acid sequence in which one or two amino acid residues are independently substituted, deleted, added, or inserted in the amino acid sequence;

(b) a heavy chain variable region comprising

CDR1 comprising an amino acid sequence of SEQ ID NO: 55 or an amino acid sequence in which one or two amino acid residues are independently substituted, deleted, added, or inserted in the amino acid sequence, CDR2 comprising an amino acid sequence of SEQ ID NO: 56 or an amino acid sequence in which one or two amino acid residues are independently substituted, deleted, added, or inserted in the amino acid sequence, and

CDR3 comprising an amino acid sequence of SEQ ID NO: 57 or an amino acid sequence in which one or two amino acid residues are independently substituted, deleted, added, or inserted in the amino acid sequence, and/or
a light chain variable region comprising
CDR1 comprising an amino acid sequence of SEQ ID NO: 58 or an amino acid sequence in which one or two amino acid residues are independently substituted, deleted, added, or inserted in the amino acid sequence, CDR2 comprising an amino acid sequence of SEQ ID NO: 59 or an amino acid sequence in which one or two amino acid residues are independently substituted, deleted, added, or inserted in the amino acid sequence, and

CDR3 comprising an amino acid sequence of SEQ ID NO: 60 or an amino acid sequence in which one or two amino acid residues are independently substituted, deleted, added, or inserted in the amino acid sequence;

(c) a heavy chain variable region comprising

CDR1 comprising an amino acid sequence of SEQ ID NO: 63 or an amino acid sequence in which one or two amino acid residues are independently substituted, deleted, added, or inserted in the amino acid sequence, CDR2 comprising an amino acid sequence of SEQ ID NO: 64 or an amino acid sequence in which one or two amino acid residues are independently substituted, deleted, added, or inserted in the amino acid sequence, and

CDR3 comprising an amino acid sequence of SEQ ID NO: 65 or an amino acid sequence in which one or two amino acid residues are independently substituted, deleted, added, or inserted in the amino acid sequence, and/or
a light chain variable region comprising
CDR1 comprising an amino acid sequence of SEQ ID NO: 66 or an amino acid sequence in which one or two amino acid residues are independently substituted, deleted, added, or inserted in the amino acid sequence,

CDR2 comprising an amino acid sequence of SEQ ID NO: 67 or an amino acid sequence in which one or two amino acid residues are independently substituted, deleted, added, or inserted in the amino acid sequence, and

CDR3 comprising an amino acid sequence of SEQ ID NO: 68 or an amino acid sequence in which one or two amino acid residues are independently substituted, deleted, added, or inserted in the amino acid sequence;

(d) a heavy chain variable region comprising

CDR1 comprising an amino acid sequence of SEQ ID NO: 71 or an amino acid sequence in which one or two amino acid residues are independently substituted, deleted, added, or inserted in the amino acid sequence,

CDR2 comprising an amino acid sequence of SEQ ID NO: 72 or an amino acid sequence in which one or two amino acid residues are independently substituted, deleted, added, or inserted in the amino acid sequence, and

CDR3 comprising an amino acid sequence of SEQ ID NO: 73 or an amino acid sequence in which one or two amino acid residues are independently substituted, deleted, added, or inserted in the amino acid sequence, and/or

a light chain variable region comprising

CDR1 comprising an amino acid sequence of SEQ ID NO: 74 or an amino acid sequence in which one or two amino acid residues are independently substituted, deleted, added, or inserted in the amino acid sequence ,

CDR2 comprising an amino acid sequence of SEQ ID NO: 75 or an amino acid sequence in which one or two amino acid residues are independently substituted, deleted, added, or inserted in the amino acid sequence, and

CDR3 comprising an amino acid sequence of SEQ ID NO: 76 or an amino acid sequence in which one or two amino acid residues are independently substituted, deleted, added, or inserted in the amino acid sequence;

(e) a heavy chain variable region comprising

CDR1 comprising an amino acid sequence of SEQ ID NO: 79 or an amino acid sequence in which one or two amino acid residues are independently substituted, deleted, added, or inserted in the amino acid sequence,

CDR2 comprising an amino acid sequence of SEQ ID NO: 80 or an amino acid sequence in which one or two amino acid residues are independently substituted, deleted, added, or inserted in the amino acid sequence, and

CDR3 comprising an amino acid sequence of SEQ ID NO: 81 or an amino acid sequence in which one or two amino acid residues are independently substituted, deleted, added, or inserted in the amino acid sequence, and/or

a light chain variable region comprising

CDR1 comprising an amino acid sequence of SEQ ID NO: 82 or an amino acid sequence in which one or two amino acid residues are independently substituted, deleted, added, or inserted in the amino acid sequence,

CDR2 comprising an amino acid sequence of SEQ ID NO: 83 or an amino acid sequence in which one or two amino acid residues are independently substituted, deleted, added, or inserted in the amino acid sequence, and

CDR3 comprising an amino acid sequence of SEQ ID NO: 84 or an amino acid sequence in which one or two amino acid residues are independently substituted, deleted, added, or inserted in the amino acid sequence;

(f) a heavy chain variable region comprising

CDR1 comprising an amino acid sequence of SEQ ID NO: 87 or an amino acid sequence in which one or two amino acid residues are independently substituted, deleted, added, or inserted in the amino acid sequence,

CDR2 comprising an amino acid sequence of SEQ ID NO: 88 or an amino acid sequence in which one or two amino acid residues are independently substituted, deleted, added, or inserted in the amino acid sequence, and

CDR3 comprising an amino acid sequence of SEQ ID NO: 89 or an amino acid sequence in which one or two amino acid residues are independently substituted, deleted, added, or inserted in the amino acid sequence, and/or

a light chain variable region comprising

CDR1 comprising an amino acid sequence of SEQ ID NO: 90 or an amino acid sequence in which one or two amino acid residues are independently substituted, deleted, added, or inserted in the amino acid sequence,

CDR2 comprising an amino acid sequence of SEQ ID NO: 91 or an amino acid sequence in which one or two amino acid residues are independently substituted, deleted, added, or inserted in the amino acid sequence, and

CDR3 comprising an amino acid sequence of SEQ ID NO: 92 or an amino acid sequence in which one or two amino acid residues are independently substituted, deleted, added, or inserted in the amino acid sequence; or

(g) a heavy chain variable region comprising

CDR1 comprising an amino acid sequence of SEQ ID NO: 39 or an amino acid sequence in which one or two amino acid residues are independently substituted, deleted, added, or inserted in the amino acid sequence, CDR2 comprising an amino acid sequence of SEQ ID NO: 40 or an amino acid sequence in which one or two amino acid residues are independently substituted, deleted, added, or inserted in the amino acid sequence, and

CDR3 comprising an amino acid sequence of SEQ ID NO: 41 or an amino acid sequence in which one or two amino acid residues are independently substituted, deleted, added, or inserted in the amino acid sequence, and/or

a light chain variable region comprising

CDR1 comprising an amino acid sequence of SEQ ID NO: 42 or an amino acid sequence in which one or two amino acid residues are independently substituted, deleted, added, or inserted in the amino acid sequence, CDR2 comprising an amino acid sequence of SEQ ID NO: 43 or an amino acid sequence in which one or two amino acid residues are independently substituted, deleted, added, or inserted in the amino acid sequence, and

CDR3 comprising an amino acid sequence of SEQ ID NO: 44 or an amino acid sequence in which one or two amino acid residues are independently substituted, deleted, added, or inserted in the amino acid sequence,

or a combination of any of them.

28. The method according to claim 26 or 27, wherein the monoclonal antibody or antibody fragment is a combination of a first antibody and a second antibody, wherein

the first antibody is: the monoclonal antibody or antibody fragment according to any one of claims 1 to 22; or a monoclonal antibody or an antibody fragment, with a heavy chain variable region comprising CDR1 comprising an amino acid sequence of SEQ ID NO: 39, CDR2 comprising an amino acid sequence of SEQ ID NO: 40, and CDR3 comprising an amino acid sequence of SEQ ID NO: 41, and a light chain variable region comprising CDR1 comprising an amino acid sequence of SEQ ID NO: 42, CDR2 comprising an amino acid sequence of SEQ ID NO: 43, and CDR3 comprising an amino acid sequence of SEQ ID NO: 44, and

the second antibody is a monoclonal antibody or an antibody fragment, wherein the monoclonal antibody or antibody fragment differs from the first antibody, and binds to a peptide that consists of an amino acid sequence selected from the group consisting of SEQ ID NOs: 1 to 18 and is capable of being bound by the first antibody.

[Fig. 1]

CROSS-REACTIVITIES OF MONOCLONAL ANTIBODIES

[Fig. 2]

[Fig. 3]

CLONE 6 ANTI-H1-LAH TITER SETTING

CLONE 6 ANTI-H3-LAH TITER SETTING

[Fig. 4]

ANTI-H1-LAH ANTIBODY TITER CALIBRATION CURVE

ANTI-H3-LAH ANTIBODY TITER CALIBRATION CURVE

[Fig. 5]

[Fig. 6]

[Fig. 7]

[Fig. 8]

[Fig. 9]

[Fig. 10]

[Fig. 11]

[Fig. 12]

## INTERNATIONAL SEARCH REPORT

| International application No. |
| --- |
| **PCT/JP2024/016344** |

**A. CLASSIFICATION OF SUBJECT MATTER**

*C07K 16/10*(2006.01)i; *A61K 39/395*(2006.01)i; *A61K 47/68*(2017.01)i; *A61P 31/16*(2006.01)i; *C12N 15/13*(2006.01)i; *G01N 33/569*(2006.01)i

FI: C07K16/10; A61K39/395 S; A61K47/68; A61K39/395 L; G01N33/569 L; C12N15/13; A61P31/16 ZNA

According to International Patent Classification (IPC) or to both national classification and IPC

**B. FIELDS SEARCHED**

Minimum documentation searched (classification system followed by classification symbols)

C07K16/10; A61K39/395; A61K47/68; A61P31/16; C12N15/13; G01N33/569

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | WO 2020/179797 A1 (JAPAN HEALTH SCIENCES FOUNDATION) 10 September 2020 (2020-09-10) abstract, paragraphs [0031], [0032], [0034], [0051], [0059], [0064], [0065], SEQ ID NO: 1, 2 | 1-28 |
| X | 安達 悠, インフルエンザ感染モデルにおける交差防御抗体の産生機序の解明, 科学研究費助成事業 研究成果報告書 [online], 30 September 2019, [retrieved on 10 July 2024], internet ＜URL: https://kaken.nii.ac.jp/ja/file/KAKENHI-PROJECT-16K19167/16K19167s eika.pdf＞, (ADACHI, Yu. Cross-protective antibody responses against influenza vires infection.), non-official translation (Grant-in-Aid for Scientific Research Research Report) column "4. Research results" | 1-3, 14-18, 20-24, 26 |
| A | ADACHI, Y. et al. Exposure of an occluded hemagglutinin epitope drives selection of a class of cross-protective influenza antibodies. NATURE COMMUNICATIONS. 2019, vol. 10, Article No. 3883, pp. 1-13 | 1-28 |

☑ Further documents are listed in the continuation of Box C.   ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **10 July 2024** | **23 July 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

International application No.

**PCT/JP2024/016344**

### C.    DOCUMENTS CONSIDERED TO BE RELEVANT

| Category* | Citation of document, with indication, where appropriate, of the relevant passages | Relevant to claim No. |
|---|---|---|
| A | 安達 悠，他, 隠れたインフルエンザヘマグルチニンエピトープに対する交差防御抗体, ウイルス, 2019, vol. 69, no. 2, pp. 161-168, (ADACHI, Yu et al. Human cross-protective antibodies to occluded epitopes in influenza hemagglutinin antigen. Uirusu.) | 1-28 |
| P, X | TONOUCHI, K. et al. Structural basis for cross-group recognition of an influenza virus hemagglutinin antibody that targets postfusion stabilized epitope. PLOS Pathogens. 09 August 2023, vol. 19, no. 8, e1011554, pp. 1-23 | 1-28 |

Form PCT/ISA/210 (second sheet) (July 2022)

**INTERNATIONAL SEARCH REPORT**

| International application No. |
| --- |
| **PCT/JP2024/016344** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/JP2024/016344**

| Patent document cited in search report | | | Publication date (day/month/year) | Patent family member(s) | | | Publication date (day/month/year) |
|---|---|---|---|---|---|---|---|
| WO | 2020/179797 | A1 | 10 September 2020 | US | 2022/0152191 | A1 | |
| | | | | EP | 3936147 | A1 | |
| | | | | abstract, paragraphs [0056], [0057], [0059], [0076], [0084], [0089], [0090], SEQ ID NO: 1, 2 | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019043937 A
- NL 72009
- NL 262009

- WO 2020179797 A
- JP 2004000127 A
- NL 181999

**Non-patent literature cited in the description**

- *VIRUS*, 2019, vol. 69 (2), 161-168
- *Front. Immunol.*, 2018, vol. 9, 116
- *Nat. Commun*, 2019, vol. 10, 3883
- EpitopeMapping. **MIKE SCHUTKOWSKI** ; **ULRICH REINEKE**. Ann NY AcadSci.. January 2010, vol. 1183, 267-87
- **MARTIN et al.** *Proc. Natl. Acad. Sci. USA*, 1989, vol. 86, 9268-9272
- **MARTIN et al.** *Methods Enzymol.*, 1991, vol. 203, 121-153
- **PEDERSEN et al.** *Immunomethods*, 1992, vol. 1, 126
- **REESET**. Protein Structure Prediction. Oxford University Press, 1996, 141-172
- **LEFRANC et al.** *Dev Comp Immunol.*, 2003, vol. 27, 55-77

- **AHMAD, Z. A. et al.** scFv antibody: principles and clinical application. *Clin. Dev. Immunol.*, 2012, vol. 2012, 980250
- **WARD et al.** *Nature*, 1989, vol. 341, 544-546
- **CLUSTAL W**. *Nucleic Acid Res.*, 1994, vol. 22 (22), 4673-4680
- **ZOLA**. Monoclonal Antibodies: A Manual of Techniques. CRC Press, Inc., 1987, 147-158
- **ONODERA T et al.** *PNAS.*, 2012, vol. 109 (7), 2485-2490
- **ONODERA T et al.** *Immunity.*, 2021, vol. 54 (10), 2385-2398.e10
- **SAITO S et al.** *PLoS Pathog*, vol. 15 (1), e1007427
- **TILLER et al.** *J. Immunol. Methods.*, 2008
- **TILLER et al.** *J. Immunol. Methods*, 2009